# EUROPEAN PATENT APPLICATION

(11) **EP 4 201 926 A1**
(43) Date of publication of application: **28.06.2023**
(21) Application number: 21905250.3
(22) Date of filing: 13.10.2021
(51) Int. Cl.: C07D 221/28, C07D 489/08

(54) **NOVEL INTERMEDIATE, PREPARATION METHOD FOR SAME, AND APPLICATIONS THEREOF**

(30) Priority: 18.12.2020 CN 202011504911; 18.01.2021 CN 202110064494
(71) Applicant: Sichuan University, Chengdu, Sichuan 610065 (CN)
(72) Inventor: QIN, Yong, Chengdu, Sichuan 610065 (CN); XUE, Fei, Chengdu, Sichuan 610065 (CN); SONG, Hao, Chengdu, Sichuan 610065 (CN); LIU, Xiaoyu, Chengdu, Sichuan 610065 (CN); ZHANG, Dan, Chengdu, Sichuan 610065 (CN); HE, Huan, Chengdu, Sichuan 610065 (CN)
(74) Representative: Cabinet Beau de Loménie
(86) International application number: PCT/CN2021/123651
(87) International publication number: WO 2022/127321

(57) **Abstract**

The present application relates to the field of drug synthesis, in particular to a novel intermediate, a method for preparing the same and application thereof. The structural formula of the novel intermediate provided by the present application is as expressed by formula I: 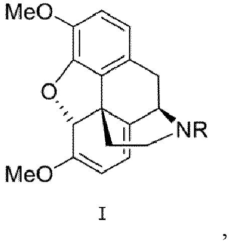 where R is a secondary amine protection group. Based on the possible biogenic pathway of morphine derivatives, the present application realizes the efficient synthesis of morphine derivatives through the strategy of biomimetic synthesis, taking the asymmetric transfer hydrogenation reaction and the intramolecular oxidative dearomatization Heck reaction in the process of preparing the intermediate as the key reactions of total synthesis. Using the novel intermediate provided by the present application to synthesize morphine derivatives has the characteristics of significantly reducing the synthesis steps, improving the yield, reducing the discharge of three wastes and reducing the production cost.

## Description

### Technical Field

The present application relates to the field of drug synthesis, in particular to an intermediate, a method for preparing the same and application thereof.

### Background

Morphine drugs, represented by hydrocodone, oxycodone, buprenorphine, nalaxone, naltrexone and the like, are mainly used as opioid receptor agonists for moderate and severe pain and palliative treatment caused by severe trauma, burn, bone fracture, cancer and the like, are used as an opioid receptor antagonists for treating respiratory depression and withdrawing opioid drug and alcohol addiction, are the basic drugs recognized by the World Health Organization. According to statistics, among the top 200 drugs in the global prescription use in 2016, morphine drugs accounted for 7 varieties, which have irreplaceable effects and extremely important clinical value in the drug market. According to the statistics of IQVIA database, in 2018, the global total production of morphine drugs was nearly 390 tons, and the sales amount of preparations reached 14.5 billion US dollars. While the clinical consumption of morphine drugs in China accounted for only 2% of the global total, the sales scale of morphine drugs in China has reached 4.4 billion yuan. Therefore, it can be predicted that under the huge population base of China, with the increase of cancer cases and the gradual attention to palliative treatment, as well as its promotion in treating respiratory depression and withdrawing drug and alcohol addiction, the market demand for such drugs will grow rapidly (World Health Organization, "18th WHO essential medicines list" (Geneva, Switzerland, 2013); Seya, M. J.; Gelders, S. F.; Achara, O. U.; Milani, B.; Scholten, W. K.; Pain Palliat, J. Care Pharmacother. 2011, 25,6).

Morphine drugs take the mother nucleus of morphine as the basic skeleton. In industrial production, morphine and tibain and their analogues are extracted through agricultural cultivation of opium poppy, and then their derivatives are semi-synthesized from morphine, tibain and their analogues. According to statistics, about 100000 hectares of opium poppy are legally planted in the world every year to extract 800 tons of raw materials (mainly morphine) to meet the legitimate drug production and scientific research needs (International Narcotics Control Board, "Narcotic drugs: Estimated world requirements for 2015-statistics for 2013", 2014). However, opium poppy cultivation not only has the problems of occupation of a large amount of farmland and illegal cultivation, but also may be affected by diseases and pests, climate, politics and other factors. The supply source is variable and unstable. It can be seen that the existing industrial production methods of morphine drugs not only have the problems of occupation of a large amount of farmland, complex production process and high cost, but also have the problems of long control process and complex procedure, which are easy to cause serious social problems due to inadequate control. Therefore, it is of great significance to develop new methods for the industrial production of morphine drugs based on total synthesis.

Morphine molecule is a highly compact five-ring fused complex molecule, which contains a five-membered dihydrofuran ring, a nitrogen-containing bridge ring, and five consecutive chiral centers including a benzyl quaternary carbon center. It is a star natural product molecule in the field of synthetic chemistry. In order to realize the efficient and practical total synthesis of morphine and solve the problem of the source of morphine drugs, since the first completion of the synthesis of morphine by the Gates Research Group in 1952, so far there have been nearly 40 reports on the research work of successfully and totally synthesizing morphine and its drugs in the world (Reed, J. W.; Hudlicky, T. Acc. Chem. Res. 2015, 48, 674; Gum, A.; Stabile, M. In Studies in Natural Products Chemistry, Vol. 18, Elsevier, Amsterdam, 1996, pp. 43-154; Taber, D. F.; Neubert, T. D.; Schlecht, M. F. In Strategies and Tactics in Organic Synthesis, Vol. 5, Ed.: Harmata, M., Elsevier, London, 2004, pp. 353-389). Compared with the cultivation, extraction and semi-synthesis of morphine drugs, these total synthesis methods have great defects in terms of cost and feasibility of industrial scale-up. Among many synthesis strategies, the biomimetic synthesis route designed based on the possible biogenic pathway of morphine, that is, imitating the catalytic effect of enzymes and constructing the morphine skeleton through the oxidative free radical coupling reaction of o-p-phenol, is the most ideal and efficient synthesis strategy. However, achieving the regional selectivity and high yield of the coupling reaction is a challenge that has been difficult for human beings to achieve in the past 70 years. Inspired by the above biogenic synthesis, the Barton Group realized the biomimetic synthesis of morphine for the first time in 1964, but the yield of the key coupling reaction was only 0.02% (Barton, D. H. R. Pure Appl. Chem. 1964, 9, 35). Subsequently, the Szantay Group and the White Group realized the biomimetic synthesis of morphine through the oxidative free radical coupling strategy of phenol, and the key reaction yields were only 2.7% and 21% (Szantay, C.; Barczai-Beke, M.; Pechy, P.; Blasko, G; Dornyei, G J. Org. Chem. 1982, 47, 594; White, J. D.; Caravatti, G; Kline, T. B.; Edstrom, E.; Rice, K. C.; Brossi, A. Tetrahedron, 1983, 39, 2393). Until 2018, the Opatz Group realized the key coupling reaction by electrochemical means with dilute reaction concentration (0.01M) and medium yield of 58-62% (Lipp, A.; Ferenc, D.; Ggtz, C.; Geffe, M.; Vierengel, N.; Schollmeyer, D.; Schafer, H. J.; Waldvogel, S. R.; Opatz, T. Angew. Chem. Int. Ed. 2018, 57, 11055). However, limited by the structure of the electrochemical reaction substrate, the Opatz Group completed the conversion from the coupling product to the morphine drug precursor thebaine through multi-step conversion. The subsequent cumbersome multi-step conversion process, the electrochemical reaction efficiency and the strict requirements on the relevant equipment limit the application of this synthesis method in industrial production.

In recent years, exploring the biosynthesis of morphine and its related drugs based on synthetic biology has become a new research focus in this field. So far, the efficiency of biosynthesis of morphine, thebaine and their analogues is far from meeting the needs of industrial production (Galanie, S.; Thodey, K.; Trenchard, I. J.; Filsinger, I. M.; Smolke, C. D. Science 2015, 349, 1095; Wang Pingping, Yang Chengshuai, Li Xiaodong, Jiang Yugo, Yan Xing, Zhou Zhihua, Organic Chemistry, 2018, 38, 2199).

Throughout the history of the synthesis of morphine and its analogues, although scientists have developed many new strategies and methods for constructing morphine skeletons, up to now, the existing total synthesis methods of morphine and its derivatives still have no practical significance and production value, and the extraction of morphine from opium poppy and the semi-synthesis of thebaine are still the only way to obtain morphine drugs in industry.

Therefore, there is an urgent need for a total synthesis method of morphine derivatives with advantages of high efficiency, simple operation and scalability.

### Summary

The purposes of the present application are to overcome serious social problems caused by the existing industrial production methods of morphine and its derivatives in the existing technology due to occupation of a large amount of farmland, complex production process, high cost, long control process, complex procedure and inadequate control, and to overcome the defects of the total synthesis method that still has no practical significance and production value, by providing an intermediate and a method for preparing the same, which can significantly improve the yield of the final product, reduce the reaction steps and decrease the production cost.

The purposes of the present application are realized through the following technical solutions:
The present application provides an intermediate, wherein the structural formula is as follow: 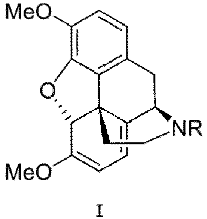 where R is a secondary amine protection group. The secondary amine protection group used in the present application is mainly selected based on the compatibility of the functional group and the avoidance of the side reaction, such as the avoidance of unnecessary side reaction in the subsequent Oxidative dearomatization Heck reaction, cyclization reaction, etc.

In some examples, the secondary amine protection group is one selected from the group consisting of benzenesulfonyl, p-toluenesulfonyl, p-nitrobenzenesulfonyl, methyl, methyl formate, tert-butoxycarbonyl, benzyl, benzyloxycarbonyl, trifluorsulfonyl, methanesulfonyl and trimethylbenzenesulfonyl.

The present application further provides a method for preparing the intermediate, which includes the following steps: 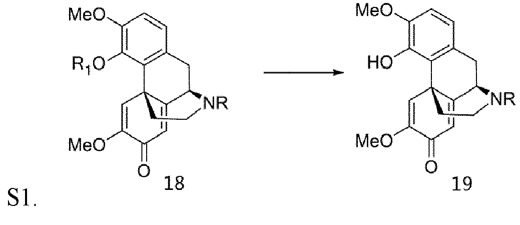 providing a compound 18 and producing a compound 19 through removal reaction of a hydroxyl protection group R₁, where R₁ is a hydroxyl protection group I; 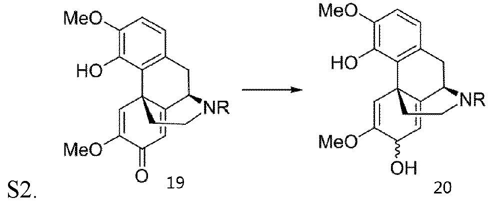 producing a compound 20 through reduction reaction of the compound 19; 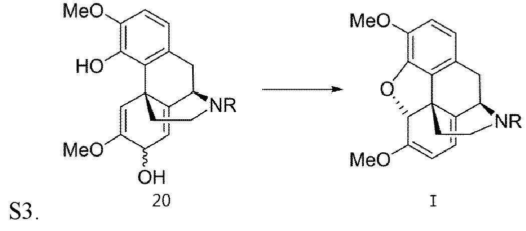 producing the intermediate I through cyclization reaction of the compound 20.

In some examples, in S 1, the hydroxyl protection group I is one selected from the group consisting of p-methoxybenzyl, benzyl, acetyl, benzyloxycarbonyl, methoxymethylene, methyl, triisopropylsilyl ether, triethylsilyl ether and tert-butyl diphenylsilyl. In the present application, the hydroxyl protection group I is also selected based on the compatibility of the functional group and the avoidance of the side reaction.

In some examples, in S 1, a removal reagent for the removal reaction of the hydroxyl protection group R₁ is one selected from the group consisting of sodium hydrosulfide, sodium sulfide, sodium ethanethiolate, thiophenol, sodium p-thiocresol, potassium fluoride, tetrabutylammonium fluoride, acetic acid, trifluoroacetic acid, hydrobromic acid, trimethyliodosilane, cerium trichloride, ceric ammonium nitrate, camphor sulfonic acid, p-toluenesulfonic acid, phosphorus oxychloride, 2,3-dichloro-5,6-dicyano-1,4-benzoquinone and hydrochloric acid. The removal reagent for the removal of the hydroxyl protection group R1 in the present application is mainly selected based on different removal of the hydroxyl protection group R₁. For example, those skilled in the art often use sodium hydrosulfide, sodium sulfide, sodium ethanethiolate, thiophenol, sodium p-thiocresol and the like to remove methyl; those skilled in the art often use potassium fluoride and tetrabutylammonium fluoride to remove silicon protection group and the like. All removal methods are common removal methods in the art.

And/or, in S 1, a reaction solvent for the removal reaction of the hydroxyl protection group R₁ is one selected from the group consisting of N.N-dimethylacetamide, N-methylpyrrolidone, methanol, N,N-dimethylformamide, acetonitrile, tetrahydrofuran, dichloromethane, 1,2-dichloroethane and acetic acid. In the present application, the reaction solvent for the removal of the hydroxyl protection group R₁ is also mainly selected based on the reason of reducing side reaction, reducing energy consumption or facilitating the forward reaction, as well as the different removal of the hydroxyl protection group R₁ and the adaptability of the removal reagent. All reaction solvents are common reaction solvents in the field.

And/or, in S 1, the reaction temperature for the removal of the hydroxyl protection group is -50 to 150°C. In the present application, the temperature for the removal of the hydroxyl protection group R₁ may be reasonably selected according to the reaction solvent, removal reagent and other conditions used for the removal of the hydroxyl protection group R₁, or based on the reasons of improving the yield, accelerating the reaction speed, reducing the side reaction and the like. For example, when the removal reagent is hydrobromic acid and the reaction solvent is N,N-dimethylformamide, the temperature may be selected to be 0-70°C; when the removal reagent is trifluoroacetic acid and the reaction solvent is dichloromethane, the temperature may be selected to be -40 to 0°C.

In addition, other reactions of the present application involve the selection of protection groups, reaction reagents and ratios, reaction conditions, etc., which may be reasonably selected by those skilled in the art according to different situations, and will not be described one by one here.

In some examples, in S 1, the molar ratio of the compound 18 to the removal reagent is 1:(3-25); and/or
in S 1, the removal reagent is hydrobromic acid, trifluoroacetic acid or sodium hydrosulfide; and/or
in S 1, the reaction solvent is dichloromethane, N,N-dimethylformamide or N,N-dimethylacetamide; and/or
in S1, the reaction temperature for removing the hydroxyl protection group R₁ is -40 to 150°C.

In some examples, in S2, a reducing agent for the reduction reaction is one selected from the group consisting of sodium borohydride, lithium borohydride, lithium aluminum hydride and lithium tri-tert-butyl aluminum hydride; and/or
in S2, the reaction solvent for the reduction reaction is one or two selected from the group consisting of methanol, ethanol, tetrahydrofuran and dichloromethane; and/or
in S2, the reaction temperature for the reduction reaction is -10 to 40°C.

In some examples, in S2, the molar ratio of the compound 19 to the reducing agent is 1 :(1.8-3); and/or
in S2, the reducing agent is sodium borohydride; and/or
in S2, the reaction solvent is methanol and dichloromethane; and/or
in S2, the temperature for the reduction reaction is 0-25°C.

In some examples, in S3, the reaction solvent for the cyclization reaction is one selected from the group consisting of N,N-dimethylformamide, N,N-dimethylformamide dimethyl acetal, acetonitrile, tetrahydrofuran, dichloromethane and 1,4-dioxane; and/or
in S3, a cyclizing reagent for the cyclization reaction is one selected from the group consisting of N,N-dimethylformamide dinovaentyl acetal, N,N-dimethylformamide dimethyl acetal, N,N-dimethylformamide diethyl acetal and N,N-dimethylformamide diisopropyl acetal; and/or
in S3, the reaction temperature for the cyclization reaction is 0-130°C.

In some examples, in S3, the molar ratio of the compound 20 to the cyclizing reagent is 1:(2-12); and/or
in S3, the cyclizing reagent is N,N-dimethylformamide dimethyl acetal; and/or
in S3, the reaction solvent is tetrahydrofuran, 1,4-dioxane or N,N-dimethylformamide dimethyl acetal; using N,N-dimethylformamide dimethyl acetal is capable of speeding up the reaction; and/or
in S3, the reaction temperature for the cyclization reaction is 50-130°C.

In some examples, a synthesis route of the compound 18 is as follows: 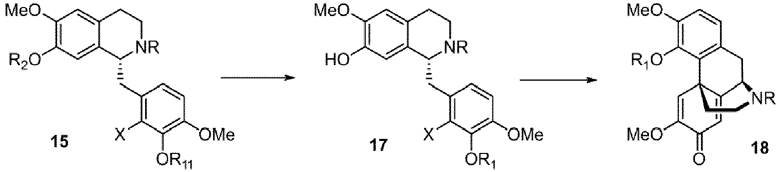
where R₂ is a hydroxyl protection group II, X is a halogen atom, R₁₁ is a hydroxyl protection group I or a hydrogen atom, and R₁ is a hydroxyl protection group I;
when R₁₁ is the hydroxyl protection group I, a synthesis method comprises the following steps:
   1) providing a compound 15;
   2) producing a compound 17 through removal of the hydroxyl protection group II from the compound 15; and
   3) producing the compound 18 through intramolecular oxidative dearomatization Heck reaction of the compound 17;
when R₁₁ is the hydrogen atom, the hydroxyl protection group I is introduced into the compound 15, and then step 2) and step 3) are performed.

In some examples, the hydroxyl protection group II is one selected from the group consisting of p-methoxybenzyl, benzyl, acetyl, benzoyl, tervalyl, tert-butyldimethylsilyl, tert-butyldiphenylsilyl, triisopropylsilyl and triethylsilyl.

In some examples, the halogen atom is one selected from the group consisting of chlorine atom, bromine atom and iodine atom.

In some examples, in step 2), a removal reagent for the removal of the hydroxyl protection group II is one or two selected from the group consisting of potassium carbonate, sodium methoxide, sodium hydroxide, potassium hydroxide, trifluoroacetic acid, hydrochloric acid, boron trichloride, acetic acid, tetrabutylammonium fluoride, tetraethyl ammonium fluoride, hydrobromic acid, potassium fluoride and cesium fluoride; and/or
in step 2), a reaction solvent for the removal of the hydroxyl protection group II is one or two selected from the group consisting of methanol, N,N-dimethylformamide, acetonitrile, tetrahydrofuran, dichloromethane and water; and/or in step 2), the reaction temperature for the removal of the hydroxyl protection group II is -20 to 90°C.

In some examples, in step 2), the removal reagent is potassium carbonate; and/or
in step 2), the reaction solvent is methanol; and/or
in step 2), the reaction temperature for the removal of the hydroxyl protection group II is 40-60°C.

In some examples, in step 2), the removal reagent is potassium fluoride; and/or
in step 2), the reaction solvent is acetonitrile and water; and/or
in step 2), the reaction temperature for the removal of the hydroxyl protection group II is 0-60°C.

In some examples, in step 3), the intramolecular oxidative dearomatization Heck reaction is performed in the presence of a reaction reagent and an alkali.

In some examples, in step 3), the reaction reagent is a complex, or a ligand II and a transition metal catalyst II.

In some examples, in step 3), the complex is one selected from the group consisting of Pd(PPh₃)₄, Pd(PPh₃)₂Cl₂, Pd(PtBu₃)₂, Pd(PCy₃)₂, Pd(PPhtBu₂)₂Cl₂, [1,2-bis(diphenylphosphoryl)ethane] palladium dichloride, [1,3-bis(diphenylphosphoryl)propane] palladium dichloride and [1,4-bis(diphenylphosphoryl)butane] palladium dichloride; and/or
the molar ratio of the compound 17 to the complex to the alkali is 1 :(0.025-0.2):(1-3).

In some examples, in step 3), the ligand II is as expressed by formula (II), or is a stereoisomer or tautomer of formula (II) or a phosphonium hydrogen halide corresponding to formula (II); 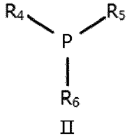 where
R₄ and R₅ are one selected from the group consisting of amaryl and tert-butyl;
R₆ is one selected from the group consisting of alkyl, cycloalkyl, aryl and heteroaryl, and the alkyl, cycloalkyl, aryl and heteroaryl are substituted by one or more independent hydrogen atoms, alkyl, halogen, cycloalkyl, aryl and heteroaryl respectively; and/or
in step 3), the transition metal catalyst II is one selected from the group consisting of [Pd(cinnamy)Cl]₂, [Pd(allyl)Cl]₂, Pd₂(dba)₃, Pd(OAc)₂, Pd(Tfa)₂, Pd(acac)₂, Pd(MeCN)₂Cl₂, Pd(PhCN)₂Cl₂, PdCl₂, Pd(Cp)(allyl), Pd(MeCN)₄(BF₄)₂, Pd(MeCN)₄(OTf)₂, Pd(cod)Cl₂, Pd(norbomadiene)Cl₂, Pd(TMEDA)Cl₂ and Pd(amphos)Cl₂; and/or
the molar ratio of the compound 17 to the ligand II to the transition metal catalyst II to the alkali is 1:(0.05-0.5):(0.05-0.15):(2-4).

In some examples, in step 3), the ligand II is selected from 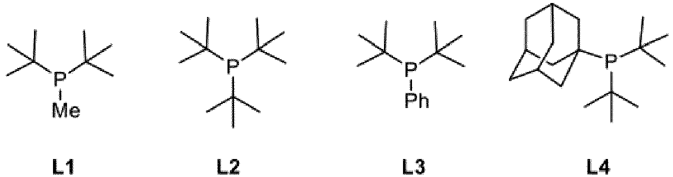 or 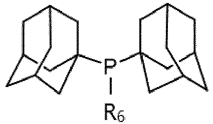 and phosphonium hydrogen halide 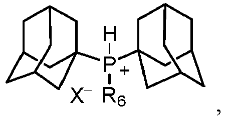 where R₆ is selected from the group consisting of C₁₋₂₀ alkyl or benzyl, and X is a halogen atom.

More preferably, in step 3), the ligand II is one selected from the following compounds:

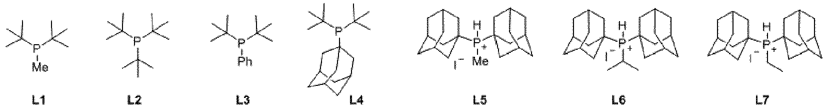

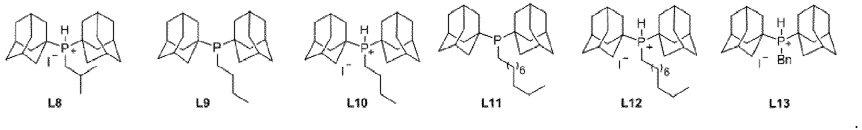

In some examples, in step 3), the alkali is one or two selected from the group consisting of potassium t-butoxide, lithium carbonate, sodium carbonate, cesium carbonate, silver carbonate, potassium bicarbonate, potassium carbonate, potassium borofluorite, potassium phosphate, dipotassium hydrogen phosphate, sodium tert-butanol, lithium tert-butanol, sodium hydride, potassium hydride, sodium acetate, sodium methoxide, sodium benzoate, potassium benzoate, pyridine, triethylamine, cesium fluoride, potassium hydroxide, and pivalate; and/or
in step 3), a reaction solvent for the intramolecular oxidative dearomatization Heck reaction is one selected from the group consisting of anisole, trifluorotoluene, N,N-dimethylformamide, N,N-dimethylacetamide, dimethyl sulfoxide, N-methylpyrrolidone, trimethylbenzene, dimethyl ether, ethanol, tert-butyl alcohol, methylbenzene, chlorobenzene, xylene, 1,4-dioxane, diethylene glycol dimethyl ether, methyl tert-butyl ether, tetrahydrofuran and ethylene glycol dimethyl ether; and/or in step 3), the concentration of the compound 17 in the intramolecular oxidative dearomatization Heck reaction is 0.05-2.5mol/L; and/or
in step 3), the temperature for the intramolecular oxidative dearomatization Heck reaction is 50-160°C.

In some examples, in step 3), the alkali is potassium phosphate and potassium carbonate; and/or
in step 3), the reaction solvent is one of N,N-dimethylformamide, N,N-dimethylacetamide and anisole; and/or
in step 3), the concentration of the compound 17 in the intramolecular Heck reaction is 0.05-1mol/L; and/or
in step 3), the temperature for the intramolecular Heck reaction is 60-150°C.

In some examples, a synthesis route of the compound 15 is as follows: 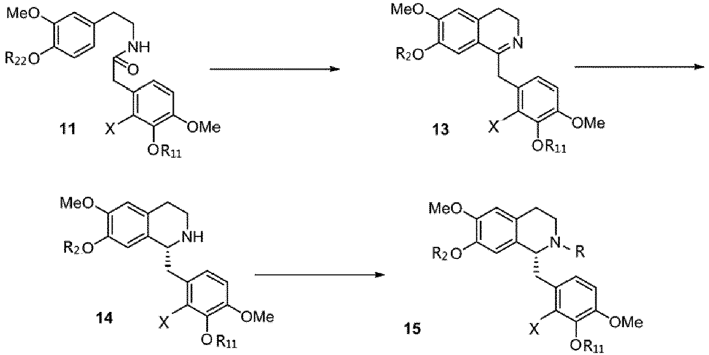
where R₂ is a hydroxyl protection group II, R₂₂ is a hydroxyl protection group II or a hydrogen atom, X is a halogen atom, and R₁₁ is a hydroxyl protection group I or a hydrogen atom;
when R₂₂ is the hydroxyl protection group II, a synthesis method includes the following steps:
   a. providing a compound 11;
   b. producing a compound 13 through Bischler-Napieralski reaction of the compound 11;
   c. producing a chiral tetrahydroisoquinoline type compound 14 through asymmetric transfer hydrogenation of the compound 13; and
   d. performing secondary amine protection on the compound 14 to produce the compound 15;
when R₂₂ is the hydrogen atom, the hydroxyl protection group II is introduced into the compound 11, and then steps b, c and d are performed.

In some examples, in step b, the Bischler-Napieralski reaction is performed in the presence of a condensation agent and an alkali; the molar ratio of the compound 11 to the condensation agent to the alkali is 1:(0.9-1.3):(1.5-2.5).

In some examples, the condensation agent is one selected from the group consisting of phosphine oxychloride, phosphorus pentoxide and trifluoromethyl sulfonic anhydride; and/or
in step b, the alkali is one selected from the group consisting of 2-fluoropyridine, pyridine, 4-dimethylaminopyridine, dimethylpyridine and triethylamine; and/or
in step b, a reaction solvent for the Bischler-Napieralski reaction is one selected from the group consisting of dichloromethane, dichloroethane, tetrahydrofuran and toluene; and/or
in step b, the temperature for the Bischler-Napieralski reaction is -50 to 40°C.

In some examples, in step b, the condensation agent is trifluoromethyl sulfonic anhydride; and/or
in step b, the alkali is 2-fluoropyridine; and/or
in step b, the reaction solvent is dichloromethane; and/or
in step b, the temperature for the Bischler-Napieralski reaction is -30 to 35°C.

In some examples, in step c, the asymmetric transfer hydrogenation reaction is performed in the presence of a chiral ligand I, a hydrogen source I and a metal catalyst I; the molar ratio of the compound 13 to the metal catalyst I to the chiral ligand I to the hydrogen source I is 1:(0.001-0.01):(0.002-0.02):(1.2-3).

In some examples, in step c, the chiral ligand I is one selected from the group consisting of 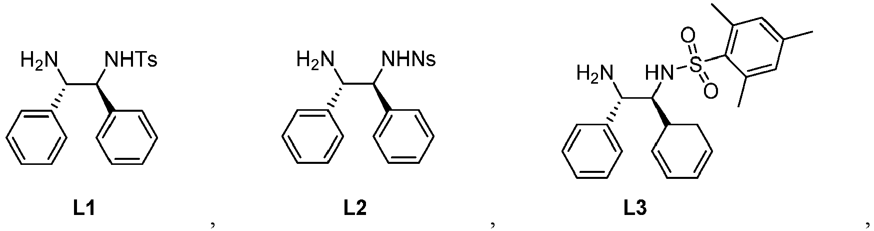 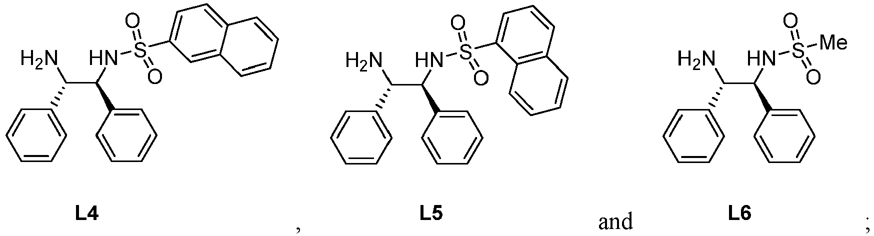 and/or
in step c, the hydrogen source I is one selected from the group consisting of formic acid, ammonium formate and a complex of formic acid and trialkylamine; and/or in step c, the metal catalyst I is one selected from the group consisting of 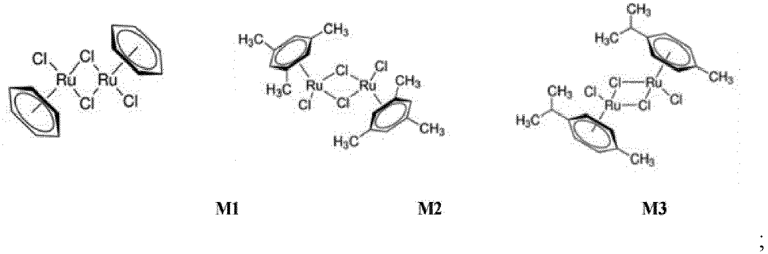 and/or
in step c, a reaction solvent for the asymmetric hydrogenation reaction is one selected from the group consisting of dichloromethane, dichloroethane, chloroform, tetrahydrofuran, dimethyl ether, tert-butyl methyl ether, trifluoroethanol, anisole, N,N-dimethylformamide, trifluorotoluene, N,N-dimethylacetamide, dimethyl sulfoxide, N-methylpyrrolidone, trimethylbenzene, ethanol, tert-butyl alcohol, toluene, chlorobenzene, xylene, 1,4-dioxane, dichlorobenzene, hexafluoroisopropanol, methanol and isopropanol; and/or
in step c, the temperature for the transfer hydrogenation reaction is -10 to 40°C.

In some examples, in step c, the hydrogen source I is a complex of methanol and triethylamine; and/or
in step c, the reaction solvent is N,N-dimethylformamide; and/or
in step c, the temperature for the transfer hydrogenation reaction is 0-35°C.

In some examples, in step d, the secondary amine protection is performed under an alkaline condition; an alkali used in the alkaline condition is one selected from the group consisting of disodium hydrogen phosphate, sodium dihydrogen phosphate, potassium carbonate, sodium carbonate, triethylamine, N,N-diisopropylethylamine, pyridine and 4-dimethylaminopyridine.

In some examples, in step d, the reaction temperature for the secondary amine protection is -10 to 50°C.

In some examples, a method for preparing the compound 11 comprises the following steps: providing a compound 9 and a compound 5, and performing amine acid condensation reaction to obtain the compound 11I, and the reaction formula is as follow: 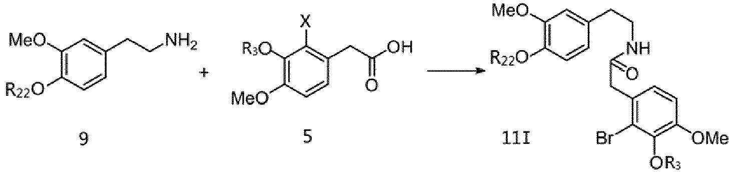 where R₃ is a methyl or hydrogen atom, X is a halogen atom, and R₂₂ is a hydrogen atom or a hydroxyl protection group II.

In some examples, the amine acid condensation reaction is performed in the presence of a condensation reagent and an alkali; the molar ratio of the compound 9 to the compound 5 to the condensation reagent to the alkali is (1-1.6): 1:(1-1.2):(1.5-3).

In some examples, the condensation reagent is one selected from the group consisting of O-benzotriazole-N,N,N',N'-tetramethylurea tetrafluoroboric acid, 1-ethyl-(3-dimethylaminopropyl)carbodiimide hydrochloride, 2-(7-azobenzotriazole)-N,N,N',N'-tetramethylurea hexafluorophosphate, dicyclohexylcarbodiimide and benzotriazole-1-yloxytris(dimethylamino) phosphonium hexafluorophosphate; and/or
the alkali is one selected from the group consisting of triethylamine, N,N-diisopropylethylamine, 4-dimethylaminopyridine and pyridine; and/or
the temperature for the amine acid condensation reaction is -10 to 50°C.

In some examples, the condensation reagent is O-benzotriazole - N,N,N',N'-tetramethylurea tetrafluoroboric acid; and/or
the alkali is triethylamine; and/or
the temperature for the amine acid condensation reaction is 0-25°C.

In some examples, a reaction formula for R₃ in the compound 11I to be substituted by the hydroxyl protection group I to obtain a compound 11II is as follow: 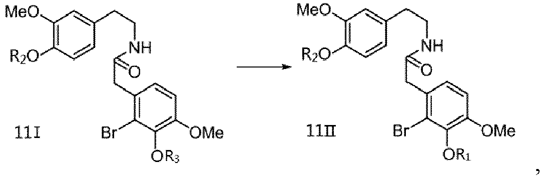 where R₁ is a hydroxyl protection group I and R₂ is a hydroxyl protection group II. It should be pointed out that the compound 11 includes all structural formulas of the compound 11I and compound 11II.

In some examples, a method for preparing the compound 9 includes the following steps: 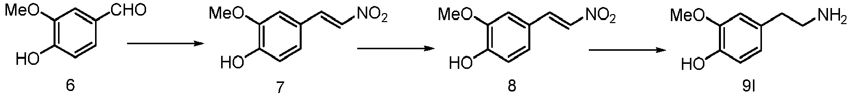
A. providing a compound 6;
B. producing a compound 7 through Henry reaction of the compound 6 and nitromethane;
C. producing a compound 8 through double bond reduction reaction of the compound 7; and
D. producing a compound 9I through nitro reduction reaction of the compound 8.

In some examples, in step B, the Henry reaction of the compound 6 and nitromethane is performed under the catalysis of an alkali, and the alkali is one or more of ethanediamine, ammonium acetate, sodium hydroxide, piperidine, diethylamine and morpholine.

In some examples, in step C, a reducing agent for the double bond reduction reaction is one selected from the group consisting of lithium aluminum hydride, sodium borohydride, palladium carbon+hydrogen, Raney nickel+hydrogen, lithium borohydride, Red-Al, zinc powder and iron powder; the Raney nickel+hydrogen mentioned in the present application refers to hydrogenation reduction using Raney nickel as a catalyst and hydrogen as a hydrogen source; palladium carbon+hydrogen refers to hydrogenation reduction using palladium carbon as a catalyst and hydrogen as a hydrogen source, which are commonly used reducing agents by those skilled in the art; and/or
in step C, a reaction solvent for the double bond reduction reaction is one selected from the group consisting of ethanol, ether, isopropyl ether, methyl tert-butyl ether, tetrahydrofuran, 1,4-dioxane, toluene, ethylene glycol dimethyl ether and diethylene glycol dimethyl ether; and/or
in step C, the temperature for the double bond reduction reaction is -10 to 10°C.

In some examples, in step C, the molar ratio of the compound 7 to the reducing agent is 1:(1-3); and/or
in step C, the reducing agent is sodium borohydride; and/or
in step C, the reaction solvent is ethanol and tetrahydrofuran; and/or
in step C, the temperature for the reduction reaction is 0°C.

In some examples, in step D, a reducing agent for the nitro reduction reaction is one selected from the group consisting of lithium aluminum hydride, sodium borohydride, palladium carbon+hydrogen, Raney nickel+hydrogen, lithium borohydride, Red-Al, zinc powder and iron powder; and/or
in step D, a reaction solvent for the nitro reduction reaction is one selected from the group consisting of ethanol, ether, isopropyl ether, methyl tert-butyl ether, tetrahydrofuran, 1,4-dioxane, toluene, ethylene glycol dimethyl ether and diethylene glycol dimethyl ether; and/or
in step D, the temperature for the nitro reduction reaction is -10 to 80°C.

In some examples, in step D, the molar ratio of the compound 8 to the reducing agent is 1:(1-3); and/or
in step D, the reducing agent is Raney nickel+hydrogen; and/or
in step D, the reaction solvent is ethanol; and/or
in step D, the temperature for the reduction reaction is 10-80°C.

In some examples, a compound 9II is further produced through hydroxyl protection reaction of the compound 9I, and the reaction formula is as follow: 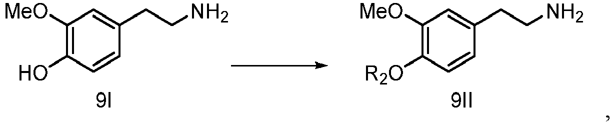 R₂ is a hydroxyl protection group II. It should be pointed out that the compound 9 includes all structural formulas of the compound 9I and the compound 9II.

In some examples, the hydroxyl protection reaction of the compound 9I is performed under an alkaline condition; an alkali used in the alkaline condition is one or two selected from the group consisting of 4-dimethylaminopyridine, sodium hydride, triethylamine, pyridine and imidazole; and/or,
a reaction solvent for the hydroxyl protection reaction of the compound 9I is one selected from the group consisting of dichloromethane, dichloroethane, tetrahydrofuran and toluene; and/or
the temperature for the hydroxyl protection reaction of the compound 9I is 0-40°C.

In some examples, a method for preparing the compound 5 includes the following steps: 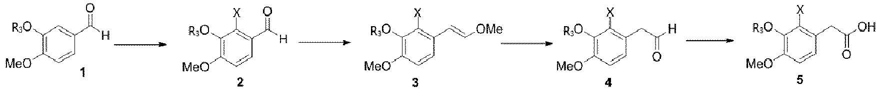 where R₃ is methyl or hydrogen atom
(1) providing a compound 1;
(2) producing a compound 2 through halogenation reaction of the compound 1;
(3) producing a compound 3 through Wittig reaction of the compound 2;
(4) producing a compound 4 through hydrolysis reaction of the compound 3; and
(5) producing the compound 5 through oxidization reaction of the compound 4.

The present application further provides application of the intermediate or method to the preparation of morphine and a morphine derivative.

In some examples, the morphine derivative includes one of codeine, oxycodone, hydrocodone, buprenorphine, nalaxone, naltrexone and nalbuphine.

In some examples, a method for producing codeine through further reaction of the intermediate I includes the following steps:
A) producing a compound 22 through hydrolysis reaction of the intermediate I under an acidic condition: 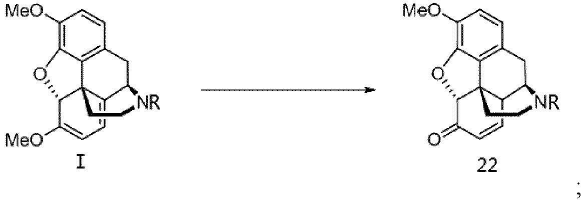
B) producing a compound 23 by removing a secondary amine protection group R from the compound 22 and reducing carbonyl: 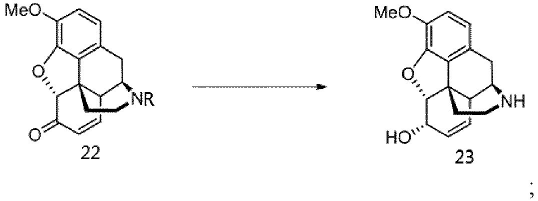 and
C) producing codeine 24 through reductive amine methylation reaction of the compound 23:

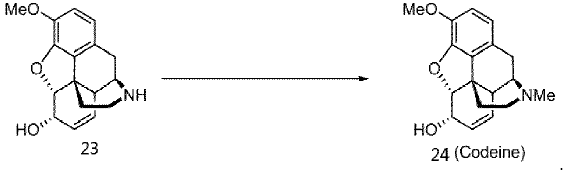

In some examples, in step A), an acid in the hydrolysis reaction is one or more selected from the group consisting of hydrogen bromide, sulfuric acid, boron trichloride, boron tribromide, silica gel, hydrochloric acid, p-toluene sulfonic acid, camphor sulfonic acid, acetic acid and trifluoroacetic acid; and/or
in step A), a solvent for the hydrolysis reaction is one or more selected from the group consisting of n-butyl ether, chloroform, isopropanol, ethanol, tetrahydrofuran, ethylene glycol dimethyl ether, methanol, dichloromethane and water; and/or in step A), the reaction temperature for the hydrolysis reaction is -40 to 80°C.

In some examples, in step B), a reaction reagent used in the process of removing the secondary amine protection group and reducing carbonyl is at least one selected from the group consisting of red aluminum, sodium-naphthalene, lithium aluminum hydride and magnesium powder; and/or
in step B), the molar ratio of the compound 23 to the reaction reagent is 1:(2-6); and/or
in step B), the reaction solvent used in the process of removing the secondary amine protection group and reducing carbonyl is at least one selected from the group consisting of tetrahydrofuran, ethylene glycol dimethyl ether and toluene; and/or
in step B), the reaction temperature is 25-80°C in the process of removing the secondary amine protection group and reducing carbonyl.

In some examples, in step C), a reaction reagent for the reductive amine methylation reaction is two or more selected from the group consisting of polyformaldehyde, formaldehyde aqueous solution and sodium borohydride; and/or
in step C), the molar ratio of the compound 22 to the reaction reagent is 1:(1-3); and/or
in step C), a reaction solvent for the reductive amine methylation reaction is at least one selected from the group consisting of methanol, ethanol, tetrahydrofuran and acetic acid; and/or
in step C), the reaction temperature for the reductive amine methylation reaction is 25-60°C.

In some examples, a method for producing oxycodone through further reaction of the intermediate I includes the following steps:
a) producing a compound 25 through oxa-D-A reaction of the intermediate I; the oxa-D-A reaction in the present application refers to the oxa-Diels-Alder reaction: 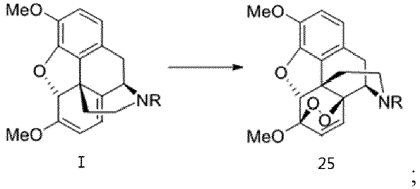
b) producing a compound 26 through catalytic hydrogenation reaction of the compound 25: 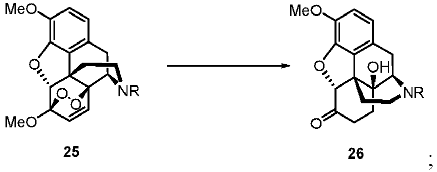
c) producing a compound 27 by removing the secondary amine protection group from the compound 26 and reducing carbonyl and alkenyl: 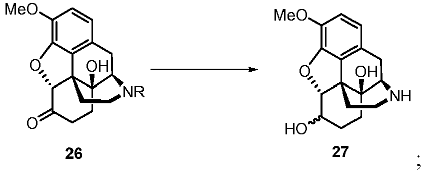
d) producing a compound 28 through reductive amine methylation reaction of the compound 27: 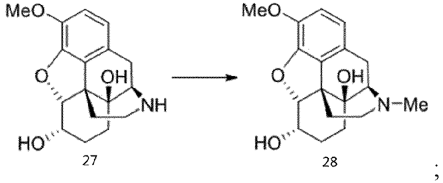 and
e) producing oxycodone 29 through selective oxidization reaction of the compound 28:

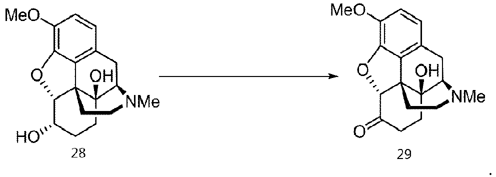

In some examples, in step a), the oxa-D-A reaction is performed under an illumination condition; a light source for the illumination condition is one selected from the group consisting of natural light and LED light.

In some examples, in step a), the oxa-D-A reaction is performed in the presence of an oxidant and a photocatalyst.

In some examples, in step a), the photocatalyst is tetraphenylporphyrin; and/or
in step a), the oxidant is one selected from the group consisting of oxygen, formic acid+hydrogen peroxide, acetic acid+hydrogen peroxide, tert-butyl peroxide and m-chloroperoxybenzoic acid; and/or
in step a), a reaction solvent for the oxa-D-A reaction is one selected from the group consisting of N,N-dimethylformamide, tetrahydrofuran, dichloromethane, dichloroethane and toluene; and/or
in step a), the temperature for the oxa-D-A reaction is 0-30°C.

In some examples, in step a), the molar ratio of the intermediate I to the photocatalyst is 1:(0.1-0.3); and/or
in step a), the oxidant is oxygen; and/or
in step a), the reaction solvent is dichloromethane; and/or
in step a), the temperature for the oxa-D-A reaction is 10-30°C.

In some examples, in step b), a catalyst for the catalytic hydrogenation reaction is one selected from the group consisting of palladium carbon, palladium chloride and palladium hydroxide; and/or
in step b), a reaction solvent for the catalytic hydrogenation reaction is one or more selected from the group consisting of methanol, ethanol, dichloromethane, dichloroethane, ethyl acetate, isopropanol, formic acid and water; and/or
in step b), the hydrogen pressure of the catalytic hydrogenation reaction is 1-25atm; and/or
in step b), the temperature for the catalytic hydrogenation reaction is 0-80°C.

In some examples, in step b), the molar ratio of the compound 25 to the catalyst is 1:(0.1-0.3); and/or
in step b), the catalyst is palladium carbon; and/or
in step b), the reaction solvent is isopropanol, water and formic acid; and/or
in step b), the hydrogen pressure of the catalytic hydrogenation reaction is 6-12atm; and/or
in step b), the temperature for the catalytic hydrogenation reaction is 10-40°C.

In some examples, in step c), a reaction reagent used in the process of removing the secondary amine protection group and reducing carbonyl and alkenyl is one selected from the group consisting of red aluminum, lithium aluminum hydride, magnesium powder and sodium naphthalene; and/or
in step c), a reaction solvent used in the process of removing the secondary amine protection group and reducing carbonyl and alkenyl is one selected from the group consisting of tetrahydrofuran, ethylene glycol dimethyl ether and toluene; and/or in step c), the reaction temperature is 25-80°C in the process of removing the secondary amine protection group and reducing carbonyl and alkenyl.

In some examples, in step c), the molar ratio of the compound 26 to the reaction reagent is 1 :(3-6); and/or
in step c), the reaction reagent is lithium aluminum hydride; and/or
in step c), the reaction solvent is tetrahydrofuran; and/or
in step c), the reaction temperature is 40-70°C.

In some examples, in step d), a reaction reagent for the reductive amine methylation reaction is one selected from the group consisting of polyformaldehyde+sodium borohydride and formaldehyde aqueous solution+sodium borohydride; and/or
in step d), a reaction solvent for the reductive amine methylation reaction is one selected from the group consisting of methanol, ethanol, tetrahydrofuran and acetic acid; and/or
in step d), the temperature for the reductive amine methylation reaction is 25-60°C.

In some examples, in step d), the molar ratio of the compound 27 to the reaction reagent is 1 :(2-5); and/or
in step d), the reaction reagent is polyformaldehyde+sodium borohydride; and/or
in step d), the reaction solvent is methanol; and/or
in step d), the temperature for the reductive amine methylation reaction is 10-40°C.

In some examples, in step e), an oxidant for the selective oxidization reaction is one selected from the group consisting of Dess-Martin oxidant, 2-iodobenzoic acid, oxalyl chloride+dimethyl sulfoxide and acetic anhydride; and/or
in step e), a reaction solvent for the selective oxidization reaction is one selected from the group consisting of dichloromethane, ethyl acetate and dimethyl sulfoxide; and/or in step e), the temperature for the selective oxidization reaction is -10 to 40°C.

In some examples, in step e), the molar ratio of the compound 28 to the oxidant is 1 :(3-5); and/or
in step e), the oxidant is Dess-Martin oxidant; and/or
in step e), the reaction solvent is dichloromethane; and/or
in step e), the temperature is 0-30°C.

In some examples, a method for producing naltrexone through further reaction of the intermediate I includes the following steps:
(1) preparing the compound 27 according to the application according to claim 53, and producing a compound 30 through reductive amination reaction of the compound 27 and cyclopropyl formaldehyde: 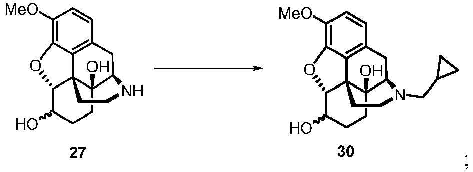
(2) producing a compound 31 through selective oxidization reaction of the compound 30: 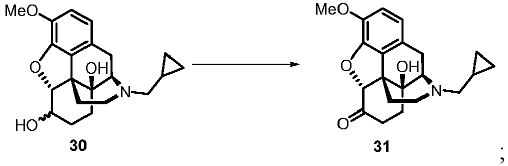 and
(3) producing naltrexone 32 through demethylation reaction of the comound 31:

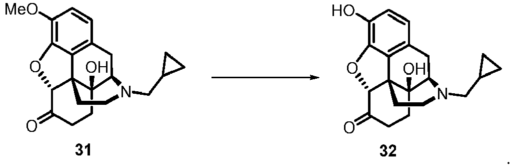

In some examples, in step (1), a reaction reagent for the reductive amination reaction is one selected from the group consisting of cyclopropyl formaldehyde+sodium borohydride, cyclopropyl formaldehyde+sodium cyanide borohydride, and cyclopropyl formaldehyde+sodium triacetoxyborohydride; and/or
in step (1), a reaction solvent for the reductive amination reaction is one selected from the group consisting of methanol, ethanol, tetrahydrofuran, 1,2-dichloroethane and acetic acid; and/or
in step (1), the temperature for the reductive amination reaction is 25-60°C.

In some examples, in step (1), the molar ratio of the compound 27 to the reaction reagent is 1 :(2-5); and/or
in step (1), the reaction reagent is cyclopropyl formaldehyde+sodium borohydride; and/or
in step (1), the reaction solvent is methanol; and/or
in step (1), the temperature for the reductive amination reaction is 10-40°C.

In some examples, in step (2), an oxidant for the selective oxidization reaction is one selected from the group consisting of Dess-Martin oxidant, 2-iodobenzoic acid, oxalyl chloride+dimethyl sulfoxide and acetic anhydride; and/or
in step (2), a reaction solvent for the selective oxidization reaction is one selected from the group consisting of dichloromethane, ethyl acetate and dimethyl sulfoxide; and/or
in step (2), the temperature for the selective oxidization reaction is -10 to 40°C.

In some examples, in step (2), the molar ratio of the compound 30 to the oxidant is 1:(3-5); and/or
in step (2), the oxidant is Dess-Martin oxidant; and/or
in step (2), the reaction solvent is dichloromethane; and/or
in step (2), the temperature is 0-30°C.

In some examples, in step (3), a demethylation reagent used in the demethylation reaction is one or two selected from the group consisting of boron tribromide, hydrobromic acid, oxalic acid, methanesulfonic acid, trifluoromethanesulfonic acid, phenylthiol, isopropyl mercaptan, sodium ethanethiolate, potassium t-butoxide, potassium hydroxide, potassium carbonate, potassium hydride, sodium hydride and sodium ethanol; and/or
in step (3), a reaction solvent for the demethylation reaction is one or two selected from the group consisting of dichloromethane, chloroform, water, methanol, ethanol, diethylene glycol, ethylene glycol, N-methylpyrrolidone, N,N-dimethylformamide, N,N-dimethylacetamide, toluene, chlorobenzene, and hexamethylphosphoric triamide; and/or
in step (3), the temperature for the demethylation reaction is -78 to 230°C.

In some examples, in step (3), the molar ratio of the compound 31 to the demethylation reagent is 1:(3-8); and/or
in step (3), the demethylation reagent is boron tribromide; and/or
in step (3), the reaction solvent is chloroform; and/or
in step (3), the temperature is 0-30°C.

In some examples, a method for producing nalaxone through further reaction of the intermediate I includes the following steps:
i. preparing a compound 27 according to the application according to claim 53, and producing a compound 33 through alkylation reaction of the compound 7 and allyl bromide: 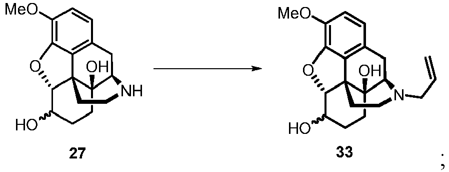
ii. producing a compound 34 through selective oxidization reaction of the compound 33: 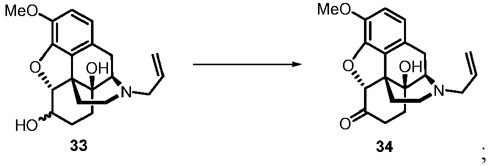 and
iii. producing nalaxone through demethylation reaction of the compound 34:

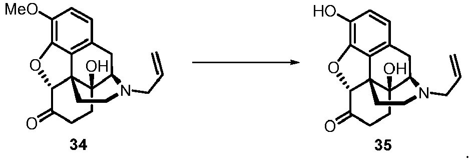

In some examples, in step i, an alkali for the nitrogen alkylation reaction is one or two selected from the group consisting of potassium carbonate, sodium carbonate, sodium bicarbonate, triethylamine and diisopropylethylamine; and/or
in step i, a solvent for the nitrogen alkylation reaction is one or two selected from the group consisting of nitromethylpyrrolidone, tetrahydrofuran, acetone and water; and/or
in step i, the temperature for the nitrogen alkylation reaction is 25-80°C.

In some examples, in step i, the molar ratio of the compound 27 to the allyl bromide to the alkali is 1 :(2-5):(1.2-2); and/or
in step i, the alkali is triethylamine; and/or
in step i, the reaction solvent is nitromethylpyrrolidone and water; and/or
in step i, the temperature for the nitrogen alkylation reaction is 50-70°C.

In some examples, in step ii, an oxidant for the selective oxidization reaction is one selected from the group consisting of Dess-Martin oxidant, 2-iodobenzoic acid, oxalyl chloride+dimethyl sulfoxide and acetic anhydride; and/or
in step ii, a reaction solvent for the selective oxidization reaction is one selected from the group consisting of dichloromethane, ethyl acetate and dimethyl sulfoxide; and/or in step ii, the temperature for the selective oxidization reaction is -10 to 40°C.

In some examples, in step ii, the molar ratio of the compound 33 to the oxidant is 1:(3-5); and/or
in step ii, the oxidant is Dess-Martin oxidant; and/or
in step ii, the reaction solvent is dichloromethane; and/or
in step ii, the temperature is 0-30°C.

In some examples, in step iii, a demethylation reagent used in the demethylation reaction is one or two selected from the group consisting of boron tribromide, hydrobromic acid, oxalic acid, methanesulfonic acid, trifluoromethanesulfonic acid, phenylthiol, isopropyl mercaptan, sodium ethanethiolate, potassium t-butoxide, potassium hydroxide, potassium carbonate, potassium hydride, sodium hydride and sodium ethylate; and/or
in step iii, a reaction solvent for the demethylation reaction is one or two selected from the group consisting of dichloromethane, chloroform, water, methanol, ethanol, diethylene glycol, ethylene glycol, N-methylpyrrolidone, N,N-dimethylformamide, N,N-dimethylacetamide, toluene, chlorobenzene, and hexamethylphosphoric triamide; and/or
in step iii, the temperature for the demethylation reaction is -78 to 230°C.

In some examples, in step iii, the molar ratio of the compound 31 to the demethylation reagent is 1:(3-8); and/or
in step iii, the demethylation reagent is boron tribromide; and/or
in step iii, the reaction solvent is chloroform; and/or
in step iii, the temperature is 0-30°C.

It should be understood that the total synthesis method in the present application is not limited to the synthesis of oxycodone or codeine, but also can be used for preparing other morphine derivatives (opioids) with similar structure, such as buprenorphine and dihydroetorphine, from the key intermediate I by using the synthesis method and route similar to that for the synthesis of nalaxone, naltrexone, oxycodone and codeine.

The present application has the following beneficial effects:
1. The present application provides an intermediate and, in particular, a representative total synthesis method of codeine, which enable the reaction substrate to be effectively converted into the expected product under the designed reaction conditions, and achieve an efficient synthesis effect with high chemical and optical yield (the shortest total of 12 steps from amine acid condensation reaction, the total yield of 29%, 99.9% ee).
2. The intermediate provided by the present application can be prepared into codeine through a few simple steps of conversion. At the same time, the prepared codeine can be further converted into morphine in one step, achieving the effect of high reaction yield and simple operation.
3. In the total synthesis method of oxycodone in the present application, the intramolecular oxidative dearomatization Heck reaction during the preparation of the intermediate is taken as the key reaction. Because of its high reaction activity at the reaction site, the reaction substrate and catalyst can be efficiently converted into the expected product after interaction, with a yield of 82%, which is significantly higher than that in the existing reported method.
4. The total synthesis method in the present application is simple in operation, and most of the synthesized intermediates can be directly used for subsequent reactions without further separation and purification, thus realizing the continuous operation of multi-step reactions. Specifically, compared with the literature report (Qin, Y CCS Chem. 2021, 3, 1376), the synthesis steps of codeine decrease from 17 steps to 12 steps, the yield increases from 14% to 29%, the synthesis steps of oxycodone decrease from 17 steps to 13 steps, the yield increases from 11% to 18%, the synthesis steps of nalaxone decrease from 21 steps to 14 steps, the yield increases from 5% to 14%, the synthesis steps of nalaxone decrease from 21 steps to 14 steps, and the yield increases from 5% to 13%, thus greatly improving the synthesis efficiency, shortening the synthesis path, reducing the discharge of three wastes and reducing the production cost. The cost of codeine synthesized by adopting the method provided by the present application is equivalent to the cost of planting and extraction; the cost of synthesized oxycodone, nalaxone, naltrexone and other morphine drugs containing hydroxy at 14-position is greatly lower than that of the traditional planting, extraction and semi-synthesis methods.
5. The total synthesis method in the present application has the advantages of mild reaction conditions, simple post-reaction treatment and easy in operation, and is suitable for the large-scale preparation of such drug.
6. The total synthesis method in the present application can also be used for the synthesis of other opioid drugs with similar structure, which is conducive to changing the current situation that existing opioid drugs mainly rely on the cultivation of opium poppy to obtain thebaine, and is conducive to the control and safe production of such drug.
7. When synthesizing codeine, oxycodone, nalaxone and naltrexone using the intermediate provided by the present application, the used reagents are common chemical reagents, which do not need to be prepared specially. At the same time, the reaction conditions are not strict, and the sensitivity to water, oxygen and other substances is not high. Moreover, the post-treatment of each reaction step is simple and highly operable.

### Description of the Embodiments

The technical solution of the present application will be further described in detail below, but the scope of protection of the present application is not limited thereto.

### Example 1 Method for Preparing Compound 5

Taking that R₃ was H and X was bromine as an example, a method for synthesizing a compound 5a included the following steps: 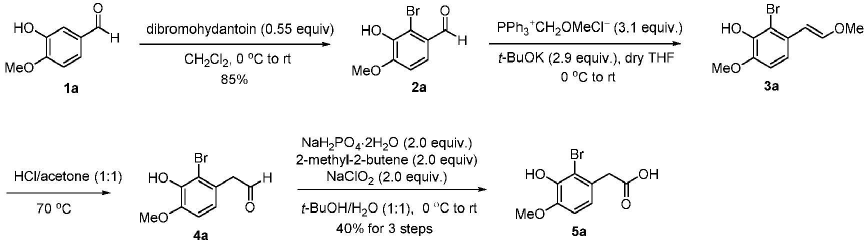 Isovanillin 1a (150g, 0.986mol, 1.0 equiv.) was dissolved in dichloromethane (2500mL). The solution was cooled to 0°C in an ice bath. Dibromohydantoin (155g, 0.542mol, 0.55 equiv.) was slowly added into the solution in batches under stirring. Then, the reaction solution was heated to room temperature for reaction. After TLC detected that the raw materials disappeared completely (about 4h), the reaction solution was cooled to 0°C. Saturated Na₂S₂O₃ aqueous solution (500mL) was added to quench the reaction. Stirring was performed for 1h at 0°C. After the precipitate was completely precipitated, filtering was performed. The filter cake was washed by using water (500mL*3). The obtained white solid 2a was collected, put in an oven for drying for 5h at 90°C, then subjected to vacuum drying for 6h at 50°C, and directly used for next reaction (193g, with yield of 85%).

Ph₃P+CH₂OMeCl⁻ (888g, 2.59mol, 3.1 equiv.) was dispersed in dry tetrahydrofuran (2500mL). The mixture was cooled to 0°C in an ice bath. Then, t-BuOK (272g, 2.42mol, 2.9 equiv.) was slowly added. The reaction solution turned orange red. Fierce stirring was performed for 45min. The compound 2a (193g, 0.835mol, 1.0 equiv.) obtained in the previous step was slowly added to the suspension in batches.

The temperature was naturally increased from 0°C to 20°C for reaction. After TLC monitored that the raw materials disappeared completely (about 1h), the reaction solution was cooled to 0°C again. Water (1000mL) was added to quench the reaction. An organic layer was separated. A water layer was extracted by using ethyl acetate (1000mL*3). The organic layers were combined. Drying was performed by using anhydrous sodium sulfate. Filtering and concentration were performed to obtain a crude brownish red compound 3a, which was directly used for subsequent reaction without purification.

The crude compound 3a was dissolved in 1M HCl/acetone (v/v=1:1, 3000mL) mixed solution. The solution was heated to 80°C for reaction. After TLC monitored that the raw materials disappeared completely (about 3h), the mixture was cooled to room temperature. Acetone was extracted under reduced pressure. Extraction was performed on the obtained mixture by using ethyl acetate (2000mL*3). Organic phases were combined. Drying was performed by using anhydrous sodium sulfate. Filtering was performed. The solvent was extracted under reduced pressure to obtain a crude brownish red compound 4a, which was directly used for subsequent reaction without purification.

The crude product 4a was dissolved in t-BuOH/H₂O (v:v=1:1, 2000mL). The solution was cooled to 0°C in an ice bath. NaH₂PO₄ • 2H₂O (261g, 1.67mol, 2.0 equiv.) was added in batches. Then, 2-methyl-2-butene (178mL, 1.67mol, 2.0 equiv.) was added. Stirring was performed for 10min at 0°C. Then, NaClO₂ (151g, 1.67mol, 2.0 equiv.) was slowly added in batches. Heat was fiercely released during reaction. Then, the temperature was naturally increased from 0°C to 20°C for reaction. After TLC detected that the raw materials disappeared completely (about 3h), the reaction solution was cooled to 0°C again. Saturated sodium bicarbonate aqueous solution was slowly added to quench the reaction until no bubbles were generated. Then, t-BuOH was removed under reduced pressure. Toluene (1000mL) was added. Fierce stirring was performed for 15min to separate a water layer. Washing was performed respectively by using toluene (1000mL*5) and dichloromethane (1000mL*5). The pH of the water layer was regulated to 5 by using 1M HCl. Then, extraction was performed by using ethyl acetate (1000mL*5). Organic layers were combined. Drying was performed by using anhydrous sodium sulfate for concentration to obtain a crude product. The crude product was dispersed into toluene (600mL). Stirring was performed for 2h at room temperature. Filtering was performed. The solid was collected and recrystallized by using isopropanol to obtain a white solid compound 5a (87.0g, with three-step yield of 40%).

### Example 2 Method for Preparing Compound 5

Taking that R₃ was Me and X was bromine as an example, a method for synthesizing a compound 5b included the following steps:

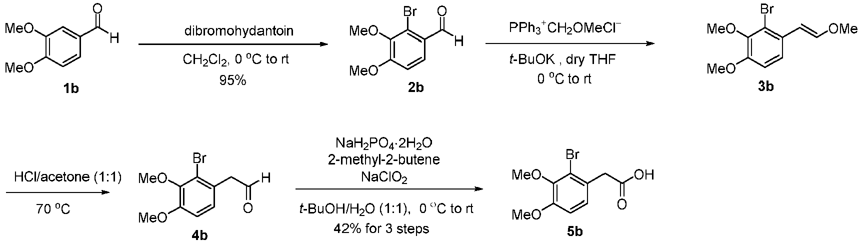

A compound 1b (150g, 0.903mol, 1.0 equiv.) was dissolved in dichloromethane (2500mL). The solution was cooled to 0°C in an ice bath. Dibromohydantoin (142 g, 0.496mol, 0.55 equiv.) was slowly added into the solution in batches under stirring. Then, the reaction solution was heated to room temperature for reaction. After TLC detected that the raw materials disappeared completely (about 4h), the reaction solution was cooled to 0°C. Saturated Na₂S₂O₃ aqueous solution (500mL) was added to quench the reaction. Stirring was performed for 1h at 0°C. After the precipitate was completely precipitated, filtering was performed. The filter cake was washed by using water (500mL*3). The obtained white solid 2a was collected, put in an oven for drying for 5h at 90°C, then subjected to vacuum drying for 6h at 50°C, and directly used for next reaction (210g, with yield of 95%).

Ph₃P+CH₂OMeCl⁻ (911g, 2.66mol, 3.1 equiv.) was dispersed in dry tetrahydrofuran (2500mL). The mixture was cooled to 0°C in an ice bath. Then, t-BuOK (279g, 2.48mol, 2.9 equiv.) was slowly added. The reaction solution turned orange red. Fierce stirring was performed for 45min. The compound 2b (210g, 0.857 mol, 1.0 equiv.) obtained in the previous step was slowly added to the suspension in batches. The temperature was naturally increased from 0°C to 20°C for reaction. After TLC monitored that the raw materials disappeared completely (about 1h), the reaction solution was cooled to 0°C again. Water (1000mL) was added to quench the reaction. An organic layer was separated. A water layer was extracted by using ethyl acetate (1000mL*3). The organic layers were combined. Drying was performed by using anhydrous sodium sulfate. Filtering and concentration were performed to obtain a crude brownish red compound 3b, which was directly used for subsequent reaction without purification.

The crude compound 3b was dissolved in 1M HCl/acetone (v/v=1:1, 3000mL) mixed solution. The solution was heated to 80°C for reaction. After TLC monitored that the raw materials disappeared completely (about 3h), the mixture was cooled to room temperature. Acetone was extracted under reduced pressure. Extraction was performed on the obtained mixture by using ethyl acetate (2000mL*3). Organic phases were combined. Drying was performed by using anhydrous sodium sulfate. Filtering was performed. The solvent was extracted under reduced pressure to obtain a crude brownish red compound 4b, which was directly used for subsequent reaction without purification.

The crude product 4b was dissolved in t-BuOH/H₂O (v:v=1:1, 2000mL). The solution was cooled to 0°C in an ice bath. NaH₂PO₄ • 2H₂O (267g, 1.71mol, 2.0 equiv.) was added in batches. Then, 2-methyl-2-butene (181mL, 1.71mol, 2.0 equiv.) was added. Stirring was performed for 10min at 0°C. Then, NaClO₂ (155g, 1.71mol, 2.0 equiv.) was slowly added in batches. Heat was fiercely released during reaction. Then, the temperature was naturally increased from 0°C to 20°C for reaction. After TLC detected that the raw materials disappeared completely (about 3h), the reaction solution was cooled to 0°C again. Saturated sodium bicarbonate aqueous solution was slowly added to quench the reaction until no bubbles were generated. Then, t-BuOH was removed under reduced pressure. Toluene (1000mL) was added. Fierce stirring was performed for 15min to separate a water layer. Washing was performed respectively by using toluene (1000mL*5) and dichloromethane (1000mL*5). The pH of the water layer was regulated to 5 by using 1M HCl. Then, extraction was performed by using ethyl acetate (1000mL*5). Organic layers were combined. Drying was performed by using anhydrous sodium sulfate for concentration to obtain a crude product. The crude product was dispersed into toluene (600mL). Stirring was performed for 2h at room temperature. Filtering was performed. The solid was collected and recrystallized by using isopropanol to obtain a white solid compound 5b (99.0g, with three-step yield of 42%).

### Example 3 Preparation of Compound 9I

Taking that R₂₂ was a hydrogen atom as an example, a compound 9a was synthesized through the following synthesis route:

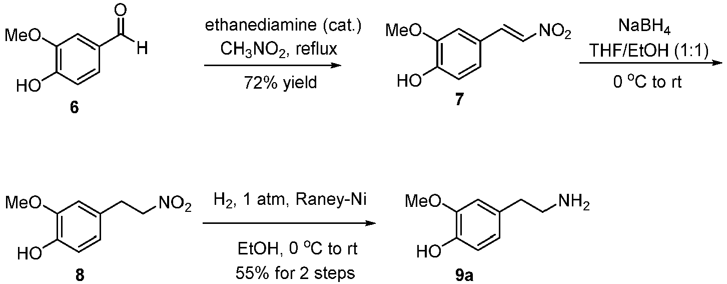

A method included the following steps:
Vanillin 6 (200g, 1.31mol, 1.0 equiv.) was dissolved in CH₃NO₂ (1000mL). Ethanediamine (1.0mL) was into the solution under stirring. Heating was performed for refluxing. After TLC monitored that the reaction was complete (about 2h), the reaction solution was cooled to room temperature. A large amount of yellow solids were precipitated. Filtering was performed. The filter cake was washed respectively by using methanol/water (v/v=1:1) (200mL*3) and anhydrous ethanol (200mL*2). The solid was collected and dried under reduced pressure through a water pump to obtain a compound 7 (bright yellow fine needle crystal, 185g, with yield of 72%).

The compound 7 (40.0g, 0.205mol, 1.0 equiv.) was dissolved in THF/EtOH (v/v=1:1, 480mL) mixed solution. The solution was cooled to 0°C in an ice bath. NaBH₄ (15.5g, 0.410mol, 2.0 equiv.) was slowly added in batches under stirring for reaction for 3h at 0°C. After TLC detected that the raw materials disappeared completely, acetic acid aqueous solution (CH₃COOH/H₂O, v/v=1:4, 250mL) was added to quench the reaction. Reduced pressure distillation was performed to remove the organic solvent. Extraction was performed on the residue by using ethyl acetate (300mL*3). Organic layers were combined. Drying was performed by using anhydrous magnesium sulfate. Filtering and concentration were performed. The obtained crude product was filtered through a silica gel pad (eluent PE:EA=2:1, v/v) to obtain a compound 8 (yellow oil), which was directly used for next reaction.

The compound 8 was dissolved in EtOH (400mL). Raney-Ni (about 4.0g) was added. The solution was put in a high-pressure hydrogenation kettle for reaction for 10h at room temperature under hydrogen pressure of 10 atm. After TLC detected that the raw materials disappeared completely, a large amount of solids were precipitated from the reaction solution. MeOH (300mL) was added. The mixture was heated to 70°C. Then, filtering was performed by using diatomite when the mixture was hot. The filter cake was washed by using MeOH (100mL*3). The filtrate was concentrated under reduced pressure until about 200mL of solvent were left. At this time, a large amount of solids have been precipitated. The solids were put at room temperature for cooling for 3h. Filtering was performed and a filter cake was collected. Vacuum drying was performed for 1h through a water pump (40°C). Then, vacuum drying was performed for about 0.5h through an oil pump to obtain a brownish solid compound 9a (22.2g, with two-step yield of 55%). **M.p.:** 139 -141□. **¹H NMR** (400 MHz, CDCl₃) *δ* 6.84 (d, *J* = 8.4 Hz, 1H), 6.72 - 6.67 (m, 2H), 3.87 (s, 3H), 2.94 (t, *J* = 6.8 Hz, 2H), 2.68 (t, *J* = 6.8 Hz, 2H). **¹³C NMR** (100 MHz, CDCl₃) *δ* 146.5, 144.0, 131.6, 121.4, 114.4, 111.3, 55.9, 43.6, 39.6. **IR** (neat): *ν*ₘₐₓ = 2512, 1610, 1496, 1469, 1232, 1153, 1128, 1033, 812 cm⁻¹. **HRMS (m/z):** [M + H]⁺ calculated for C₉H₁₄NO₂⁺, 168.1019; found, 168.1025.

### Example 4 Preparation of Compound 9II

Taking that R₂₂ was TBDPS as an example, a compound 9b was synthesized through the following synthesis route:

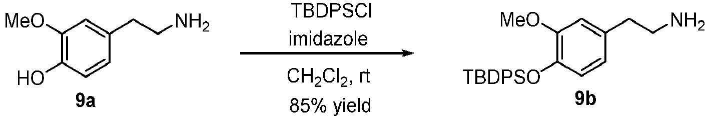

A compound 9a (20.0g, 0.120mol, 1.0 equiv.) and imidazole (12.2g, 0.179mol, 1.5 equiv.) were dissolved in dry CH₂Cl₂ (250mL). Stirring was performed for 10min at room temperature. Then, TBDPSCl (34.5g, 0.125mol, 1.05 equiv.) was added. After reaction for 5h at room temperature, TLC detected that the reaction was complete. Saturated NH₄Cl aqueous solution (300mL) was added to quench the reaction. The obtained mixture was filtered by using diatomite. The filtrate was layered. The water layer was extracted by using CH₂Cl₂ (100mL*2). The organic layers were combined. Washing was performed by using saturated NaCl aqueous solution (100mL*2). Drying was performed by using anhydrous magnesium sulfate. Filtering was performed. The filtrate was concentrated under reduced pressure. The obtained crude product were purified through silica gel column chromatography (dichloromethane/methanol=6:1, v/v, containing 0.5% of ammonia water) to obtain an oily compound 9b (41.3g, with yield of 85%). **¹H NMR** (400 MHz, CDCl₃) *δ* 7.72-7.70 (m, 4H), 7.41-7.32 (m, 6H), 6.64 (d, *J* = 8.0 Hz, 1H), 6.59 (s, 1H), 6.47 (dd, *J* = 8.0, 1.6 Hz, 1H), 3.55 (s, 3H), 2.88 (t, *J* = 6.8 Hz, 2H), 2.62 (t, *J* = 6.8 Hz, 2H), 1.11 (s, 9H). **¹³C NMR** (100 MHz, CDCl₃) *δ* 150.4, 143.5, 135.4, 134.8, 133.6, 132.5, 129.5, 127.6, 127.4, 120.6, 120.0, 113.0, 55.4, 43.2, 38.9, 26.7, 19.7. **IR** (neat): *ν*ₘₐₓ = 3053, 2933, 2858, 1587, 1513, 1264 cm⁻¹. **HRMS (m/z):** [M + H]⁺ calculated for C₂₅H₃₂NO₂Si⁺, 406.2197; found, 406.2190.

### Example 5 Preparation of Compound 9II

Taking that R₂₂ was TBS as an example, a compound 9c was synthesized through the following synthesis route: 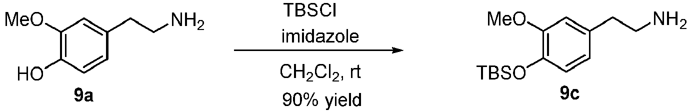 For the synthesis route, refer to example 4. The difference lay in that TBSCl was added and an oily compound 9c was obtained in example 5. **¹H NMR** (400 MHz, CDCl₃) *δ* 6.77 (d, *J* = 8.0 Hz, 1H), 6.68-6.63 (m, 2H), 3.79 (s, 3H), 2.93 (t, *J* = 6.8 Hz, 2H), 2.67 (t, *J* = 6.8 Hz, 2H), 1.30 (s, 2H), 0.99 (s, 9H), 0.14 (s, 6H). **¹³C NMR** (100 MHz, CDCl₃) *δ* 150.8, 143.3, 133.2, 120.9, 120.7, 112.8, 55.5, 43.6, 39.7, 25.7, 18.4, -4.66. **IR** (neat): *ν*ₘₐₓ= 2929, 2856, 1578, 1463, 1275, 1156, 1126, 1034, 838 cm⁻¹. **HRMS (m/z):** [M + H]⁺ calculated for C₁₅H₂₈NO₂Si⁺, 282.1884; found, 282.1881.

### Example 6 Preparation of Compound 11

Taking that R₂₂ was TBDPS and R₁₁ was a hydrogen atom as an example, a compound 11a was synthesized through the following synthesis route:

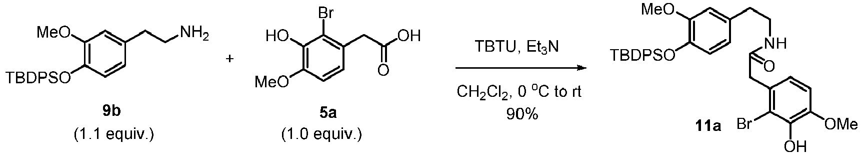

A method included the following steps:
A compound 9b (51.3g, 0.126mol, 1.1 equiv.), a compound 5a (30.0g, 0.115mol, 1.0 equiv.) and TBTU (44.3g, 0.138mol, 1.2 equiv.) were dissolved in dry CH₂Cl₂ (300mL). Triethylamine (40.0mL, 0.287mol, 2.5 equiv.) was added under the presence of an ice bath. Then, the temperature was increased to room temperature for reaction for 4h. After TLC detected that the raw materials disappeared completely, saturated ammonium chloride aqueous solution (300mL) was added to quench the reaction. The organic layer was separated. The water layer was extracted by using CH₂Cl₂ (400mL^{∗}1). The organic layers were combined. Washing was performed sequentially by using water (200mL^{∗}1) and saturated sodium chloride solution (100mL* 1). Drying was performed by using anhydrous magnesium sulfate. Filtering and reduced-pressure concentration were performed. The obtained crude product was dissolved by using ethyl acetate (300mL). Then, washing was performed sequentially by using 0.1M HCl (100mL*2), saturated NaHCO₃ (100mL*2), water (100mL*1) and saturated sodium chloride solution (100mL^{∗}1). Drying was performed by using anhydrous sodium sulfate. Filtering and concentration were performed. The obtained crude product was purified by silica gel column chromatography (petroleum ether/acetone=4:1, v/v) to obtain a white foam-like solid 11a (67.1g, with yield of 90%). **¹H NMR** (400 MHz, CDCl₃) *δ* 7.71-7.68 (m, 4H), 7.42-7.32 (m, 6H), 6.71 (q, *J* = 8.0 Hz, 2H), 6.56 (d, *J* = 8.0 Hz, 1H), 6.49 (d, *J* = 2.0 Hz, 1H), 6.29 (dd, *J* = 8.0, 2.0 Hz, 1H), 6.01 (s, 1H), 5.35 (t, *J* = 4.0 Hz, 1H), 3.84 (s, 3H), 3.59 (s, 2H), 3.50 (s, 3H), 3.38 (q, *J* = 6.0 Hz, 2H), 2.59 (t, *J* = 6.9 Hz, 2H), 1.10 (s, 9H).**¹³C NMR** (100 MHz, CDCl₃) *δ* 170.0, 150.6, 146.5, 143.7, 135.5, 133.8, 132.0, 129.7, 127.8, 127.6, 122.1, 120.7, 120.1, 112.9, 111.3, 109.9, 56.5, 55.5, 43.7, 40.8, 35.2, 26.8, 19.9. **IR** (neat): *ν*ₘₐₓ = 3297, 3050, 2932, 2857, 1650, 1605, 1512, 1488, 1111, 1034, 700 cm⁻¹. **HRMS (m/z):** [M + H]⁺ calculated for C₃₄H₃₉⁷⁹BrNO₅Si⁺, 648.1775; found, 648.1778; C₃₄H₃₉⁸¹BrNO₅Si⁺, 650.1755; found, 650.1763.

### Example 7 Preparation of Compound 11

Taking that R₂₂ was TBDPS, X was bromine and R₁₁ was Me as an example, a compound 11b was synthesized through the following synthesis route:

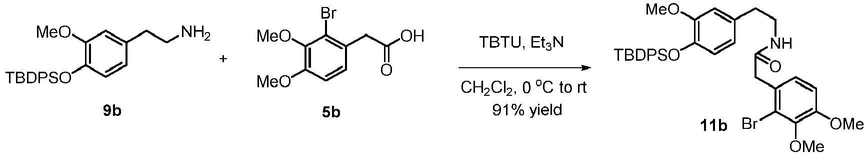

A compound 9b (64.9g, 0.160mol, 1.1 equiv.), a compound 5b (40.0g, 0.145mol, 1.0 equiv.) and TBTU (55.9g, 0.174mol, 1.2 equiv.) were dissolved in dry CH₂Cl₂ (400mL). Triethylamine (50.6mL, 0.364mol, 2.5 equiv.) was added in the presence of an ice bath. Then, the temperature was increased to room temperature for reaction. After TLC detected that the raw materials disappeared completely, saturated ammonium chloride aqueous solution (400mL) was added to quench the reaction. The organic layer was separated and the water layer was extracted by using CH₂Cl₂ (500mL*1). The organic layers were combined. Washing was performed sequentially by using water (300mL*1) and saturated sodium chloride solution (200mL*1). Drying was performed by using anhydrous magnesium sulfate. Filtering and reduced-pressure concentration were performed. The obtained crude product was dissolved by using ethyl acetate (400mL). Then, washing was performed sequentially by using 0.1M HCl (150mL*2), saturated NaHCO₃ (150mL*2), water (150mL*1) and saturated sodium chloride solution (150mL*1). Drying was performed by using anhydrous sodium sulfate. Filtering and concentration were performed. The obtained crude product was purified by silica gel column chromatography (petroleum ether/acetone=4:1, v/v) to obtain a white foam-like solid 11b (87.7g, with yield of 91%). **¹H NMR** (400 MHz,CDCl₃) *δ* 7.70-7.68 (m, 4H), 7.43-7.37 (m, 2H), 7.37-7.30 (m, 4H), 6.93 (d, *J=* 8.0 Hz, 1H), 6.76 (d, *J* = 8.0 Hz, 1H), 6.57 (d, *J* = 8.0 Hz, 1H), 6.51 (d, *J* = 1.8 Hz, 1H), 6.31 (dd, *J* = 8.0, 1.6 Hz, 1H), 5.38 (t, *J* = 4.8 Hz, 1H), 3.820 (s, 3H, overlapped), 3.818 (s, 3H, overlapped), 3.59 (s, 2H), 3.51 (s, 3H), 3.39 (q, *J* = 6.0 Hz, 2H), 2.60 (t, *J* = 6.8 Hz, 2H), 1.10 (s, 9H). **¹³C NMR** (100 MHz, CDCl₃) *δ* 169.8, 152.8, 150.5, 146.8, 143.6, 135.3, 133.6, 131.8, 129.6, 127.6, 127.4, 126.3, 120.7, 120.5, 120.0, 112.7, 111.5, 60.4, 56.0, 55.3, 43.6, 40.7, 35.1, 26.6, 19.7. **IR** (neat): *v*ₘₐₓ = 3311, 3052, 2934, 2858, 1663, 1512, 1486, 1265, 1034, 733, 701 cm⁻¹. **HRMS (m/z):** [M + H]⁺ calculated for C₃₅H₄₁79BrNO₅Si⁺, 662.1932; found, 662.1930; C₃₅H₄₁⁸¹BrNO₅Si⁺, 664.1911; found, 664.1922.

### Example 8 Preparation of Compound 11

Taking that R₂₂ was a hydrogen atom, X was bromine and R₁₁ was Me as an example, a compound 11c was synthesized through the following synthesis route:

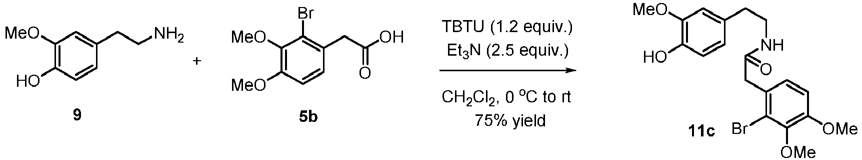

A compound 9a (2.00g, 0.012mol, 1.1 equiv.), a compound 5b (3.00g, 0.011mol, 1.0 equiv.) and TBTU (4.24g, 0.013mol, 1.2 equiv.) were dissolved in dry CH₂Cl₂ (30mL). Triethylamine (3.8mL, 0.027mol, 2.5 equiv.) was added in the presence of an ice bath.

Then, the temperature was increased to room temperature for reaction for 13h. After TLC detected that the raw materials disappeared completely, saturated ammonium chloride aqueous solution (30mL) was added to quench the reaction. The organic layer was separated. The water layer was extracted by using CH₂Cl₂ (20mL*3). The organic layers were combined. Washing was performed sequentially by using 1M HCl (50mL*2), saturated NaHCO₃ (50mL*1), and saturated sodium chloride solution (50mL^{∗}1). Drying was performed by using anhydrous magnesium sulfate. Filtering and reduced-pressure concentration were performed. The obtained crude product was purified by silica gel column chromatography (petroleum ether/acetone=3:1, v/v) to obtain a white foam-like solid 11c (3.5g, with yield of 75%). **¹H NMR** (400 MHz, CDCl₃) *δ* 6.96-6.50 (m, 5H), 5.83 (s, 1H), 5.52 (s, 1H), 3.85 (s, 6H, overlapped), 3.82-3.81 (m, 3H), 3.60 (m, 2H), 3.45-3.40 (m, 2H), 2.67-2.64 (m, 2H). **¹³C NMR** *δ* 169.9, 152.8, 146.7, 146.6, 144.2, 130.3, 127.5, 126.3, 121.2, 120.7, 114.3, 111.5, 111.0, 60.4, 56.0, 55.8, 43.6, 40.7, 35.1. **IR** (neat): *ν*ₘₐₓ = 3307, 1650, 1598, 1523, 1488, 1271, 1031 cm⁻¹. **HRMS (m/z):** [M + H]⁺ calculated for C₁₉H₂₃⁷⁹BrNO₅⁺, 424.0754; found, 424.0748; C₁₉H₂₃⁸¹BrNO₅⁺, 426.0734; found, 426.0731.

### Example 9 Preparation of Compound 11

Taking that R₂₂ was TBS, X was bromine and R₁₁ was a hydrogen atom as an example, a compound 11d was synthesized through the following synthesis route:

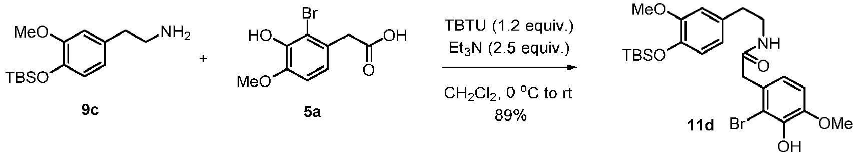

The synthesis route of the compound 11d is as shown by the figure above. For the synthesis operation, refer to the method in example 6. **¹H NMR** (400 MHz, CDCl₃) *δ* 6.77-6.73 (m, 2H), 6.67 (d, *J* = 8.0 Hz, 1H), 6.59-6.58 (m, 1H), 6.48-6.46 (m, 1H), 6.09 (s, 1H), 5.39 (m, 1H), 3.90 (s, 3H), 3.75 (s, 3H), 3.62 (s, 2H), 3.44 (q, *J* = 6.8 Hz, 2H), 2.66 (t, *J* = 6.8 Hz, 2H), 0.98 (s, 9H), 0.13 (s, 6H).**¹³C NMR** (100 MHz, CDCl₃) *δ* 169.9, 150.9, 146.4, 143.6, 143.5, 132.0, 127.6, 121.9, 120.8, 120.7, 112.5, 111.2, 109.7, 56.3, 55.4, 43.5, 40.6, 35.1, 25.7, 18.4, -4.69. **IR** (neat): *ν*ₘₐₓ = 3300, 2931, 2855, 1646, 1604, 1513, 1488, 1277, 1231, 1032 cm⁻¹. **HRMS (m/z):** [M + H]⁺ calculated for C₂₄H₃₅⁷⁹BrNO₅Si⁺, 524.1462; found, 524.1464; C₂₄H₃₅⁷⁹BrNO₅Si⁺, 526.1442; found, 526.1445.

### Example 10 Preparation of Compound 11

Taking that R₂₂ was TBS, X was bromine and R₁₁ was Me as an example, a compound 11e was synthesized through the following synthesis route:

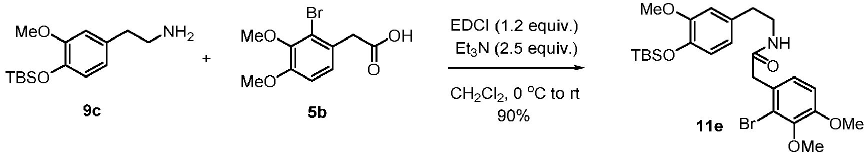

The synthesis route of the compound 11e is as shown by the figure above. For the synthesis operation, refer to the method in example 6. **¹H NMR** (400 MHz, CDCl₃) *δ* 6.96 (d, *J* = 8.4 Hz, 1H), 6.81 (d, *J* = 8.4 Hz, 1H), 6.69 (d, *J* = 8.0 Hz, 1H), 6.60 (d, *J* = 2.0 Hz, 1H), 6.48 (dd, *J* = 8.0, 2.0 Hz, 1H), 5.44 (t, *J* = 5.8 Hz, 1H), 3.87 (s, 3H), 3.84 (s, 3H), 3.75 (s, 3H), 3.61 (s, 2H), 3.45 (q, *J* = 6.6 Hz, 2H), 2.67 (t, *J* = 6.8 Hz, 2H), 0.98 (s, 9H), 0.13 (s, 6H). **¹³C NMR** (100 MHz, CDCl₃) *δ* 169.9, 152.8, 150.9, 146.8, 143.5, 132.0, 127.5, 126.3, 120.8, 120.7, 112.4, 111.5, 60.4, 56.0, 55.4, 43.7, 40.7, 35.1, 25.7, 18.4, -4.7. **IR** (neat): *ν*ₘₐₓ = 3055, 1669, 1512, 1264, 1036, 731 cm⁻¹. **HRMS (m/z):** [M + H]⁺calculated for C₂₅H₃₇⁷⁹BrNO₅Si⁺, 538.1619; found, 538.1622; C₂ₛH₃₇⁸¹BrNOₛSi⁺, 540.1598; found, 540.1603.

### Example 11 Preparation of Compound 12

When R₂₂ in the compound 11 was a hydrogen atom, a hydroxyl protection group II was introduced into the compound 11 to obtain a compound 12.

Taking that R₂₂ was a hydrogen atom, X was bromine, R₁₁ was Me and an introduced hydroxyl protection group II was Bn as an example, a compound 12ca was synthesized through the following synthesis route:

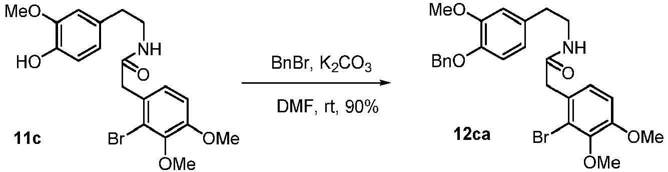

A compound 11c (830mg, 1.96mmol, 1.0 equiv.) and anhydrous potassium carbonate (540mg, 3.92mmol, 2.0 equiv.) were dissolved in dry DMF (8mL). Under the protection of argon, benzyl bromide (0.35mL, 2.94mmol, 1.5 equiv.) was added for reaction for 1h at room temperature. After TLC showed that the reaction of the raw materials was complete, water was added to quench the reaction. Ethyl acetate (10mL) was added. A large amount of solid was precipitated. Filtering was performed. The solid was washed by using methyl tert-butyl ether (10mL*2). The solid was collected and dried in vacuum to obtain a compound 12ca (white powder solid, 982mg, with yield of 90%). **M.p.:** 151-152□. **¹H NMR** (400 MHz, CDCl₃) *δ* 7.45-7.43 (m, 2H), 7.37 (t, *J* = 7.2 Hz, 2H), 7.32-7.28 (m, 1H), 6.97 (d, *J* = 8.4 Hz, 1H), 6.80 (d, *J=* 8.4 Hz, 1H), 6.75 (d, *J* = 8.0 Hz, 1H), 6.66 (d, *J* = 1.6 Hz, 1H), 6.52 (dd, *J* = 8.0, 1.6 Hz, 1H), 5.42 (m, 1H), 5.12 (s, 2H), 3.86 (s, 3H), 3.84 (s, 3H, overlapped), 3.84 (s, 3H, overlapped), 3.62 (s, 2H), 3.45 (q, *J* = 6.4 Hz, 2H), 2.68 (t, *J* = 7.2 Hz, 2H). **¹³C NMR** (100 MHz, CDCl₃) *δ* 170.0, 153.0, 149.9, 146.9, 137.4, 131.9, 128.6, 127.9, 127.7, 127.4, 126.5, 120.9, 120.8, 114.4, 112.5, 111.6, 71.3, 56.2, 56.1, 43.8, 40.8, 35.2. **IR** (neat): *ν*ₘₐₓ = 3304, 2936, 1642, 1592, 1515, 1487, 1453, 1266, 1230, 1034 cm⁻¹. **HRMS (m/z):** [M + H]⁺ calculated for C₂₆H₂₉⁷⁹BrNO₅⁺, 514.1224; found, 514.1219; C₂₆H₂₉⁸¹BrNO₅⁺, 516.1203; found, 516.1201.

### Example 12 Preparation of Compound 12

When R₂₂ in the compound 11 was a hydrogen atom, a hydroxyl protection group II was introduced into the compound 11 to obtain a compound 12.

Taking that R₂₂ was a hydrogen atom, X was bromine, R₁₁ was Me and an introduced hydroxyl protection group II was PMB as an example, a compound 12cb was synthesized through the following synthesis route:

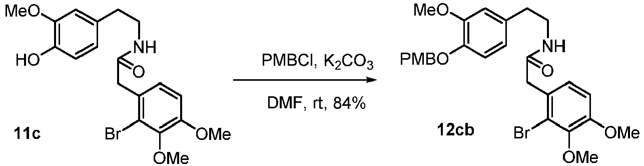

A compound 11c (674mg, 1.60mmol, 1.0 equiv.) and anhydrous potassium carbonate (442mg, 3.20mmol, 2.0 equiv.) were dissolved in dry DMF (8mL). Under the protection of argon, PMBCl (0.33mL, 2.40mmol, 1.5 equiv.) was added for reaction for 3h at room temperature. After TLC showed that the reaction of the raw materials was complete, water was added to quench the reaction. Ethyl acetate (10mL) was added. A large amount of solid was precipitated. Filtering was performed. The solid was washed by using methyl tert-butyl ether (10mL*2). The solid was collected and dried in vacuum to obtain a compound 12cb (white powder solid, 788mg, with yield of 84%). **M.p.:** 144-146 □. **¹H NMR** (400 MHz, CDCl₃) *δ* 7.36 (d, *J* = 8.8 Hz, 2H), 6.97 (d, *J* = 8.4 Hz, 1H), 6.90 (d, *J* = 8.4 Hz, 2H), 6.81 (d, *J* = 8.4 Hz, 1H), 6.76 (d, *J* = 8.0 Hz, 1H), 6.65 (d, *J* = 1.6 Hz, 1H), 6.53 (dd, *J* = 8.0, 1.6 Hz, 1H), 5.40 (m, 1H), 5.04 (s, 2H), 3.86 (s, 3H), 3.84(s , 3H), 3.83 (s, 3H), 3.81 (s, 3H), 3.62 (s, 2H), 3.45 (q, *J* = 6.4 Hz, 2H), 2.68 (t, *J* = 6.8 Hz, 2H).**¹³C NMR** (100 MHz, CDCl₃) *δ* 170.0, 159.5, 153.0, 150.0, 147.0, 131.9, 129.5, 129.2, 127.7, 126.5, 120.9, 120.8, 114.5, 114.1, 112.5, 111.7, 71.1, 56.2, 56.1, 55.4, 43.8, 40.9, 35.2. **IR** (neat): *ν*ₘₐₓ= 3313, 2932, 1646, 1591, 1515, 1249, 1033 cm⁻¹. **HRMS (m/z):** [M + H]⁺ calculated for C₂₇H₃₁⁷⁹BrNO₆⁺, 544.1329; found, 544.1325; C₂₇H₃₁⁸¹BrNO₆⁺, 546.1309; found, 546.1306.

### Example 13 Preparation of Compound 12

When R₂₂ in the compound 11 was a hydrogen atom, a hydroxyl protection group II was introduced into the compound 11 to obtain a compound 12.

Taking that R₂₂ was a hydrogen atom, X was bromine, R₁₁ was Me and an introduced hydroxyl protection group II was Ac as an example, a compound 12cc was synthesized through the following synthesis route:

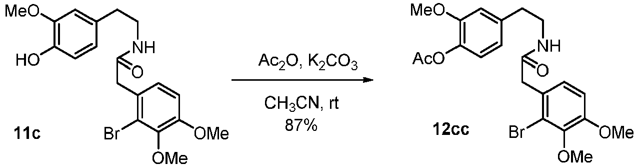

A compound 11c (1.00g, 2.36mmol, 1.0 equiv.) was dissolved in dry acetonitrile (20mL). Under the protection of argon, anhydrous potassium carbonate (651.5mg, 4.71mmol, 2.0 equiv.) and acetic anhydride (0.27mL, 2.83mmol, 1.2 equiv.) were added sequentially for reaction for 2h at room temperature. After TLC showed that the reaction of the raw materials was complete, water was added to quench the reaction. The water layer was extracted by using ethyl acetate (20mL*4). The organic layers were combined. Drying was performed by using anhydrous magnesium sulfate. Filtering and reduced-pressure concentration were performed to obtain a crude brown foam-like solid product. Methyl tert-butyl ether (5mL) was added. Stirring was performed for 20min at room temperature. Filtering was performed. The solid was collected to obtain a compound 12cc (gray white solid, 957mg, with yield of 87%). **M.p.:** 128-130□. **¹H NMR** (400 MHz, CDCl₃) *δ* 6.97 (d, *J* = 8.4 Hz, 1H), 6.88 (d, *J* = 8.0 Hz, 1H), 6.83 (d, *J* = 8.4 Hz, 1H), 6.72 (s, 1H), 6.66-6.61 (m, 1H), 5.47 (t, *J* = 6.0 Hz,, 1H), 3.87 (s, 3H), 3.84 (s, 3H), 3.78 (s, 3H), 3.63 (s, 2H), 3.51-3.43 (m, 2H), 2.74 (t, *J* = 7.2 Hz, 2H), 2.30 (s, 3H).**¹³CNMR** (100 MHz, CDCl₃) *δ* 170.0, 169.2, 152.9, 151.0, 146.8, 138.3, 137.7, 127.5, 126.4, 122.7, 120.8, 120.7, 112.8, 111.6, 60.5, 56.1, 55.9, 43.7, 40.6, 35.5, 20.7. **IR** (neat): *ν*ₘₐₓ= 3290, 2937, 1761, 1652, 1597, 1486, 1268, 1195, 1031 cm⁻¹. **HRMS (m/z):** [M + H]⁺ calculated for C₂₁H₂₅⁷⁹BrNO₆⁺, 466.0860; found, 466.0859; C₂₁H₂₅⁸¹BrNO₆⁺, 468.0839; found, 468.0840.

### Example 14 Preparation of Compound 12

When R₂₂ in the compound 11 was a hydrogen atom, a hydroxyl protection group II was introduced into the compound 11 to obtain a compound 12.

Taking that R₂₂ was a hydrogen atom, X was bromine, R₁₁ was Me and an introduced hydroxyl protection group II was Bz as an example, a compound 12cd was synthesized through the following synthesis route:

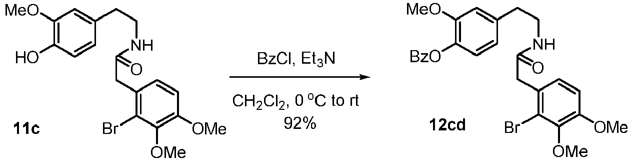

A compound 11c (1.00g, 2.36mmol, 1.0 equiv.) was dissolved in dry dichloromethane (20mL). Under the protection of argon, cooling to 0°C was performed. Triethylamine (0.66mL, 4.71mmol, 2.0 equiv.) and benzoyl chloride (0.33mL, 2.83mmol, 1.2 equiv.) were added sequentially. The temperature was increased to room temperature for reaction for 1h. After TLC showed that the reaction of the raw materials was complete, saturated ammonium chloride aqueous solution was added to quench the reaction. The water layer was extracted by using dichloromethane (20mL*4). The organic layers were combined. Drying was performed by using anhydrous magnesium sulfate. Filtering and reduced-pressure concentration were performed. The obtained crude product was purified by silica gel column chromatography (petroleum ether/acetone=10:1 to 2:1, v/v) to obtain a white solid 12cd (1.15g, with yield of 92%). **M.p.:** 145-147 □. **¹H NMR** (400 MHz, CDCl₃) *δ* 8.23-8.17 (m, 2H),7.68-7.58 (m, 1H), 7.54-7.47 (m, 2H), 7.03-6.96 (m, 2H), 6.85 (d, *J* = 8.4 Hz, 1H), 6.77 (d, *J* = 1.6 Hz, 1H), 6.69 (dd, *J* = 8.0, 2.0 Hz, 1H), 3.86 (s, 3H), 3.85 (s, 3H), 3.77 (s, 3H), 3.65 (s, 2H), 3.54-3.45 (m, 2H), 2.78 (t, *J* = 7.2 Hz, 2H).**¹³C NMR** (100 MHz, CDCl₃) *δ* 170.0, 164.8, 152.9, 151.3, 146.8,138.5, 137.7, 133.5, 130.2, 129.4, 128.5, 127.5, 126.4, 122.8, 120.8, 120.7, 112.9, 111.6, 60.5, 56.0, 55.9, 43.7, 40.6, 35.5. **IR** (neat): *ν*ₘₐₓ = 3055, 2939, 1736, 1665, 1598, 1510, 1487, 1264, 1033, 731, 704 cm⁻¹. **HRMS (m/z):** [M + H]⁺ calculated for C₂₆H₂₇⁷⁹BrNO₆⁺, 528.1012; found, 528.1016; C₂₆H₂₇⁸¹BrNO₆⁺, 530.0996; found, 530.0994.

### Example 15 Preparation of Compound 12

When R₂₂ in the compound 11 was a hydrogen atom, a hydroxyl protection group II was introduced into the compound 11 to obtain a compound 12.

Taking that R₂₂ was a hydrogen atom, X was bromine, R₁₁ was Me and an introduced hydroxyl protection group II was Piv as an example, a compound 12ce was synthesized through the following synthesis route:

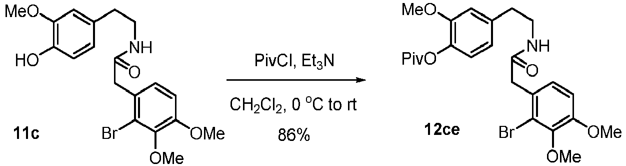

A compound 11c (1.00g, 2.36mmol, 1.0 equiv.) was dissolved in dry dichloromethane (20mL). Under the protection of argon, cooling to 0°C was performed. Triethylamine (0.66mL, 4.71mmol, 2.0 equiv.) and pivaloyl chloride (0.35mL, 2.83mmol, 1.2 equiv.) were added sequentially. The temperature was increased to room temperature for reaction for 2h. After TLC showed that the reaction of the raw materials was complete, saturated ammonium chloride aqueous solution was added to quench the reaction. The water layer was extracted by using dichloromethane (20mL*4). The organic layers were combined. Drying was performed by using anhydrous magnesium sulfate. Filtering and reduced-pressure concentration were performed. The obtained crude product was purified by silica gel column chromatography (petroleum ether/acetone=10:1 to 6:1, v/v) to obtain a white foam-like solid 12ce (1.03g, with yield of 86%). **¹H NMR** (400 MHz, CDCl₃) *δ* 6.96 (d, *J* = 8.8 Hz, 1H), 6.97-6.91 (m, 2H), 6.70 (d, *J* = 1.6 Hz, 1H), 6.63 (dd, *J* = 8.0, 2.0 Hz, 1H), 3.87 (s, 3H), 3.84 (s, 3H), 3.75 (s, 3H), 3.63 (s, 2H), 3.51-3.43 (m, 2H), 2.74 (t, *J* = 6.8 Hz, 2H), 1.35 (s, 9H). **¹³C NMR (100 MHz, CDCl₃)** *δ* 176.7, 170.0, 152.9, 151.2, 146.8, 138.7, 137.3, 127.5, 126.4, 122.6, 120.7, 120.7, 112.8, 111.6, 60.5, 56.0, 55.9, 43.6, 40.6, 39.0, 35.4, 27.2. **IR** (neat): *ν*ₘₐₓ= 2968, 1752, 1683, 1598, 1511, 1486, 1268, 1114, 1032 cm⁻¹. **HRMS (m/z):** [M + H]⁺ calculated for C₂₆H₂₇⁷⁹BrNO₆⁺, 528.1012; found, 528.1016; C₂₆H₂₇⁸¹BrNO₆⁺, 530.0996; found, 530.0994.

### Example 16 Preparation of Compound 15 (Bischler-Napieralski/Asymmetric Transfer Hydrogenation)

Taking that R₂ was TBDPS, X was bromine, R₁₁ was a hydrogen atom and R was CO₂Me as an example, a compound 15ab was synthesized through the following synthesis route:

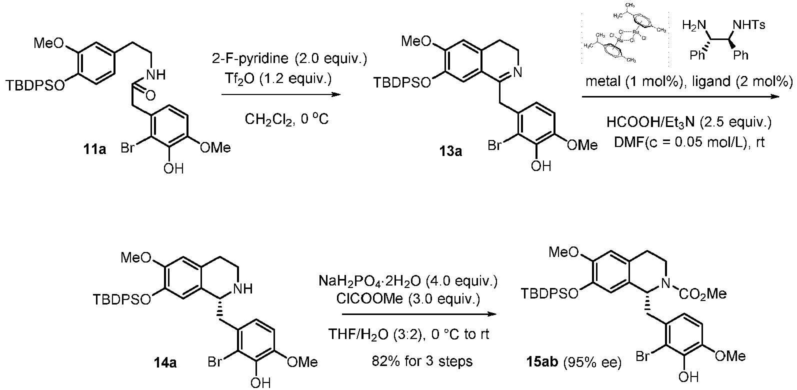

A solid compound 11a (100.0mg, 0.154mmol, 1.0 equiv.) was dissolved in dry CH₂Cl₂ (1mL). Under stirring at 0°C, 2-fluoropyridine (27uL, 0.308mmol, 2.0 equiv.) and trifluoromethyl sulfonic anhydride (32uL, 0.185mmol, 1.2 equiv.) were added sequentially. Then, the temperature was increased to room temperature for reaction for 10min. After TLC showed that the raw materials disappeared completely, the reaction solution was cooled to 0°C. Saturated NH₄Cl aqueous solution (1mL) was added to quench the reaction. The organic layer was separated. The water layer was extracted by using CH₂Cl₂ (2mL*3). The organic layers were combined. Washing was performed by using saturated NaCl (2mL*1). Drying was performed by using anhydrous magnesium sulfate. Filtering and reduced-pressure concentration were performed to obtain a crude product of a compound 13a, which was directly used for subsequent reaction without separation and purification.

The crude product 13a was dissolved in dry degassed DMF (2.9mL). Stirring was performed at room temperature. Another reaction flask was taken, added with a metal catalyst (1.0mg, 0.0154mmol, 0.01 equiv.) and a ligand (1S, 2S)-(+)-N-p-toluenesulfonyl-1,2-diphenylethylenediamine (1.2mg, 0.0308mmol, 0.02 equiv.). Air was extracted and changed. Under the protection of argon, degassed dry DMF (40uL) was added. Stirring was performed for 30min. The mixed solution was added into DMF solution of the compound 13a. Stirring was continuously performed for 10min at room temperature. Then, cooling to 0°C was performed. HCOOH/Et₃N (5:2 complex) (55uL, 0.385mmol, 2.5 equiv.) was added. The temperature was increased to room temperature for reaction for 17h at room temperature. After TLC detected that the reaction was complete, the reaction solution was cooled to 0°C. Saturated NaHCO₃ aqueous solution was added to quench the reaction. The pH was regulated to 9. The organic layer was separated. The water layer was extracted by using ethyl acetate (1mL*4). The organic layers were combined. Washing was performed sequentially by using water (2mL*1) and saturated NaCl (2mL*1). The organic layer was dried by using anhydrous magnesium sulfate. Filtering and reduced-pressure concentration were performed to obtain a crude product of a compound 14a, which was directly used for subsequent reaction without separation and purification.

The compound 14a was dissolved in THF/H₂O mixed solvent (2mL, v/v=3:2). Cooling to 0°C was performed. Sodium dihydrogen phosphate dihydrate (96.1mg, 0.616mmol, 4.0 equiv.) and methyl chloroformate (0.462mmol, 3.0 equiv.) were added. The temperature was increased to room temperature. Stirring was performed for reaction for 1h. After TLC showed that the raw materials disappeared completely, H₂O was added. Extraction was performed by using ethyl acetate (2mL*3). The organic layers were combined. Drying was performed by using anhydrous magnesium sulfate. Filtering and reduced-pressure concentration were performed. The obtained crude product was purified by silica gel column chromatography (petroleum ether/acetone=7:1, v/v) to obtain a white foam-like solid 15ab (87mg, with three-step total yield of 82%, ee=95%). HPLC conditions: OD-H column, Hexane: i-PrOH = 80:20, flow rate: 1mL/min, column temperature: 25°C, detection wavelength: 254nm, retention time of two enantiomers: *t*ₘₐⱼₒᵣ = 8.705min, *t*ₘᵢₙₒᵣ = 6.352min. **Optical rotation:** [α]_{D}²⁵ = -52.5 (*c* = 0.2, CHCl₃). **¹H NMR** (400 MHz, CDCl₃, some signals appeared in pairs due to amide rotational isomerism) *δ* 7.77-7.70 (m, 4H), 7.46-7.33 (m, 6H), 6.72 (d, *J* = 8.4 Hz, 1H), 6.69-6.60 (m, 1H), 6.53 (s, 0.8H), 6.53 (s, 0.2H), 6.46-6.44 (m, 1H), 5.92 (s, 0.8H), 5.87 (s, 0.2H), 5.16-5.12 (m, 0.2H), 5.05-5.01 (m, 0.8H), 4.28 (dd, *J* = 13.2, 4.8 Hz, 0.7H), 3.94-3.89 (m, 0.3H), 3.85-3.84 (m, 3H), 3.69 (s, 2.3H), 3.57 (d, *J* = 6.8 Hz, 1.4H), 3.22-3.15 (m, 1H), 3.12 (s, 2.3H), 2.94-2.77 (m, 2H), 2.68-2.49 (m, 2H), 1.13 (s, 7H), 1.11 (s, 2H, overlapped). **¹³C NMR** (100 MHz, CDCl₃, some signals appeared in pairs due to amide rotational isomerism) *δ* 156.0, 155.8, 149.4, 149.2, 145.5, 143.5, 143.3, 142.8, 142.7, 135.54, 135.47, 135.4, 135.35, 133.5, 133.4, 133.3, 130.9, 130.7, 129.75, 129.68, 129.64, 128.5, 128.3, 127.65, 127.62, 127.60, 127.53, 126.8, 126.6, 121.6, 121.5, 118.8, 118.4, 112.5, 112.1, 111.8, 111.2, 109.0, 56.3, 56.2, 55.8, 55.6, 54.2, 53.2, 52.4, 51.9, 42.0, 41.0, 38.3, 36.8, 29.7, 28.2, 26.71, 26.67, 19.72. **IR** (neat): *v*ₘₐₓ= 2928, 2856, 1692, 1609, 1488, 1463, 1262, 1106, 1033 cm⁻¹; **HRMS (m/z):** [M + H]⁺ calculated for C₃₆H₄₁⁷⁹BrNO₆Si⁺, 690.1881; found, 690.1880; C₄₁H₄₅⁸¹BrNO₆SSi⁺, 692.1861; found, 692.1868.

### Examples 17-21 Preparation of Compound 15 (Bischler-Napieralski/Asymmetric Transfer Hydrogenation)

Taking that R₂ was TBDPS, X was bromine, R₁₁ was a hydrogen atom and R was CO₂Me as an example, a compound 15ab was synthesized through the following synthesis route:

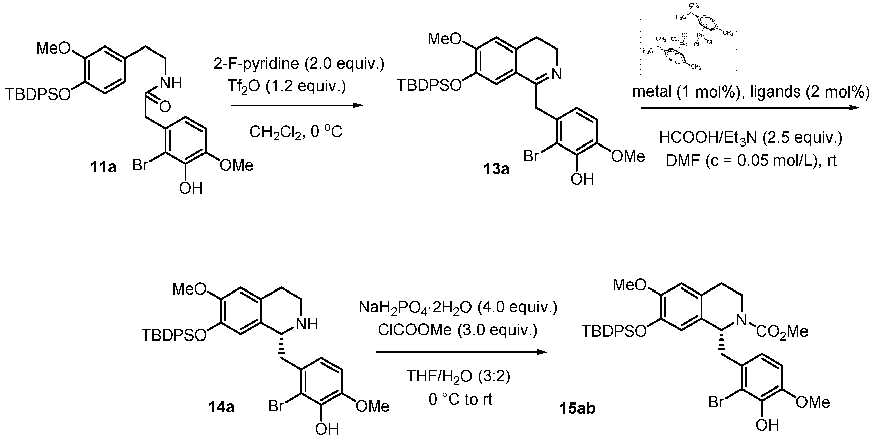

The synthesis steps in examples 17-21 were the same as those in example 16. The synthesis conditions such as temperature, time, reagent and amount were as shown in the synthesis route. The difference between the examples only lay in that different ligands were used in the asymmetric hydrogenation reaction for preparing the compound 14a from the intermediate 13a in the synthesis process. The results were as shown in the following table:

| Example | Ligand | Name | Yield % (three-step total yield from 11a to 15ab) | ee % |
|---|---|---|---|---|
| Example 17 | 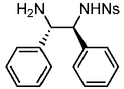 | (1S, 2S) - (+)-N-2,4-dinitrobenzenesulfonyl -1,2-diphenylethylenediamine | 78% | 93% (*R*) |
| Example 18 | 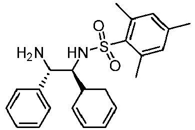 | (1S, 2S) - (+)-N-2,4,6-trimethylbenzenesulfonyl-1,2-diphenylethylenediamine | 77% | 92% (*R*) |
| Example 19 | 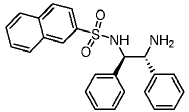 | (1S, 2S) - (+) - N-1-naphthalenesulfonyl-1,2-diphenylethylenediamine | 75% | 94% (*R*) |
| Example 20 | 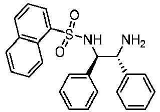 | (1S, 2S) - (+)-N-2-naphthalenesulfonyl-1,2-diphenylethylenediamine | 81% | 92% (*R*) |
| Example 21 | 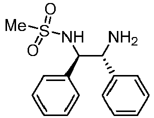 | (1R, 2R) - (+) - N-methanesulfonyl-1,2-diphenylethylenediamine | 79% | 84% (*S*) |

### Examples 22-25 Preparation of Compound 15 (Bischler-Napieralski/Asymmetric Transfer Hydrogenation)

Taking that R₂ was TBDPS, X was bromine, R₁₁ was a hydrogen atom and R was CO₂Me as an example, a compound 15ab was synthesized through the following synthesis route:

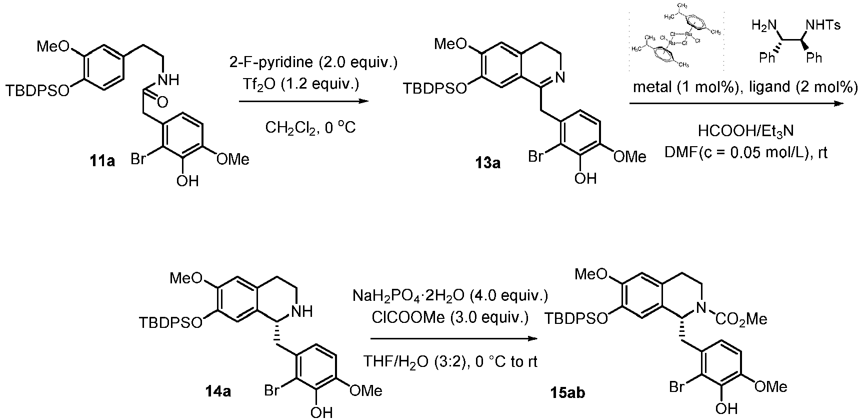

The synthesis steps of the compound 15ab in examples 22-25 were the same as those in example 16. The synthesis conditions such as temperature, time, reagent and amount were as shown in the synthesis route. The difference between the examples only lay in that the amount of HCOOH/Eₜ₃N in the asymmetric hydrogenation reaction for preparing the compound 14a from the intermediate 13a was different in the synthesis process. The results were as shown in the following table:

| Example | Amount of HCOOH/Et₃N (equiv.) | Yield % (three-step total yield from 11a to 15ab) | ee % |
|---|---|---|---|
| Example 22 | 3 | 74% | 94% (*R*) |
| Example 23 | 2 | 81% | 94% (*R*) |
| Example 24 | 1.5 | 71% | 95% (*R*) |
| Example 25 | 1.2 | 67% | 95% (*R*) |

### Examples 26-29 Preparation of Compound 15 (Bischler-Napieralski/Asymmetric Transfer Hydrogenation)

Taking that R₂ was TBDPS, X was bromine, R₁₁ was a hydrogen atom and R was CO₂Me as an example, a compound 15ab was synthesized through the following synthesis route:

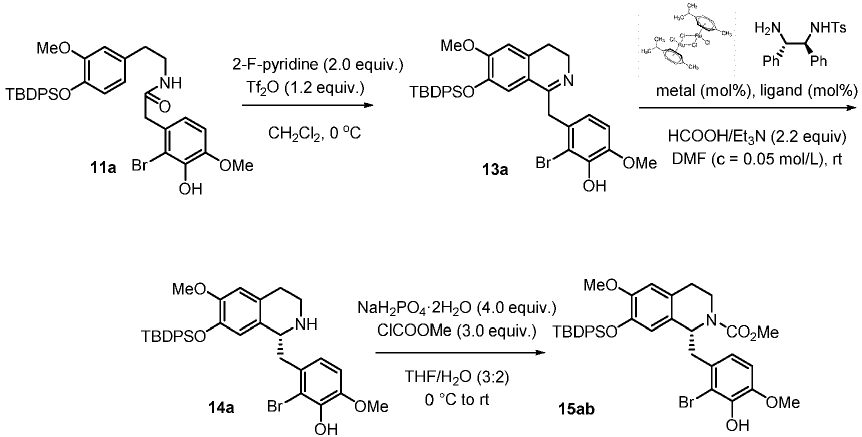

The synthesis steps of the compound 15ab in examples 26-29 were the same as those in example 16. The synthesis conditions such as temperature, time, reagent and amount were as shown in the synthesis route. The difference between the examples only lay in that the amount of the metal catalyst and the ligand in the asymmetric hydrogenation reaction for preparing the compound from the intermediate 13a was different in the synthesis process. The results were as shown in the following table:

| Example | Amount of metal catalyst (equiv.) | Amount of ligand (equiv.) | Yield % (three-step total yield from 11a to 15ab) | ee % |
|---|---|---|---|---|
| Example 26 | 0.75 | 1.5 | 78% | 95% (*R*) |
| Example 27 | 0.50 | 1 | 80% | 95% (*R*) |
| Example 28 | 0.25 | 0.5 | 71% | 90% (*R*) |
| Example 29 | 0.1 | 0.2 | 42% | 83% (*R*) |

### Examples 30-31 Preparation of Compound 15 (Bischler-Napieralski/Transfer Hydrogenation)

Taking that R₂ was TBDPS, X was bromine, R₁₁ was a hydrogen atom and R was CO₂Me as an example, a compound 15ab was synthesized through the following synthesis route:

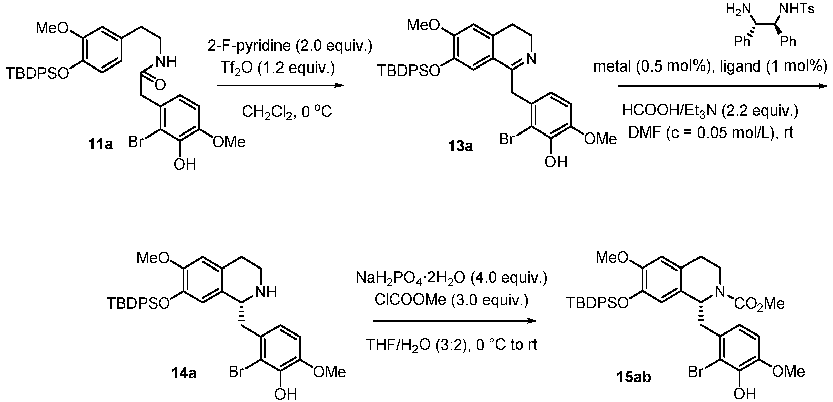

The synthesis steps of the compound 15ab in examples 30-31 were the same as those in example 16. The synthesis conditions such as temperature, time, reagent and amount were as shown in the synthesis route. The difference between the examples only lay in that the metal catalysts in the asymmetric hydrogenation reaction for preparing the compound 14a from the intermediate 13a were different in the synthesis process. The results were as shown in the following table:

| Example | Metal | Yield % (three-step total yield from 11a to 15ab) | ee % |
|---|---|---|---|
| Example 30 | 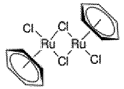 | 69% | 79% (*R*) |
| Example 31 | 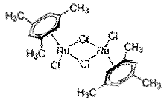 | 57% | 94% (*R*) |

### Examples 32-36 Preparation of Compound 15 (Bischler-Napieralski/Asymmetric Transfer Hydrogenation)

Taking that R₂ was TBDPS, X was bromine, R₁₁ was a hydrogen atom and R was CO₂Me as an example, a compound 15ab was synthesized through the following synthesis route:

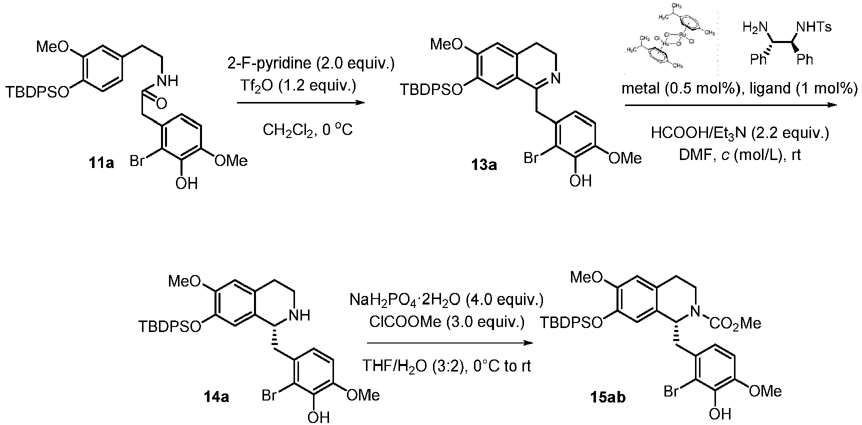

The synthesis steps of the compound 15ab in examples 32-36 were the same as those in example 16. The synthesis conditions such as temperature, time, reagent and amount were as shown in the synthesis route. The difference between the examples only lay in that the concentration for the asymmetric hydrogenation reaction for preparing the compound 14a from the intermediate 13a was different in the synthesis process. The results were as shown in the following table:

| Example | Concentration (mol/L) | Yield % (three-step total yield from 11a to 15ab) | ee % |
|---|---|---|---|
| Example 32 | 0.1 | 72% | 95% (*R*) |
| Example 33 | 0.15 | 73% | 94% (*R*) |
| Example 34 | 0.2 | 73% | 95% (*R*) |
| Example 35 | 0.3 | 70% | 96% (*R*) |
| Example 36 | 0.5 | 70% | 96% (*R*) |

### Example 37 Preparation of Compound 15 (Bischler-Napieralski/Asymmetric Transfer Hydrogenation)

Taking that R₂ was TBDPS, X was bromine, R₁₁ was a hydrogen atom and R was Ts as an example, a compound 15aa was synthesized through the following synthesis route:

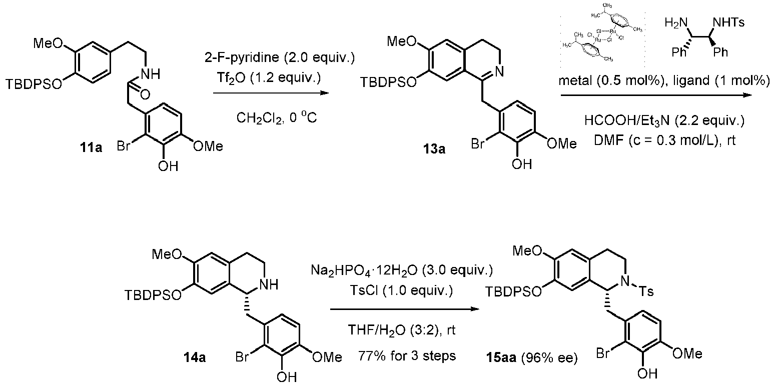

The solid compound 11a (10.00g, 15.42mmol, 1.0 equiv.) was dissolved in dry CH₂Cl₂ (100mL). Under stirring at 0°C, 2-fluoropyridine (2.65mL, 30.83mmol, 2.0 equiv.) and trifluoromethyl sulfonic anhydride (3.10mL, 18.50mmol, 1.2 equiv.) were added. The temperature was increased to room temperature for reaction for 10min. After TLC showed that the raw materials disappeared completely, the reaction solution was cooled to 0°C,. Saturated NH₄Cl aqueous solution (100mL) was added to quench the reaction. The organic layer was separated. The water layer was extracted by using CH₂Cl₂ (100mL^{∗}3). The organic layers were combined. Washing was performed by using saturated NaCl (50mL^{∗}1). Drying was performed by using anhydrous magnesium sulfate. Filtering and reduced-pressure concentration were performed to obtain a crude product of a compound 13a, which was directly used for subsequent reaction without separation and purification.

The crude product 13a was dissolved in dry degassed DMF (46mL). Stirring was performed at room temperature. Another reaction flask was taken and added with a metal catalyst (47.2mg, 0.077mmol, 0.005 equiv.) and a ligand (1S, 2S) - (+) - N-p-toluenesulfonyl-1,2-diphenylethylenediamine (56.5mg, 0.154mmol, 0.01 equiv.). Air was extracted. Under the protection of argon, degassed dry DMF (4mL) was added. Stirring was performed for 30min at room temperature. The mixed solution was added into DMF solution of a compound 12. Stirring was continuously performed for 10min at room temperature. Then cooling to 0°C was performed. HCOOH/Et₃N (5:2 complex) (4.90mL, 33.9mmol, 2.2 equiv.) was added. The temperature was increased to room temperature for reaction for 17h. After TLC detected that the reaction was complete, the reaction solution was cooled to 0°C. Saturated NaHCO₃ aqueous solution was added to quench the reaction. The pH was regulated to 9. The organic layer was separated. The water layer was extracted by using ethyl acetate (100mL^{∗}4). The organic layers were combined. Washing was performed sequentially by using water (50mL^{∗}1) and saturated NaCl (50mL^{∗}1). The organic layer was dried by using anhydrous magnesium sulfate. Filtering and reduced-pressure concentration were performed to obtain a crude product of a compound 14a, which was directly used for subsequent reaction without separation and purification.

The compound 14a was dissolved in THF/H₂O mixed solvent (150mL, v/v=3:2) Disodium hydrogen phosphate dodecahydrate (16.57g, 46.26mmol, 3.0 equiv.) and p-toluenesulfonyl chloride (2.94g, 15.42mmol, 1.0 equiv.) were added sequentially at room temperature. Stirring was performed at room temperature for reaction for 1h. After TLC showed that the raw materials disappeared completely, H₂O was added for dilution to an extent that the disodium hydrogen phosphate solid was dissolved. Extraction was performed by using ethyl acetate (100mL^{∗}3) . The organic layers were combined. Drying was performed by using anhydrous magnesium sulfate. Filtering and reduced-pressure concentration were performed. The obtained crude product was purified by silica gel column chromatography (petroleum ether/acetone=7:1, v/v) to obtain a white foam-like solid 15aa (9.34g, with three-step total yield of 77%, ee=96%). HPLC conditions: IC-H column, Hexane: *i*-PrOH =70:30, flowrate: 1mL/min, column temperature: 25°C, detection length: 254 nm, *t*ₘₐⱼₒᵣ = 27.883min, *t*ₘᵢₙₒᵣ = 21.832min. **Optical rotation:** [α]_{D}²⁵ = -119.5 (*c* = 0.44, CHCl₃); **¹H NMR** (400 MHz, CDCl₃) *δ* 7.74-7.71 (m, 4H), 7.47-7.33 (m, 6H), 7.30-7.26 (m, 2H), 6.95 (d, *J* = 8.0 Hz, 2H), 6.63 (d, *J* = 8.0 Hz, 1H), 6.51 (d, *J* = 10.4 Hz, 1H), 6.49 (s, 1H, overlapped), 6.35 (s, 1H), 5.83 (s, 1H), 4.81 (dd, *J* = 10.0, 4.4 Hz, 1H), 3.91 (dd, *J* = 14.4, 5.6 Hz, 1H), 3.86 (s, 3H), 3.59 (s, 3H), 3.49-3.42 (m, 1H), 2.80-2.71 (m, 2H), 2.69-2.58 (m, 1H), 2.43 (dd, *J* = 16.0 Hz, 2.4Hz, 1H), 2.31 (s, 3H), 1.12 (s, 9H); **¹³C NMR** (100 MHz, CDCl₃) *δ* 149.5, 145.7, 143.3, 142.8, 142.5, 137.4, 135.5, 135.5, 133.4, 133.4, 130.0, 129.8, 129.7, 128.9, 127.7, 127.6, 127.6, 127.0, 125.7, 122.1, 118.6, 112.4, 111.3, 109.0, 56.1, 55.6, 55.4, 42.5, 38.7, 26.7, 26.5, 21.4, 19.7; **IR** (neat): *v*ₘₐₓ = 3431, 2933, 2857, 1609, 1513, 1489, 1442, 1228, 1154, 1114, 1033, 702 cm⁻¹; **HRMS (m/z):** [M + H]⁺ calculated for C₄₁H₄₅⁷⁹BrNO₆SSi⁺, 786.1915; found, 786.1920; C₄₁H₄₅⁸¹BrNO₆SSi⁺, 788.1894; found, 788.1904.

### Example 38 Preparation of Compound 15 (Bischler-Napieralski/Asymmetric Transfer Hydrogenation)

Taking that R₂ was TBDPS, X was bromine, R₁₁ was a hydrogen atom and R was Cbz as an example, a compound 15ac was synthesized through the following synthesis route:

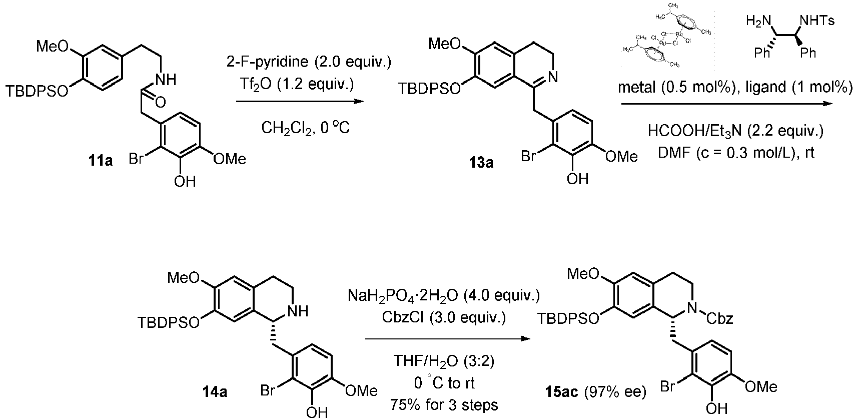

The synthesis route and reaction conditions of the compound 15ac were as shown in the figure above. The ligand used was (1S, 2S)-(+)-N-p-toluenesulfonyl-1,2-diphenylethylenediamine. For the synthesis operation, refer to the synthesis of 15ab (with three-step total yield of 75%, ee=97% *(R))*. HPLC conditions: C00C3-QG035 column, Hexane: *i*-PrOH = 80:20, flowrate: 1mL/min, column temperature: 25°C, detection wavelength: 254 nm, *t*ₘₐⱼₒᵣ = 10.179min, *t*ₘᵢₙₒᵣ = 8.966min. **Optical rotation**: [α]_{D}²⁵ = -69.0 (*c* = 0.68, CHCl₃); **¹H NMR** (400 MHz, CDCl₃) *δ* 7.75-7.72 (m, 4H), 7.43-7.22 (m, 9.6H), 7.02-7.01 (m, 1.4H), 6.61- 6.42 (m, 4H), 5.87-5.76 (m, 1H), 5.19-5.02 (m, 1H), 4.97-4.76 (m, 1H), 4.41 (d, *J* = 12.4 Hz, 1H), 4.34-3.98 (m, 1H), 3.82-3.81 (m, 3H), 3.68 (s, 2.3H), 3.59 (s, 0.7H), 3.33-3.19 (m, 1H), 2.92-2.47 (m, 4H), 1.13-1.11 (m, 9H); **¹³C NMR** (100 MHz, CDCl₃) *δ* 155.4, 155.0, 149.4, 149.2, 145.57, 145.55, 143.5, 143.4, 142.8, 142.7, 137.1, 136.1, 135.53, 135.49, 135.5, 135.4, 133.44, 133.40, 133.3, 130.8, 130.7, 129.73, 129.70, 129.65, 128.5, 128.4, 128.3, 128.2, 127.8, 127.7, 127.64, 127.62, 127.60, 127.5, 126.7, 126.6, 121.54, 121.51, 118.8, 118.5, 112.5, 112.2, 111.8, 111.4, 109.00, 108.9, 66.9, 66.6, 56.2, 55.8, 55.6, 54.3, 53.5, 41.9, 41.0, 38.4, 36.9, 28.3, 28.1, 26.70, 26.68, 19.7; **IR** (neat): *v*ₘₐₓ = 2961, 2857, 1688, 1487, 1428, 1261, 1100, 1031, 753, 700 cm⁻¹; **HRMS (m/z):** [M + H]⁺ calculated for C₄₂H₄₅⁷⁹BrNO₆Si⁺, 766.2194; found,766.2194; C₄₂H₄₅⁸¹BrNO₆Si⁺, 768.2174; found, 768.2187.

### Example 39 Preparation of Compound 15 (Bischler-Napieralski/Asymmetric Transfer Hydrogenation)

Taking that R₂ was TBS, X was bromine, R₁₁ was a hydrogen atom and R was CO₂Me as an example, a compound 15da was synthesized through the following synthesis route:

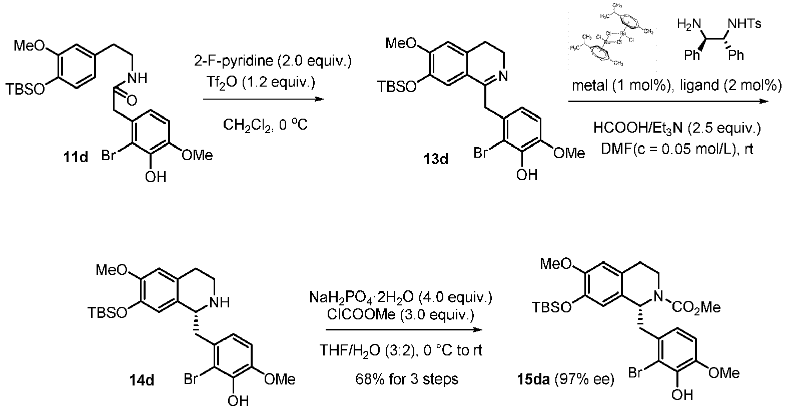

The synthesis route and reaction conditions of the compound 15da were as shown in the figure above. For the synthesis operation, refer to the synthesis of the compound 15ab in example 16. The ligand used was (1R, 2R)-(+)-N-p-toluenesulfonyl-1,2-diphenylethylenediamine (with three-step total yield of 69%, ee=97% *(S))*. HPLC conditions: OD-H column, Hexane: *i*-PrOH = 85:15, flowrate: 0.5mL/min, column temperature: 25°C, detection wavelength: 254 nm, *t*ₘₐⱼₒᵣ = 15.247min, *t*ₘᵢₙₒᵣ = 18.434min. **Optical rotation:** [α]_{D}²⁵ = + 55.3 (*c* = 2.0, CHCl₃); **¹H NMR** (400 MHz, CDCl₃, some signals appeared in pairs due to amide rotational isomerism) *δ* 6.73-6.67 (m, 2H), 6.58-6.43 (m, 1.7H), 6.43 (s, 0.3H), 5.99-5.91 (m, 1H), 5.35-5.29 (m, 1H), 4.30-4.25 (m, 0.7H), 3.91-3.87 (m, 3.3H, overlapped), 3.76-3.77 (m, 3H), 3.64 (s, 0.8H), 3.48-3.42 (m, 0.3H), 3.35-3.28 (m, 0.7H, overlapped), 3.26 (s, 2.2H), 3.24-3.07 (m, 1H), 2.98-2.84 (m, 1H), 2.81-2.65 (m, 1H), 1.00 (s, 6H), 0.95 (s, 3H), 0.16-0.08 (m, 6H);**¹³C NMR** (100 MHz, CDCl₃, some signals appeared in pairs due to amide rotational isomerism) *δ* 156.1, 156.0, 149.81, 149.76, 145.7, 145.6, 143.4, 143.1, 143.0, 142.8, 130.79, 130.76, 128.6, 127.2, 127.0, 121.80, 121.75, 119.6, 119.1, 112.1, 111.9, 111.8, 111.3, 109.3, 109.2, 56.3, 56.2, 55.6, 54.9, 53.6, 52.5, 52.1, 42.3, 41.1, 39.0, 37.4, 28.2, 25.72, 25.67, 18.5, 18.4, -4.8, -4.8, -4.7, -4.6; **IR** (neat): *v*ₘₐₓ = 2930, 2856, 1685, 1512, 1488, 1260, 1226, 1033, 755 cm⁻¹; **HRMS (m/z):** [M + H]⁺ calculated for C₂₆H₃₇⁷⁹BrNO₆Si⁺, 566.1568; found, 566.1565; C₂₆H₃₇⁸¹BrNO₆Si⁺, 568.1548; found, 568.1545.

### Example 40 Preparation of Compound 15 (Bischler-Napieralski/Asymmetric Transfer Hydrogenation)

Taking that R₂ was TBDPS, X was bromine, R₁₁ was Me and R was Ts as an example, a compound 15ba was synthesized through the following synthesis route:

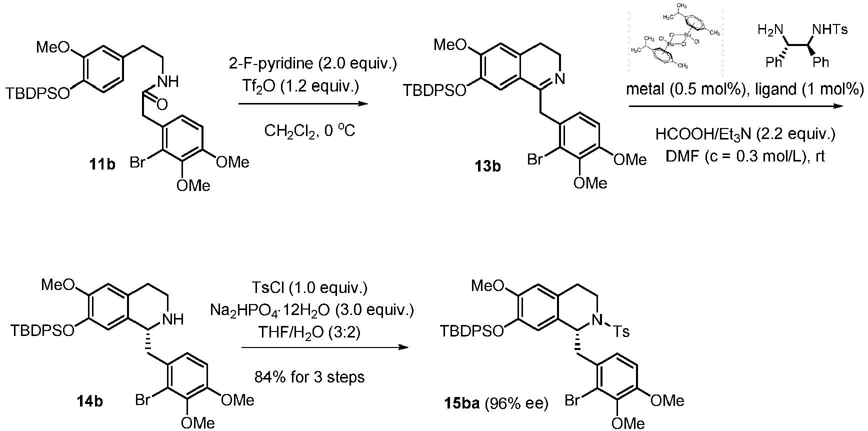

The solid compound 11b (30.00g, 45.27mmol, 1.0 equiv.) was dissolved in dry CH₂Cl₂ (300mL). Under stirring at 0°C, 2-fluoropyridine (7.8mL, 90.54mmol, 2.0 equiv.) and trifluoromethyl sulfonic anhydride (9.2mL, 54.32mmol, 1.2 equiv.) were added sequentially. Then, the temperature was increased to room temperature for reaction for 10min. After TLC showed that the raw materials disappeared completely, the reaction solution was cooled to 0°C. Saturated NH4Cl aqueous solution (300mL) was added to quench the reaction. The organic layer was separated. The water layer was extracted by using CH₂Cl₂ (300mL^{∗}3). The organic layers were combined. Washing was performed by using saturated NaCl (100mL^{∗}1). Drying was performed by using anhydrous magnesium sulfate. Filtering and reduced-pressure concentration were performed to obtain a crude product of a yellow foam-like solid 13b, which was directly used for subsequent reaction without separation and purification.

The crude product 13b was dissolved in dry degassed DMF (140mL). Stirring was performed at room temperature. Another reaction flask was taken. A metal catalyst (139mg, 0.226mmol, 0.005 equiv.) and a ligand (1S, 2S)-(+)-N-p-toluenesulfonyl-1,2-diphenylethylenediamine (166mg, 0.453mmol, 0.01equiv.) were added. Air was extracted and changed. Under the protection of argon, degassed dry DMF (10mL) was added. Stirring was performed for 30min at room temperature. The mixed solution was added into DMF solution of a compound 13b. Stirring was continuously performed for 10min at room temperature. Then, cooling to 0°C was performed. HCOOH/Et₃N (5:2 complex) (14.2mL, 99.6mmol, 2.2 equiv.) was added. The temperature was increased to room temperature for reaction for 17h. After TLC detected that the reaction was complete, the reaction solution was cooled to 0°C. Saturated NaHCO₃ aqueous solution was added to quench the reaction. The pH was regulated to 9. The organic layer was separated. The water layer was extracted by using ethyl acetate (300mL^{∗}3). The organic layers were combined. Washing was performed sequentially by using water (50mL^{∗}1) and saturated NaCl (50mL^{∗}1). The organic layer was dried by using anhydrous magnesium sulfate. Filtering and reduced-pressure concentration were performed to obtain a crude product of a black foam-like solid 14b, which was directly used for subsequent reaction without separation and purification.

The compound 14b was dissolved in THF/H₂O mixed solvent (300mL, v/v=3:2). Disodium hydrogen phosphate dodecahydrate (48.64g, 135.8mmol, 3.0 equiv.) and p-toluenesulfonyl chloride (8.63g, 45.27mmol, 1.0 equiv.) were added sequentially at room temperature. Stirring was performed for reaction for 1h at room temperature. After TLC showed that the raw materials disappeared completely, H₂O was added for dilution to an extent that the disodium hydrogen phosphate solid was dissolved. Extraction was performed by using ethyl acetate (200mL^{∗}3). The organic layers were combined. Drying was performed by using anhydrous magnesium sulfate. Filtering and reduced-pressure concentration were performed. The obtained crude product was purified by silica gel column chromatography (petroleum ether/acetone=7:1, v/v) to obtain a white foam-like solid 15ba (29.9g, with three-step total yield of 84%, ee=96%). HPLC conditions: AD-H column, Hexane: i-PrOH =70:30, flowrate: 1.0mL/min, column temperature: 25°C, detection wavelength: 254 nm, *t*ₘₐⱼₒᵣ = 5.585min, *t*ₘᵢₙₒᵣ = 4.769min. **Optical rotation:** [α]_{D}²⁵ = -117.9 (*c* = 0.8, CHCl₃); **¹H NMR** (400 MHz, CDCl₃) *δ* 7.73-7.70 (m, 4H), 7.46-7.28 (m, 8H), 6.96 (d, *J* = 8.2 Hz, 2H), 6.70 (s, 2H), 6.50 (s, 1H), 6.32 (s, 1H), 4.89 (q, *J* = 4.8 Hz, 1H), 3.88-3.87 (m, 1H, overlapped), 3.84 (s, 3H), 3.81 (s, 3H), 3.58 (s, 3H), 3.50-3.42 (m, 1H), 2.83-2.72 (m, 2H), 2.56-2.48 (m, 1H), 2.41-2.36 (m, 1H), 2.30 (s, 3H), 1.12 (s, 9H); **¹³C NMR** (100 MHz, CDCl₃) *δ* 152.2, 149.5, 146.2, 143.3, 142.6, 137.6, 135.5, 135.44, 133.38, 133.3, 130.0, 129.74, 129.71, 129.0, 127.7, 127.6, 126.8, 126.5, 125.6, 120.7, 118.5, 112.2, 110.6, 60.4, 55.8, 55.53, 55.46, 42.6, 38.6, 26.7, 26.2, 21.4, 19.7; **IR** (neat): *v*ₘₐₓ = 2933, 1513, 1487, 1448, 1261, 1155, 1113, 1033, 701 cm⁻¹; **HRMS (m/z):** [M + H]⁺ calculated for C₄₂H₄₇⁷⁹BrNO₆SSi⁺, 800.2071; found, 800.2066; C₂₆H₃₇⁸¹BrNO₆Si⁺, 802.2051; found, 802.2050.

### Example 41 Preparation of Compound 15 (Bischler-Napieralski/Asymmetric Transfer Hydrogenation)

Taking that R₂ was TBDPS, X was bromine, R₁₁ was Me and R was CO₂Me as an example, a compound 15bb was synthesized. For the synthesis route, refer to example 40. The ligand used was (1R, 2R)-(+)-N-p-toluenesulfonyl-1,2-diphenylethylenediamine. 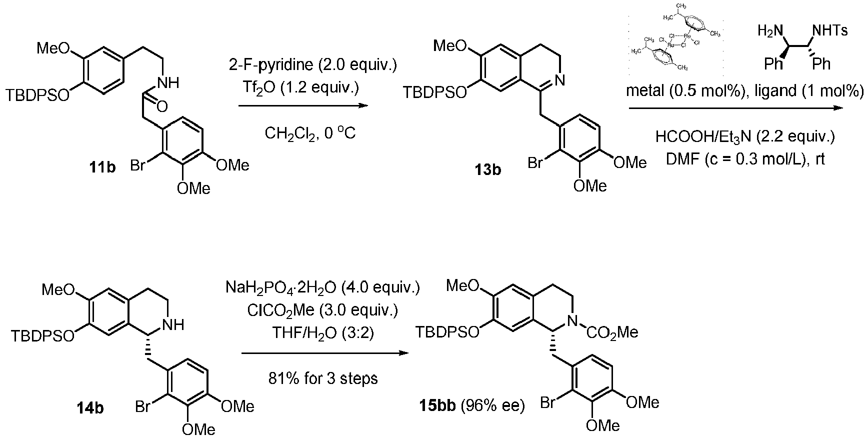 **15bb** (with three-step yield of 81%, 96% ee (*S*)), HPLC conditions: AD-H column, Hexane: i-PrOH = 95:5, flowrate: 0.8mL/min, column temperature: 25°C, detection wavelength: 254 nm, *t*ₘₐⱼₒᵣ = 8.100min, *t*ₘᵢₙₒᵣ = 10.942min. **Optical rotation:** [α]_{D}²⁵ = -52.5 (*c* = 0.2, CHCl₃); **Optical rotation:** [α]_{D}²⁵ = + 53.6 (*c* = 0.8, CHCl₃); **¹H NMR** (400 MHz, CDCl₃, some signals appeared in pairs due to amide rotational isomerism) *δ* 7.76-7.70 (m, 4H), 7.46-7.32 (m, 6H), 6.66 (d, *J* = 8.4 Hz, 1H), 6.62-6.61 (m, 2H), 6.53 (s, 0.8H), 6.49 (s, 0.2H), 5.15-5.12 (m, 0.2H), 5.06-5.02 (m, 0.8H), 4.27 (dd, *J* = 13.2, 4.4 Hz, 1H), 3.84 (s, 2.4H), 3.82 (s, 3.6H, overlapped), 3.69 (s, 2.4H), 3.56 (d, *J* = 8.8 Hz, 1.2H), 3.29-3.14 (m, 1H), 3.11 (s, 2.4H), 2.91-2.77 (m, 2H), 2.68-2.56 (m, 2H), 1.14 (s, 7H), 1.10 (s, 2H, overlapped); **¹³C NMR** (100 MHz, CDCl₃,some signals appeared in pairs due to amide rotational isomerism) *δ* 155.9, 155.7, 152.00, 149.4, 149.2, 146.2, 143.5, 143.3, 135.51, 135.46, 135.41, 135.36, 133.4, 133.3, 130.9, 130.8, 129.73, 129.66, 129.6, 128.5, 128.3, 127.62, 127.60, 127.57, 127.5, 126.8, 126.6, 126.0, 121.1, 120.6, 118.8, 118.3, 112.4, 112.1, 110.7, 60.4, 60.4, 56.0, 56.0, 55.8, 55.5, 54.3, 53.2, 52.4, 51.9, 42.1, 41.1, 38.4, 36.8, 28.20, 28.15, 26.71, 26.68, 19.7; **IR** (neat): *v*ₘₐₓ = 2932, 2857, 1696, 1593, 1513, 1486, 1447, 1260, 1104, 1032, 701 cm⁻¹; **HRMS (m/z):** [M + H]⁺ calculated for C₃₇H₄₃⁷⁹BrNO₆Si⁺, 704.2038; found, 704.2037; C₂₆H₃₇⁸¹BrNO₆Si⁺, 706.2017; found, 706.2018.

### Example 42 Preparation of Compound 15 (Bischler-Napieralski/Asymmetric Transfer Hydrogenation)

Taking that R₂ was TBDPS, X was bromine, R₁₁ was Me and R was Cbz as an example, a compound 15bc was synthesized. For the synthesis route, refer to example 40. The ligand used was (1S, 2S)-(+)-N-p-toluenesulfonyl-1,2-diphenylethylenediamine. 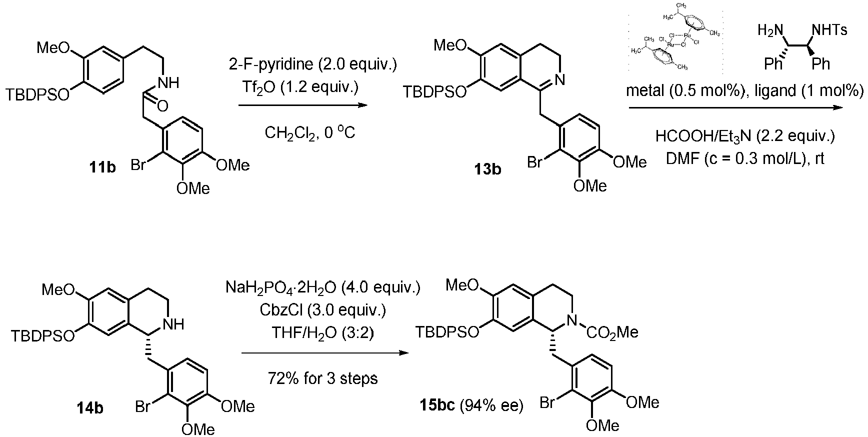 **15bc** (with three-step yield of 72%, 94% ee (*R*)), HPLC conditions: ADH0CE-EK072 column, gradient elution, 0-5.5min, Hexane: *i*-PrOH = 60:40 to 40:60; 5.5-25min, Hexane: *i*-PrOH = 40:60, flowrate: 1mL/min, column temperature: 40°C, detection wavelength: 254 nm, *t*ₘₐⱼₒᵣ = 11.470min, *t*ₘᵢₙₒᵣ = 4.066min. **Optical rotation**: [α]_{D}²⁵ = -66.3 (*c* = 0.76, CHCl₃); **¹H NMR** (400 MHz, CDCl₃, some signals appeared in pairs due to amide rotational isomerism) *δ*7.75-7.70 (m, 4H), 7.44-7.22 (m, 9.5H), 7.04-7.02 (m, 1.5H), 6.68-6.46 (m, 4H), 5.20-5.07 (m, 1H), 5.05-4.84 (m, 1H), 4.42-3.97 (m, 2H), 3.80-3.79 (m, 3.8H), 3.67 (d, *J* = 5.8 Hz, 4.4H), 3.59 (s, 0.8H), 3.32-3.20 (m, 1H), 2.95-2.44 (m, 4H), 1.13-1.11 (m, 9H); **¹³C NMR** (100 MHz, CDCl₃, some signals appeared in pairs due to amide rotational isomerism) *δ* 155.3, 155.0, 152.04, 151.99, 149.4, 149.2, 146.3, 146.2, 143.44, 143.37, 137.1, 136.4, 135.51, 135.49, 135.44, 135.40, 133.4, 133.3, 130.79, 130.76, 129.73, 129.66, 128.5, 128.4, 128.3, 128.2, 127.7, 127.64, 127.59, 127.54, 127.45, 126.8, 126.6, 126.0, 121.1, 120.1, 118.7, 118.5, 112.4, 112.2, 110.72, 110.68, 66.71, 66.65, 60.4, 60.3, 56.0, 55.7, 55.6, 54.2, 53.6, 41.9, 41.1, 38.4, 37.0, 28.3, 28.0, 26.9, 26.7, 26.7, 19.7; **IR** (neat): *v*ₘₐₓ = 2932, 2857, 1697, 1593, 1513, 1486, 1427, 1261, 1102, 1034, 700 cm⁻¹; **HRMS** (m/z): [M + H]⁺ calculated for C₄₃H₄₇⁷⁹BrNO₆Si⁺, 780.2351 ; found, 780.2355; C₄₃H₄₇⁸¹BrNO₆Si⁺, 782.2330; found, 782.2340.

### Example 43 Preparation of Compound 15 (Bischler-Napieralski/Asymmetric Transfer Hydrogenation)

Taking that R₂ was TBS, X was bromine, R₁₁ was Me and R was Ts as an example, a compound 15ea was synthesized through the following synthesis route:

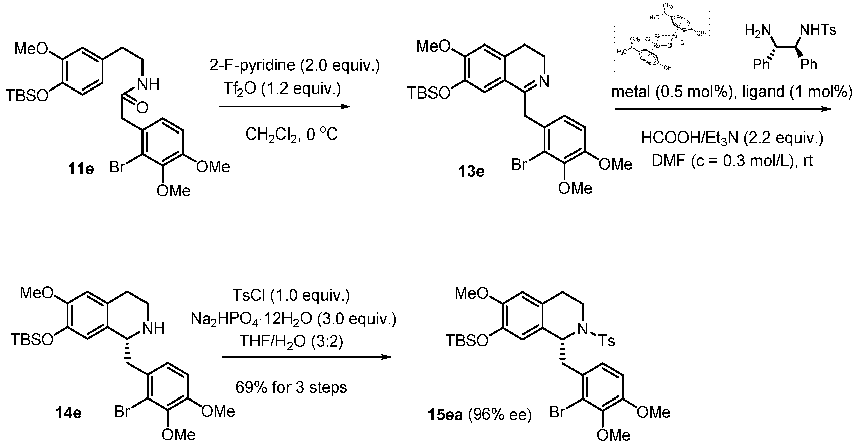

The compound 15ea was prepared by using 11e as a starting material, through intermediates 13e and 14e, and then by introducing a Cbz protection group. The synthesis steps were the same as those of 15aa. The reaction conditions and reagent amount from 11e to 15ea were as shown in the figure above. 15ea: The ligand used was (1S, 2S)-(+)-N-p-toluenesulfonyl-1,2-diphenylethylenediamine, and the three-step yield was 69%, 96% ee _{(S)}. HPLC conditions: IC00C3-QG035 column, Hexane: *i*-PrOH = 60:40, flowrate: 1mL/min, column temperature: 25°C, detection length: 254 nm, *t*ₘₐⱼₒᵣ = 16.703min, *t*ₘᵢₙₒᵣ = 13.134min. **Optical rotation**: [α]_{D}²⁵ = -111.8 (*c* = 0.6, CHCl₃). **¹H NMR** (400 MHz, CDCl₃, some signals appeared in pairs due to amide rotational isomerism) *δ* 7.44 (d, *J* = 8.0 Hz, 2H), 7.03 (d, *J* = 8.0 Hz, 2H), 6.82 (d, *J* = 8.4 Hz, 1H), 6.74 (d, *J* = 8.4 Hz, 1H), 6.46 (s, 1H), 6.40 (s, 1H), 5.16-5.12 (m, 1H), 3.89-3.87 (m, 1H, overlapped), 3.85 (s, 3H), 3.83 (s, 3H), 3.72 (s, 3H), 3.62-3.55 (m, 1H), 3.15-3.06 (m, 2H), 2.70-2.50 (m, 2H), 2.31 (s, 3H), 0.96 (s, 9H), 0.10 (s, 6H). **¹³C NMR** (100 MHz, CDCl₃, some signals appeared in pairs due to amide rotational isomerism) *δ* 152.3, 149.9, 146.4, 143.1, 142.7, 137.4, 130.1, 129.2, 127.8, 127.0, 126.7, 126.1, 120.8, 119.2, 111.9, 110.8, 60.4, 55.9, 55.8, 55.5, 43.0, 39.2, 26.5, 25.7, 21.4, 18.4, -4.66, -4.73. **IR** (neat): *v*ₘₐₓ = 2931, 2857, 1511, 1487, 1259, 1156, 1092, 1033, 801 cm⁻¹. **HRMS (m/z):** [M + H]⁺ calculated for C₃₂H₄₃⁷⁹BrNO₆SSi⁺, 676.1758; found, 676.1752; C₃₂H₄₃⁷⁹BrNO₆SSi⁺, 678.1738; found, 678.1735.

### Examples 44-50 Preparation of Compound 15 (Bischler-Napieralski/Transfer Hydrogenation)

Taking that R₂ was a hydroxyl protection group II, X was bromine, R₁₁ was Me or PMB and R was a secondary amine protection group as an example, a compound 15 was synthesized through the following synthesis route:

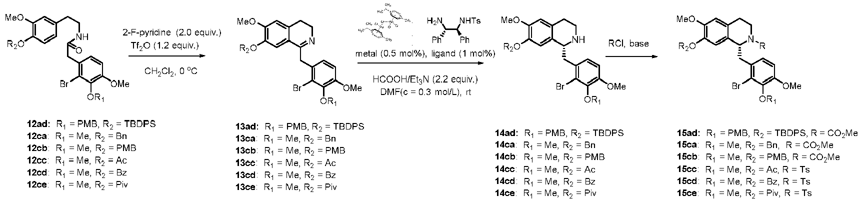

In example 44, taking that R₂ was TBDPS, X was bromine, R₁₁ was PMB and R was COOMe as an example, a compound 15ad was synthesized. The ligand used was (1R, 2R)-(+)-N-p-toluenesulfonyl-1,2-diphenylethylenediamine. The three-step total yield was 80%, ee=86% (*S*). HPLC conditions: IC-H column, Hexane: *i*-PrOH =85:15, flowrate: 0.8mL/min, column temperature: 25°C, detection wavelength: 254 nm, *t*ₘₐⱼₒᵣ = 21.438min, *t*ₘᵢₙₒᵣ = 24.448min. **Optical rotation**: [α]_{D}²⁵ = + 49.9 (*c* = 0.68, CHCl₃); **¹H NMR** (400 MHz, CDCl₃, some signals appeared in pairs due to amide rotational isomerism) *δ* 7.77-7.69 (m, 4H), 7.53-7.31 (m, 8H), 6.97-6.90 (m, 2H), 6.74-6.45 (m, 4H), 5.17-5.03 (m, 1H), 4.91-4.90 (m, 2H), 4.30-4.12 (m, 1H), 3.85-3.80 (m, 6.2H), 3.67-3.56 (m, 3.6H), 3.21-3.14 (m, 1H), 3.11 (s, 2H), 2.97-2.77 (m, 2H), 2.68-2.40 (m, 2H), 1.14-1.11 (m, 9H). **¹³C NMR** (100 MHz, CDCl₃, some signals appeared in pairs due to amide rotational isomerism) *δ* 159.6, 159.4, 156.0, 155.8, 152.2, 149.4, 149.2, 145.2, 145.1, 143.5, 143.3, 135.53, 135.48, 135.44, 135.39, 133.5, 133.3, 131.0, 130.8, 130.3, 130.2, 129.72, 129.65, 129.4, 128.5, 128.4, 127.64, 127.61, 127.5, 126.9, 126.6, 126.0, 121.6, 121.1, 118.8, 118.4, 113.7, 113.6, 112.4, 112.1, 110.7, 74.4, 74.2, 56.1, 56.0, 55.7, 55.6, 55.32, 55.28, 54.3, 53.2, 52.4, 52.0, 42.2, 41.1, 38.4, 36.9, 28.22, 28.15, 26.74, 26.71, 19.7. **IR** (neat): *v*ₘₐₓ = 2932, 2858, 1697, 1612, 1592, 1513, 1484, 1447, 1260, 1106, 1032, 752, 702 cm⁻¹. **HRMS (m/z):** [M + H]⁺ calculated for C₄₄H₄₉⁷⁹BrNO₇Si⁺, 810.2456; found, 810.2450; C₄₄H₄₉⁸¹BrNO₇Si⁺, 812.2436; found, 812.2441.

In example 45, taking that R₂ was Bn, X was bromine, R₁₁ was Me and R was COOMe as an example, a compound 15ca was synthesized. The ligand used was (1R, 2R)-(+)-N-p-toluenesulfonyl-1,2-diphenylethylenediamine. The three-step total yield was 82%, ee=96% (*S*). HPLC conditions: IC-H column, Hexane: *i*-PrOH = 60:40, flowrate: 1.0mL/min, column temperature: 25°C, detection wavelength: 254 nm, *t*ₘₐⱼₒᵣ = 18.441min, *t*ₘᵢₙₒᵣ = 14.962min. **Optical rotation:** [α]_{D}²⁵ = + 75.3 (*c* = 0.68, CHCl₃). **¹H NMR** (400 MHz, CDCl₃, some signals appeared in pairs due to amide rotational isomerism) *δ* 7.46-7.28 (m, 5H), 6.79-6.70 (m, 2.8H), 6.61 (d, *J* = 12.4 Hz, 1H), 6.37 (s, 0.2H), 5.31-5.23 (m, 1H), 5.14 (s, 1.5H), 4.32 (dd, *J =* 13.2, 4.2 Hz, 0.5H), 4.34-4.30 (m, 0.7H), 3.96-3.91 (m, 0.3H), 3.88-3.82 (m, 9H), 3.63 (s, 0.7H), 3.45-3.38 (m, 0.3H), 3.28 (td, *J* = 12.8, 4.0 Hz, 0.8H), 3.21 (s, 2H), 3.13-3.04 (m, 1.2H), 2.92-2.76 (m, 2H), 2.68-2.61 (m, 1H).**¹³C NMR** (100 MHz, CDCl₃, some signals appeared in pairs due to amide rotational isomerism) *δ* 155.91, 155.87, 152.14, 148.5, 148.4, 146.4, 146.3, 146.2, 137.2, 137.1, 130.8, 130.7, 128.6, 128.5, 128.3, 128.2, 127.9, 127.8, 127.2, 127.1, 126.8, 126.7, 126.3, 126.2, 121.3, 120.6, 113.2, 112.9, 111.8, 111.6, 110.9, 110.8, 71.4, 71.1, 60.5, 60.4, 56.1, 56.02, 55.98, 54.7, 53.5, 52.5, 52.1, 42.3, 41.2, 38.7, 37.0, 28.2, 28.1. **IR** (neat): *v*ₘₐₓ = 2929, 1695, 1594, 1515, 1486, 1448, 1256, 1101, 1032 cm⁻¹. **HRMS (m/z):** [M + H]⁺ calculated for C₂₈H₃₁BrNO₆⁺, 556.1329; found, 556.1326; C₄₄H₄₉⁸¹BrNO₇Si⁺, 558.1309; found, 558.1310.

In example 46, taking that R₂ was PMB, X was bromine, R₁₁ was Me and R was COOMe as an example, a compound 15cb was synthesized. The ligand used was (1R, 2R)-(+)-N-p-toluenesulfonyl-1,2-diphenylethylenediamine. The three-step total yield was 68%, ee=96% (*S*). HPLC conditions: IC-H column, Hexane: i-PrOH = 60:40, flowrate: 1mL/min, column temperature: 25 °C, detection wavelength: 254 nm, *t*ₘₐⱼₒᵣ = 30.306min, *t*ₘᵢₙₒᵣ = 24.274min. **Optical rotation:** [α]_{D}²⁵ = + 66.3 (*c* = 0.48, CHCl₃). **¹H NMR** (400 MHz, CDCl₃, some signals appeared in pairs due to amide rotational isomerism) *δ* 7.38 (d, *J* = 8.8 Hz, 1.5H), 7.30 (d, *J* = 8.4 Hz, 0.5H), 6.92-6.86 (m, 2H), 6.78-6.71 (m, 2.8H), 6.62-6.59 (m, 1H), 6.38 (s, 0.2H), 5.33-5.25 (m, 1H), 5.05 (s, 1.5H), 4.85 (q, *J* = 12.0 Hz, 0.5H), 4.32 (dd, *J* = 13.2, 5.6 Hz, 0.7H), 3.98-3.91 (m, 0.3H), 3.88-3.80 (m, 12H), 3.63 (s, 0.7H), 3.45-3.38 (m, 0.2H), 3.32-3.25 (m, 0.8H, overlapped), 3.22 (s, 2.3H), 3.15-3.11 (m, 1.2H), 2.91-2.76 (m, 1.8H), 2.68-2.61 (m, 1H). **¹³C NMR** (100 MHz, CDCl₃,some signals appeared in pairs due to amide rotational isomerism) *δ* 159.34, 159.28, 155.92, 155.88, 152.2, 148.6, 148.4, 146.5, 146.3, 146.2, 130.9, 130.7, 129.2, 129.1, 129.0, 128.9, 128.3, 128.2, 126.7, 126.6, 126.3, 126.2, 121.3, 120.6, 114.0, 113.9, 113.3, 113.0, 111.8, 111.5, 110.9, 110.8, 71.1, 70.9, 60.5, 60.4, 56.1, 56.0, 55.96, 55.3, 55.2, 54.7, 53.5, 52.5, 52.1, 42.3, 41.2, 38.7, 37.0, 28.2, 28.1. **IR** (neat): *v*ₘₐₓ = 3013, 2935, 1690, 1613, 1486, 1451, 1243, 1102, 748 cm⁻¹. **HRMS (m/z):** [M + H]⁺ calculated for C₂₉H₃₃⁷⁹BrNO₇⁺, 586.1435; found, 586.1434; C₂₉H₃₃⁸¹BrNO₇⁺, 588.1414; found, 588.1420.

In example 47, taking that R₂ was Ac, X was bromine, R₁₁ was Me and R was Ts as an example, a compound 15cc was synthesized. The ligand used was (1R, 2R)-(+)-N-p-toluenesulfonyl-1,2-diphenylethylenediamine. The three-step total yield was 84%, ee=94% (*S*) (measured after removing Ac). HPLC conditions: IC-H column, Hexane: i-PrOH = 85:15, flowrate: 1.5mL/min, column temperature: 25°C, detection wavelength: 254 nm, *t*ₘₐⱼₒᵣ = 34.567min, *t*ₘᵢₙₒᵣ = 44.746min. **Optical rotation:** [α]_{D}²⁵ = +87.4 (*c* = 0.88, CHCl₃). **¹H NMR** (400 MHz, CDCl₃) *δ* 7.40 (d, *J* = 8.4 Hz, 2H), 7.05 (d, *J* = 8.0 Hz, 2H), 6.80-6.69 (m, 2H), 6.67 (s, 1H), 6.55 (s, 1H), 5.18 (dd, *J* = 8.0, 6.0 Hz, 1H), 3.92 (m, 1H), 3.82 (s, 3H), 3.81 (s, 3H), 3.76 (s, 3H), 3.50-3.62 (m, 1H), 3.14-3.05 (m, 2H), 2.73-2.85 (m, 1H), 2.53 -2.64 (m, 1H), 2.33 (s, 3H), 2.28 (s, 3H). **¹³C NMR** (100 MHz, CDCl₃) *δ* 168.8, 152.3, 149.8, 146.3, 142.9, 137.9, 137.1, 131.7, 129.7, 129.2, 128.0, 127.0, 126.7, 121.2, 120.7, 112.3, 110.9, 60.4, 55.9, 55.8, 55.3, 42.9, 38.7, 27.1, 21.4, 20.6. IR (neat): *v*ₘₐₓ = 2962, 1763, 1596, 1511, 1488, 1261, 1155, 1032 cm⁻¹. **HRMS (m/z):** [M + H]⁺ calculated for C₂₈H₃₁⁷⁹BrNO₇S⁺, 604.0999; found, 604.1004; C₂₈H₃₁⁸¹BrNO₇S⁺, 606.0979; found, 606.0984.

In example 48, taking that R₂ was Bz, X was bromine, R₁₁ was Me and R was Ts as an example, a compound 15cd was synthesized. The ligand used was (1R, 2R)-(+)-N-p-toluenesulfonyl-1,2-diphenylethylenediamine. The three-step total yield was 84%, ee=94% *(S)*. HPLC conditions: AD-H column, Hexane: *i*-PrOH = 80:20, flowrate: 1.0mL/min, column temperature: 25°C, detection wavelength: 254 nm, *t*ₘₐⱼₒᵣ = 15.023min, *t*ₘᵢₙₒᵣ = 18.524min. **Optical rotation:** [α]_{D}²⁵ = +120.5 (*c* = 0.64, CHCl₃). **¹H NMR** (400 MHz, CDCl₃) *δ* 8.21-8.15 (m, 2H), 7.67-7.60 (m, 1H), 7.54-7.40 (m, 4H), 7.10-7.03 (m, 2H),,6.79 (d, *J* = 9.2 Hz, 1H), 6.77 (s, 1H,overlap), 6.73 (d, *J* = 8.4 Hz, 1H), 6.61 (s, 1H), 5.21 (t, *J* = 7.6 Hz, 1H), 4.04-3.89 (m, 1H), 3.83 (s, 3H), 3.81 (s, 3H), 3.75 (s, 3H), 3.66-3.54 (m, 1H), 3.19-3.07 (m, 2H), 2.88-2.76 (m, 1H), 2.68-2.59 (m, 1H), 2.34 (s, 3H).**¹³C NMR** (100 MHz, CDCl₃) *δ* 164.6, 152.4, 150.1, 146.3, 143.0, 138.2, 137.2, 133.5, 131.8, 130.2, 129.8, 129.3, 129.3, 128.5, 128.1, 127.1, 126.8, 121.4, 120.7, 112.5, 110.9, 60.4, 55.9, 55.9, 55.4, 42.9, 38.8, 27.2, 21.4. **IR** (neat): *v*ₘₐₓ = 1738, 1511, 1487, 1450, 1261, 1213, 1154, 1025, 811, 729 cm⁻¹. **HRMS** (m/z): [M + H]⁺ calculated for C₃₃H₃₃⁷⁹BrNO₇S⁺, 666.1156; found, 666.1151; C₃₃H₃₃⁸¹BrNO₇S⁺, 668.1135; found, 668.1135.

In example 49, taking that R₂ was Piv, X was bromine, R₁₁ was Me and R was Ts as an example, a compound 15ce was synthesized. The ligand used was (1R, 2R)-(+)-N-p-toluenesulfonyl-1,2-diphenylethylenediamine. The three-step total yield was 82%, ee=95% (S). HPLC conditions: IC00C3-QG035, H₂O: MeOH = 10:90, flowrate: 1mL/min, column temperature: 25°C, detection wavelength: 254 nm, *t*ₘₐⱼₒᵣ = 16.37min, *t*ₘᵢₙₒᵣ = 18.44min. **Optical rotation:** [α]_{D}²⁵ = + 104.3 (*c* = 0.6, CHCl₃). **¹H NMR** (400 MHz, CDCl₃) *δ* 7.45-7.41 (m, 2H), 7.06 (d, *J* = 8.0 Hz, 2H), 6.78-6.69 (m, 2H), 6.54(s, 1H), 6.53(s, 1H), 5.18 (t, *J* = 7.2 Hz, 1H), 3.95-3.87 (m, 1H), 3.86 (s, 3H), 3.83 (s, 3H), 3.73 (s, 3H), 3.62-3.52 (m, 1H), 3.11 (d, *J* = 7.6 Hz, 2H), 2.81-2.70 (m, 1H), 2.64-2.54 (m, 1H), 2.33 (s, 3H), 1.33 (s, 9H).**¹³C NMR** (100 MHz, CDCl₃) *δ* 176.5, 152.3, 150.0, 146.3, 143.0, 138.3, 137.3, 131.3, 129.8, 129.3, 127.8, 127.0, 126.8, 121.2, 120.8, 112.3, 110.9, 60.4, 55.9, 55.9, 55.3, 42.9, 39.0, 38.8, 27.2, 27.1, 21.4. **IR** (neat): *v*ₘₐₓ= 1751, 1511, 1486, 1449, 1272, 1154, 1097, 1030, 749 cm⁻¹. **HRMS (m/z):** [M + H]⁺ calculated for C₃₁H₃₇⁷⁹BrNO₇S⁺, 646.1469; found, 646.1469; C₃₁H₃₇⁸¹BrNO₇S⁺, 648.1448; found, 648.1457.

### Example 50 Preparation of Compound 17 (Preparation of Substrate for Intramolecular Oxidative Dearomatization Heck Coupling Reaction)

When R₁₁ in the compound 15 was a hydrogen atom, a compound 17 was prepared after introducing a hydroxyl protection group I (PMB) into the compound 15.

Taking that R₂ was TBDPS, X was bromine, R₁₁ was a hydrogen atom and R was Ts as an example, a compound 17aab was synthesized through the following synthesis route:

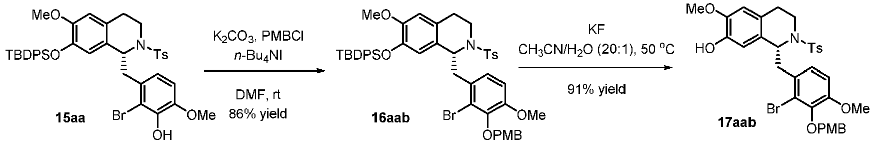

A compound 15aa (10.00g, 12.71mmol, 1.0 equiv.), potassium carbonate (5.27g, 38.13mmol, 3.0 equiv.) and TBAI (469mg, 1.27mmol, 0.1 equiv.) were placed in a reaction flask. Air was extracted and changed. Under the protection of argon, dry DMF (180mL) was added. Under stirring at room temperature, PMBCl (3.45mL, 25.42mmol, 2.0 equiv.) was added for reaction for about 6h at room temperature. After TLC showed that the raw materials disappeared completely, dimethylamine (1.30mL, 25.42mmol, 2.0 equiv.) was added into the reaction solution. Stirring was performed for 2h at room temperature. Then, saturated NH₄Cl solution (100mL) was added to quench the reaction. Extraction was performed by using ethyl acetate (100mL*4) . The organic layers were combined. Washing was performed sequentially by using water (100mL^{∗}1) and saturated sodium chloride solution (100mL^{∗}2). The organic layer was dried by using anhydrous magnesium sulfate. Filtering, concentration and draining were performed. The obtained crude product was purified by silica gel column chromatography (petroleum ether/dichloromethane/acetone=100:100:1, v/v, containing 0.5% ammonia water; silica gel was treated by using petroleum ether containing 0.5% ammonia water and then loaded onto the column) to obtain a white foam-like solid 16aab (9.9g, with yield of 86%). **Data of compound 16aab: Optical rotation:** [α]_{D}²⁵ = -77.6 (*c* = 1.32, CHCl₃). **¹H NMR** (400 MHz, CDCl₃) *δ* 7.77-7.70 (m, 4H), 7.49 (d, *J* = 8.6 Hz, 2H), 7.43-7.25 (m, 9H), 6.96 (d, *J* = 8.0 Hz, 2H), 6.93 (d, *J* = 8.8 Hz, 2H), 6.70 (s, 1H), 6.52 (s, 1H), 6.31 (s, 1H), 4.93-4.90 (m, 1H, overlapped), 4.90 (s, 3H), 3.87-3.85 (m, 1H), 3.83 (s, 3H, overlapped), 3.82 (s, 3H, overlapped), 3.57 (s, 3H), 3.48-3.41 (m, 1H), 2.79-2.76 (m, 2H), 2.56-2.48 (m, 1H), 2.40-2.35 (m, 1H), 2.28 (s, 3H), 1.12 (s, 9H).**¹³C NMR (100 MHz, CDCl₃)** *δ* 159.4, 152.5, 149.5, 145.1, 143.3, 142.6, 137.6, 135.49, 135.47, 134.8, 133.4, 133.4, 130.2, 130.1, 129.7, 129.71, 129.69, 129.1, 127.73, 127.68, 127.58, 127.56, 126.9, 126.5, 125.6, 121.2, 118.6, 113.6, 112.2, 110.7, 74.2, 55.9, 55.53, 55.48, 55.3, 42.8, 38.7, 26.7, 26.2, 21.4, 19.7. **IR** (neat): *v*ₘₐₓ = 2932, 1513, 1485, 1463, 1428, 1248, 1155, 1033, 749 cm⁻¹. **HRMS (m/z):** [M + H]⁺ calculated for C₄₉H₅₃⁷⁹BrNO₇SSi⁺, 906.2490; found, 906.2494; C₄₉H₅₃⁸¹BrNO₇SSi⁺, 908.2469; found, 908.2481.

The compound 16aab (9.00g, 9.92mmol, 1.0 equiv.) was dissolved in CH₃CN/H₂O mixed solvent (210mL, v/v=20:1). KF (1.15g, 19.84mmol, 2.0 equiv.) was added at room temperature. Heating to 50°C was performed for reaction for about 3h. After TLC showed that the raw materials disappeared completely, cooling to 0°C was performed. Saturated NaHCO₃ aqueous solution (100mL) was added to quench the reaction. Reduced-pressure distillation was performed on the mixture to remove CH₃CN. The residue was extracted by using ethyl acetate (100mL^{∗}3). The organic layers were combined. Washing was performed by using saturated sodium chloride aqueous solution (100mL^{∗}2). Drying was performed by using anhydrous magnesium sulfate. Filtering and reduced-pressure concentration were performed. The crude product was purified by silica gel column chromatography (petroleum ether/acetone=4:1, v/v, containing 0.5% ammonia water; silica gel was treated by using petroleum ether containing 0.5% ammonia water, and then loaded onto the column) to obtain a white foam-like solid 17aab (6.04g, with yield of 91%).

Data of compound 17aab: **Optical rotation:** [α]_{D}²⁵ = -103.2 (*c* = 0.6, CHCl₃). **¹H NMR** (400 MHz, CDCl₃) *δ* 7.48 (d, *J* = 8.4 Hz, 2H), 7.39 (d, *J* = 8.4 Hz, 2H), 7.03 (d, *J* = 8.0 Hz, 2H), 6.92-6.90 (m, 2H), 6.82 (d, *J* = 8.4 Hz, 1H), 6.74 (d, *J* = 8.4 Hz, 1H), 6.64 (s, 1H), 6.45 (s, 1H), 5.46 (s, 1H), 5.12 (dd, *J* = 9.6, 4.8 Hz, 1H), 4.92 (s, 2H), 3.93-3.88 (m, 1H), 3.85 (s, 2H), 3.82 (s, 3H, overlapped), 3.82(s, 3H, overlapped), 3.61-3.53 (m, 1H), 3.17 (dd, *J* = 14.0, 4.8 Hz, 1H), 3.05 (dd, *J* = 14.0, 9.6 Hz, 1H), 2.80-2.71(m, 1H), 2.57-2.51 (m, 1H), 2.30 (s, 3H).**¹³C NMR** (100 MHz, CDCl₃) *δ* 159.6, 152.7, 145.8, 145.4, 143.9, 143.0, 137.3, 130.3, 129.3, 128.8, 127.2, 126.8, 124.8, 121.5, 113.8, 112.9, 110.9, 74.4, 56.0, 55.4, 43.1, 39.2, 26.9, 21.6. **IR** (neat): *v*ₘₐₓ = 1596, 1512, 1484, 1462, 1441, 1245, 1151, 1029, 748 cm⁻¹. **HRMS (m/z):** [M + H]⁺ calculated for C₃₃H₃₅⁷⁹BrNO₇S⁺, 668.1312; found, 668.1313; C₃₃H₃₅⁸¹BrNO₇S⁺, 670.1292; found, 670.1298.

### Example 51 Preparation of Compound 17 (Preparation of Substrate for Intramolecular Oxidative Dearomatization Heck Coupling Reaction)

When R₁₁ in the compound 15 was a hydrogen atom, a compound 17 was prepared after introducing a hydroxyl protection group I (Bn) into the compound 15.

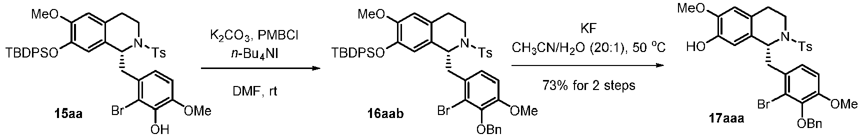

Taking that R₂ was TBDPS, X was bromine, R₁₁ was a hydrogen atom and R was Ts as an example, a compound 17aaa was synthesized. For the synthesis route, refer to example 48. Data of compound 17aaa: **¹H NMR**(400 MHz, CDCl₃) *δ* 7.57 (d, *J* = 7.2 Hz, 2H), 7.41-7.37 (m, 4H), 7.34-7.31 (m, 1H), 7.03 (d, *J* = 8.0 Hz, 2H), 6.84 (d, *J* = 8.4 Hz, 1H), 6.75 (d, *J* = 8.4 Hz, 1H), 6.65 (s, 1H), 6.45 (s, 1H), 5.47 (s, 1H), 5.13 (dd, *J* = 9.6, 4.8 Hz, 1H), 4.98 (s, 2H), 3.94-3.89 (m, 1H), 3.85 (s, 3H), 3.82 (s, 3H), 3.61-3.54 (m, 1H), 3.17 (dd, *J* = 14.0, 5.2 Hz, 1H), 3.05 (dd, *J* = 14.0, 9.6 Hz, 1H), 2.79-2.71 (m, 1H), 2.57-2.51 (m, 1H), 2.30 (s, 3H).**¹³C NMR** (100 MHz, CDCl₃) *δ* 152.7, 145.8, 145.3, 143.9, 143.0, 137.6, 137.3, 130.3, 129.3, 128.7, 128.5, 128.4, 128.0, 127.2, 126.8, 124.8, 121.4, 112.9, 111.0, 110.8, 74.6, 56.0, 43.1, 39.2, 26.9, 21.6. **IR** (neat): *v*ₘₐₓ = 1511, 1484, 1454, 1271, 1150, 1028, 750 cm⁻¹. **HRMS (m/z):** [M + H]⁺ calculated for C₃₂H₃₃BrNO₆S⁺, 638.1206; found, 638.1211; C₃₃H₃₅⁸¹BrNO₇S⁺, 640.1186; found, 640.1196.

### Example 52 Preparation of Compound 17 (Preparation of Substrate for Intramolecular Oxidative Dearomatization Heck Coupling Reaction)

When R₁₁ in the compound 15 was a hydroxyl protection group I, a compound 17 was directly prepared by using the compound 15.

Taking that R₂ was Ac, X was bromine, R₁₁ was Me and R was Ts as an example, a compound 17aac was synthesized through the following synthesis route:

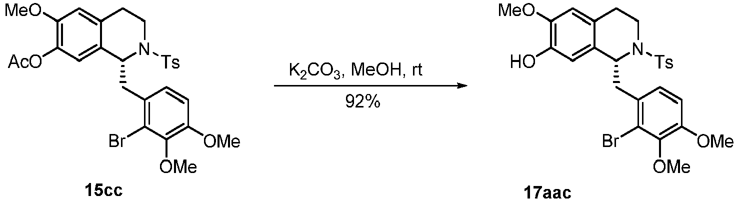

A compound (*S*)-15cc (1.00g, 1.65mmol, 1.0 equiv.) was dissolved in MeOH (10mL). K₂CO₃ (0.57g, 4.13mmol, 2.5 equiv.) was added at room temperature. Stirring was performed for reaction for about 0.5h. After TLC showed that the raw materials disappeared completely, cool to 0°C was performed. Water was added to quench the reaction. Extraction was performed by using ethyl acetate (20mL^{∗}3). The organic layers were combined. Drying was performed by using anhydrous magnesium sulfate. Filtering and reduced-pressure concentration were performed. The crude product was purified by silica gel column chromatography (petroleum ether/acetone=6:1, v/v,) to obtain a white foam-like solid (S)-17aac (854mg, with yield of 92%). **Optical rotation:** [α]_{D}²⁵ = +83.7 (*c* = 0.84, CHCl₃). **¹H NMR** (400 MHz, CDCl₃) *δ* 7.39 (d, *J* = 8.0 Hz, 2H), 7.02 (d, *J* = 8.0 Hz, 2H), 6.83 (d, *J* = 8.4 Hz, 1H), 6.73 (d, *J* = 8.8 Hz, 1H), 6.66 (s, 1H), 6.45 (s, 1H), 5.48 (s, 1H), 5.12 (dd, *J* = 10.0, 4.8 Hz, 1H), 3.94-3.89 (m, 1H), 3.86 (s, 3H), 3.82 (s, 6H), 3.61-3.54 (m, 1H), 3.16 (dd, *J* = 14.0, 4.8 Hz, 1H), 3.04 (dd, *J* = 14.0, 9.6 Hz, 1H), 2.79-2.70 (m, 1H), 2.57-2.51 (m, 1H), 2.32 (s, 3H).**¹³C NMR** (100 MHz, CDCl₃) *δ* 152.5, 146.5, 145.8, 144.0, 143.0, 137.4, 130.2, 129.3, 128.8, 127.2, 126.7, 124.8, 121.0, 112.9, 111.0, 56.0, 43.0, 39.2, 26.9, 21.6. **IR** (neat): *v*ₘₐₓ = 3428, 1512, 1487, 1449, 1265, 1149, 1031 cm⁻¹. **HRMS (m/z):** [M + H]⁺ calculated for C₂₆H₂₉⁷⁹BrNO₆S⁺, 562.0893; found, 562.0893; C₃₃H₃₅⁸¹BrNO₇S⁺, 564.0873; found, 564.0873.

### Example 53 Preparation of Compound 17 (Preparation of Substrate for Intramolecular Oxidative Dearomatization Heck Coupling Reaction)

When R₁₁ in the compound 15 was a hydroxyl protection group I, a compound 17 was directly prepared by using the compound 15.

Taking that R₂ was TBDPS, X was bromine, R₁₁ was Me and R was Ts as an example, a compound 17aac was synthesized through the following synthesis route:

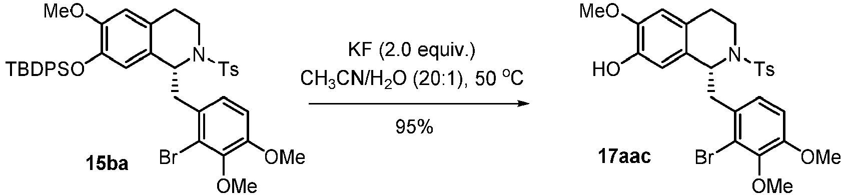

A compound *(R)*-15ba (10.00g, 12 49mmol, 1.0 equiv.) was dissolved in CH₃CN/H₂O mixed solvent (210mL, v/v=20:1). KF (1.45g, 24.97mmol, 2.0 equiv.) was added at room temperature. Heating to 50°C was performed for reaction for about 3h. After TLC showed that the raw materials disappeared completely, cooling to 0°C was performed. Saturated NaHCO₃ aqueous solution (100mL) was added to quench the reaction. Reduced-pressure distillation was performed on the mixture to remove CH₃CN. The residue was extracted by using ethyl acetate (100mL^{∗}3). The organic layers were combined. Washing was performed by using saturated sodium chloride aqueous solution (100mL^{∗}2). Drying was performed by using anhydrous magnesium sulfate. Filtering and reduced-pressure concentration were performed. The crude product was purified by silica gel column chromatography (petroleum ether/acetone=4: 1, v/v,) to obtain a white foam-like solid *(R)*-17aac (6.67g, with yield of 95%). **Optical rotation:** [α]_{D}²⁵ = -96.9 (*c* = 0.8, CHCl₃).

### Example 54 Preparation of Compound 18 (Intramolecular Oxidative Dearomatization Heck Reaction)

Taking that X was bromine, R₁ was Me and R was Ts as an example, a compound 18aac was synthesized through the following synthesis route:

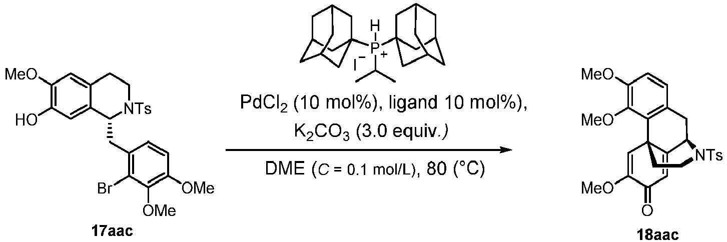

A compound 17aac (200mg, 0.356mmol, 1.0 equiv.), palladium chloride (6.3mg, 0.0356mmol, 0.1 equiv.), a phosphine ligand (16.8mg, 0.0356mmol, 0.1 equiv.) and potassium carbonate (147mg, 1.067mmol, 3.0 equiv.) were placed in a reaction vessel. Air was extracted and changed. Under the protection of argon, degassed dry DMF (4mL, c=0.1mol/L) was added. The reaction vessel was placed in an oil bath at 80°C for reaction for 12h. After TLC monitored that the raw materials disappeared completely, the reaction solution was cooled to room temperature. Water (4mL) was added at 0°C to quench the reaction. Extraction was performed by using ethyl acetate (5mL^{∗}3). The organic layers were combined. Washing was performed sequentially by using water (10mL^{∗}1) and saturated sodium chloride solution (10mL^{∗}1). The organic layer was dried by using anhydrous magnesium sulfate. Filtering and concentration were performed. The obtained crude product was purified by silica gel column chromatography (petroleum ether/dichloromethane/acetone=15:15:1, v/v) to obtain a white foam-like solid 18aac (27mg, with yield of 16%). **Data of 18aac: Optical rotation**: [α]_{D}²⁵ = +4.5 (*c* = 0.4, CHCl₃). **¹H NMR** (400 MHz, CDCl₃) *δ* 7.66 (d, *J =* 8.4 Hz, 2H), 7.27 (d, *J* = 4.0Hz, 2H), 7.16 (s, 1H), 6.84-6.77 (m, 2H), 6.22 (s, 1H), 4.95 (d, *J* = 3.6 Hz, 1H), 3.92 (s, 3H), 3.86 (s, 3H), 3.74 (s, 3H), 3.73-3.68 (m, 1H) , 3.28 (dd, *J* = 17.6, 4.8 Hz, 1H), 3.20 (dd, *J* = 17.6, 1.6 Hz, 1H), 3.04-2.96 (m, 1H), 2.41 (s, 3H), 2.22-2.19 (m, 1H), 1.40-1.26 (m, 1H). **¹³C NMR** (100 MHz, CDCl₃) *δ* 180.8, 157.9, 152.2, 151.3, 147.4, 143.9, 137.2, 130.3, 130.0, 127.8, 127.1, 124.0, 122.4, 120.0, 112.3, 56.0, 55.0, 43.6, 40.3, 39.0, 21.7. IR (neat): *v*ₘₐₓ = 2936, 1674, 1649, 1616, 1483, 1280, 1213, 1159 cm⁻¹. **HRMS (m/z):** [M + H]⁺ calculated for C₂₆H₂₈NO₆S⁺, 482.1632; found, 482.1636.

### Examples 55-58 Preparation of Compound 18 (Screening of Solvent for Intramolecular Oxidative Dearomatization Heck Reaction)

Taking that X was bromine, R₁ was Me and R was Ts as an example, a compound 18aac was synthesized through the following synthesis route:

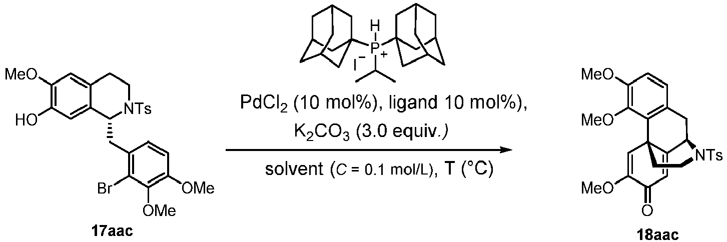

The synthesis steps of the compound 18aac in examples 55-58 were the same as those in example 54. Conditions such as the synthesis reagent and amount thereof were as shown in the synthesis route. The difference between the examples only lay in that different solvents and temperatures were used in the reaction of preparing the compound 18aac from the compound 17aac. The results were as shown in the following table:

| Example | Solvent | Temperature (°C) | Yield % |
|---|---|---|---|
| Example 55 | Toluene | 110 | 19% |
| Example 56 | Dimethylbezene | 125 | 55% |
| Example 57 | PhOMe | 125 | 62% |
| Example 58 | DMF | 125 | 67% |

### Examples 59-62 Preparation of Compound 18 (Screening of Temperature for Intramolecular Oxidative Dearomatization Heck Reaction)

Taking that X was bromine, R₁ was Me and R was Ts as an example, a compound 18aac was synthesized through the following synthesis route:

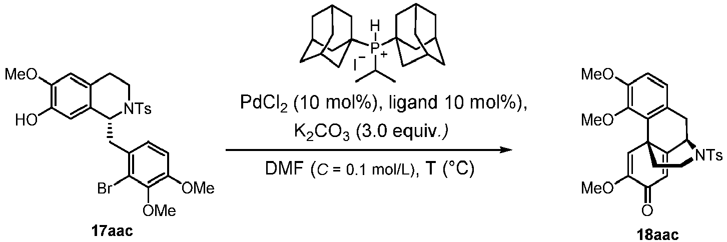

The synthesis steps of the compound 18aac in examples 59-62 were the same as those in example 58. Conditions such as the synthesis reagent and amount thereof were as shown in the synthesis route. The difference of the examples from example 58 only lay in that different reaction temperatures were used in the reaction of preparing the compound 18aac from the compound 17aac.

| Example | Temperature (°C) | Yield % |
|---|---|---|
| Example 59 | 100 | 11% |
| Example 60 | 125 | 61% |
| Example 61 | 135 | 68% |
| Example 62 | 145 | 69% |

### Examples 63-67 Preparation of Compound 18 (Screening of Alkali for Intramolecular Oxidative Dearomatization Heck Reaction)

Taking that X was bromine, R₁ was Me and R was Ts as an example, a compound 18aac was synthesized through the following synthesis route:

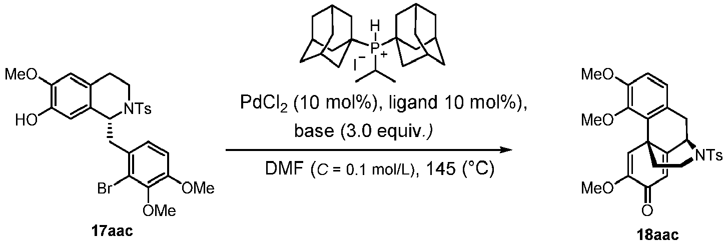

The synthesis steps of the compound 18aac in examples 63-67 were the same as those in example 62. Conditions such as the synthesis reagent and amount thereof were as shown in the synthesis route. The difference between the examples only lay in that different alkalis were used in the reaction of preparing the compound 18aac from the compound 17aac. The results were as shown in the following table:

| Example | Alkali | Yield % |
|---|---|---|
| Example 63 | Cs₂CO₃ | 15% |
| Example 64 | *t*-BuOK | 36% |
| Example 65 | KH | 42% |
| Example 66 | KOH | 46% |
| Example 67 | K₃PO₄ | 68% |

### Examples 68-69 Preparation of Compound 18 (Screening of Catalyst and Ligand Equivalent for Intramolecular Oxidative Dearomatization Heck Reaction)

Taking that X was bromine, R₁ was Me and R was Ts as an example, a compound 18aac was synthesized through the following synthesis route:

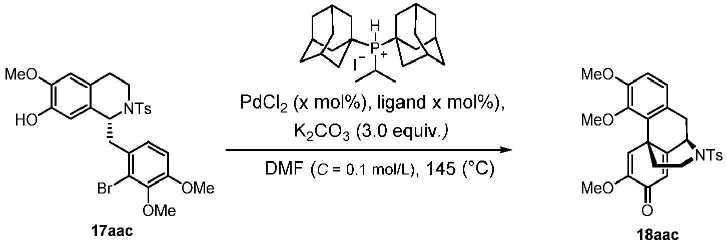

The synthesis steps of the compound 18aac in examples 68-69 were the same as those in example 62. Conditions such as the synthesis reagent, amount thereof and reaction temperature were as shown in the synthesis route. The difference between the examples only lay in that the equivalents of palladium chloride and the ligand used in the reaction of preparing the compound 18aac from the compound 17aac were different. The results were as shown in the following table:

| Example | Amount of PdCl₂ (mol%) | Amount of ligand (mol%) | Yield % |
|---|---|---|---|
| Example 68 | 7.5% | 7.5% | 57% |
| Example 69 | 5% | 5% | 51% |

### Examples 70-74 Preparation of Compound 18 (Screening of Concentration for Intramolecular Oxidative Dearomatization Heck Reaction)

Taking that X was bromine, R₁ was Me and R was Ts as an example, a compound 18aac was synthesized through the following synthesis route:

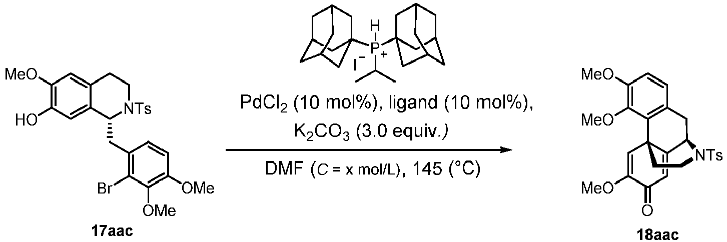

The synthesis steps of the compound 18aac in examples 70-74 were the same as those in example 62. Conditions such as the synthesis reagent, amount thereof and reaction temperature were as shown in the synthesis route. The difference between the examples only lay in that the concentrations for the reaction of preparing the compound 18aac from the compound 17aac were different. The results were as shown in the following table:

| Example | Reaction concentration *c* (mol/L) | Yield % |
|---|---|---|
| Example 70 | 0.6 | 40% |
| Example 71 | 0.4 | 40% |
| Example 72 | 0.2 | 51% |
| Example 73 | 0.075 | 72% |
| Example 74 | 0.05 | 67% |

### Example 75 Preparation of Compound 18 (Application of Preferred Concentration for Intramolecular Oxidative Dearomatization Heck Reaction)

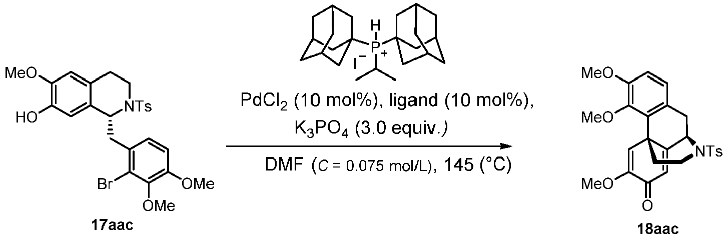

The synthesis steps of the compound 18aac in example 73 were the same as those in example 67. Conditions such as the synthesis reagent, amount thereof and reaction temperature were as shown in the synthesis route. The difference from example 67 only lay in that the concentrations for the reaction of preparing the compound 18aac from the compound 17aac were different. The results were as shown in the following table:

| Example | Reaction concentration *c* (mol/L) | Yield % |
|---|---|---|
| Example 75 | 0.075 | 73% |

### Examples 76-87 Preparation of Compound 18 (Screening of Ligand for Intramolecular Oxidative Dearomatization Heck Reaction)

Taking that X was bromine, R₁ was Me and R was Ts as an example, a compound 18aac was synthesized through the following synthesis route:

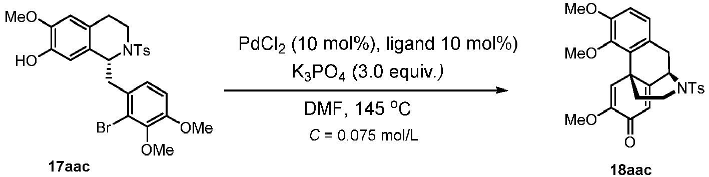

The synthesis steps of the compound 18aac in examples 76-87 were the same as those in example 75. The used alkali was potassium phosphate. Conditions such as the synthesis reagent, amount thereof and reaction temperature were as shown in the synthesis route. The difference between the examples only lay in that the ligands used in the reaction of preparing the compound 18aac from the compound 17aac were different. The results were as shown in the following table:

| Example | Ligand | Yield % | Example | Ligand | Yield % |
|---|---|---|---|---|---|
| Example 76 | 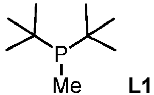 | 18% | Example 82 | 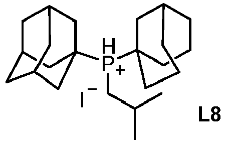 | 21% |
| Example 77 | 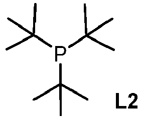 | Trace | Example 83 | 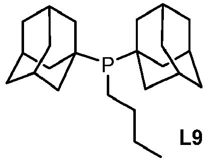 | 70% |
| Example 78 | 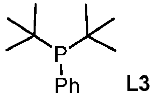 | trace | Example 84 | 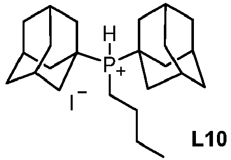 | 69% |
| Example 79 | 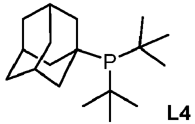 | 10% | Example 85 | 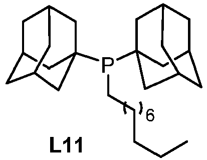 | 69% |
| Example 80 | 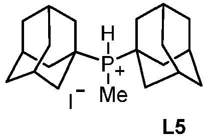 | 8% | Example 86 | 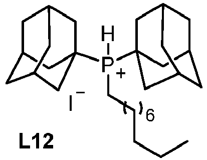 | 68% |
| Example 81 | 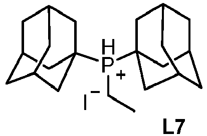 | 70% | Example 87 | 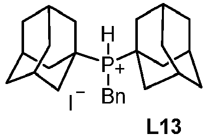 | 5% |

### Examples 88-97 Preparation of Compound 18 (Screening of Ratio of Amount of Metal Catalyst to Amount of Ligand for Intramolecular Oxidative Dearomatization Heck Reaction)

Taking that X was bromine, R₁ was Me and R was Ts as an example, a compound 18aac was synthesized through the following synthesis route:

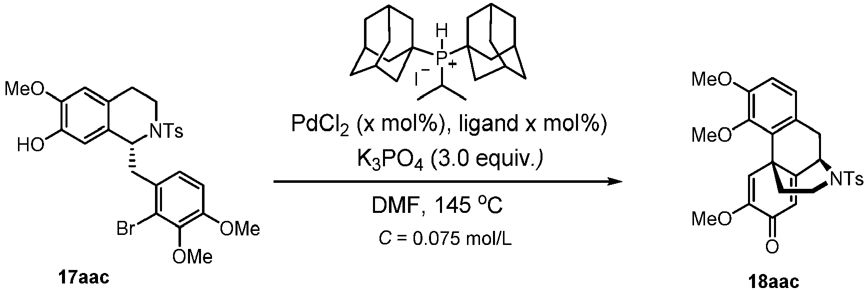

The synthesis steps of the compound 18aac in examples 88-97 were the same as those in example 75. Conditions such as the synthesis reagent, amount thereof and reaction temperature were as shown in the synthesis route. The difference between the examples only lay in that the amounts of palladium chloride and the ligand used in the reaction of preparing the compound 18aac from the compound 17aac were different. The results were as shown in the following table:

| Example | Amount of PdCl₂ (mol%) | Amount of ligand (mol%) | Yield % |
|---|---|---|---|
| Example 88 | 10% | 20% | 76% |
| Example 89 | 10% | 30% | 82% |
| Example 90 | 10% | 40% | 80% |
| Example 91 | 10% | 50% | 79% |
| Example 92 | 7.5% | 22.5% | 74% |
| Example 93 | 7.5% | 15% | 71% |
| Example 94 | 5% | 15% | 73% |
| Example 95 | 5% | 10% | 67% |
| Example 96 | 2.5% | 7.5% | 51% |
| Example 97 | 2.5% | 5% | 46% |

### Examples 98-100 Preparation of Compound 18 (Screening of Preferred Ratio of Amount of Ligand to Amount of Metal Catalyst for Intramolecular Oxidative Dearomatization Heck Reaction)

Taking that X was bromine, R₁ was Me and R was Ts as an example, a compound 18aac was synthesized through the following synthesis route:

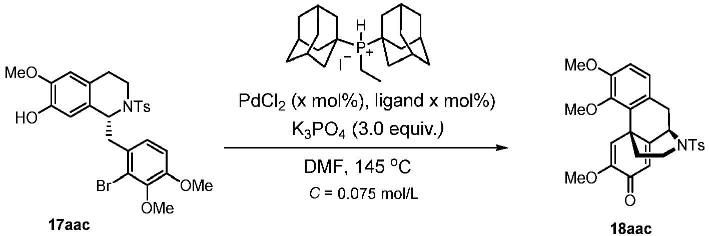

The synthesis steps of the compound 18aac in examples 98-100 were the same as those in example 81. Conditions such as the synthesis reagent, amount thereof and reaction temperature were as shown in the synthesis route. The difference between the examples only lay in that the amounts of palladium chloride and the ligand used in the reaction of preparing the compound 18aac from the compound 17aac were different. The results were as shown in the following table:

| Example | Amount of PdCl₂ (mol%) | Amount of ligand (mol%) | Yield % |
|---|---|---|---|
| Example 98 | 5% | 15% | 72% |
| Example 99 | 7.5% | 22.5% | 73% |
| Example 100 | 10% | 30% | 79% |

### Examples 101-102 Preparation of Compound 18 (Screening of Preferred Ratio of Amount of Ligand to Amount of Metal Catalyst for Intramolecular Oxidative Dearomatization Heck Reaction)

Taking that X was bromine, R₁ was Me and R was Ts as an example, a compound 18aac was synthesized through the following synthesis route:

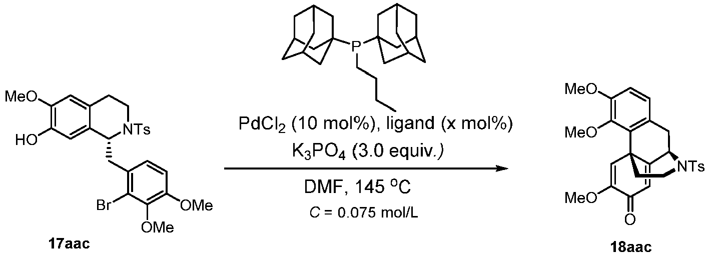

The synthesis steps of the compound 18aac in examples 101-102 were the same as those in example 83. Conditions such as the synthesis reagent, amount thereof and reaction temperature were as shown in the synthesis route. The difference between the examples only lay in that the amounts of the ligand used in the reaction of preparing the compound 18aac from the compound 17aac were different. The results were as shown in the following table:

| Example | Amount of PdCl₂ (mol%) | Amount of ligand (mol%) | Yield % |
|---|---|---|---|
| Example 101 | 10% | 20% | 74% |
| Example 102 | 10% | 30% | 80% |

### Examples 103-104 Preparation of Compound 18 (Screening of Preferred Ratio of Amount of Ligand to Amount of Metal Catalyst for Intramolecular Oxidative Dearomatization Heck Reaction)

Taking that X was bromine, R₁ was Me and R was Ts as an example, a compound 18aac was synthesized through the following synthesis route:

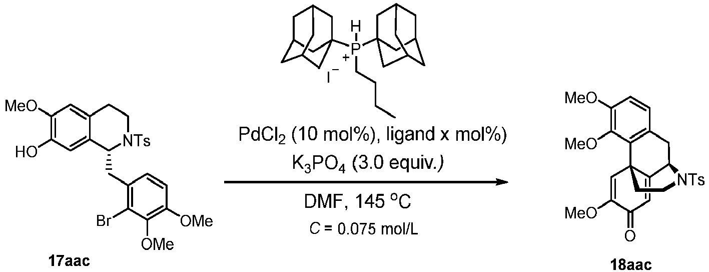

The synthesis steps of the compound 18aac in examples 103-104 were the same as those in example 84. Conditions such as the synthesis reagent, amount thereof and reaction temperature were as shown in the synthesis route. The difference between the examples only lay in that the amounts of the ligand used in the reaction of preparing the compound 18aac from the compound 17aac were different. The results were as shown in the following table:

| Example | Amount of PdCl₂ (mol%) | Amount of ligand (mol%) | Yield % |
|---|---|---|---|
| Example 103 | 10% | 20% | 75% |
| Example 104 | 10% | 30% | 81% |

### Examples 105-106 Preparation of Compound 18 (Application of Preferred Ratio of Amount of Ligand to Amount of Metal Catalyst for Intramolecular Oxidative Dearomatization Heck Reaction)

Taking that X was bromine, R₁ was Me and R was Ts as an example, a compound 18aac was synthesized through the following synthesis route:

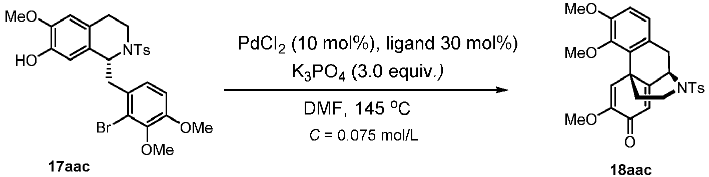

The synthesis steps of the compound 18aac in examples 105-106 were the same as those in example 89. Conditions such as the synthesis reagent, amount thereof and reaction temperature were as shown in the synthesis route. The difference between the examples only lay in that the ligands used in the reaction of preparing the compound 18aac from the compound 17aac were different. The results were as shown in the following table:

| Example | Ligand | Yield % | Example | Ligand | Yield % |
|---|---|---|---|---|---|
| Example 105 | 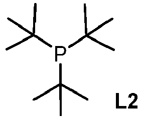 | 62% | Example 106 | 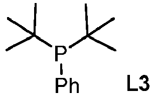 | 26% |

### Example 107 Refining of Compound 18aac

The product (R)-18aac in example 54-106 was recrystallized by using ethanol. The recrystallization yield was 80%. The ee value of the product was increased to 99.9% *(R),* M.p.: 162-164°C. **Optical rotation**: [α]_{D}²⁵ = +11.9 (*c* = 0.52, CHCl₃). HPLC conditions: AD-H column, Hexane: *i*-PrOH = 60:40, flowrate: 1mL/min, column temperature: 25°C, detection wavelength: 254 nm, *t*ₘₐⱼₒᵣ = 16.632min, *t*ₘᵢₙₒᵣ = 9.754min.

(S)-18aac was recrystallized by using isopropanol. The recrystallization yield was 93%. The ee value of the product was increased to 99.9 (S), M.p.: 162-164°C. **Optical rotation:** [α]_{D}²⁵ = -11.3 (*c* = 0.68, CHCl₃). HPLC conditions: AD-H column, Hexane: *i*-PrOH = 60:40, flowrate: 1mL/min, column temperature: 25°C, detection wavelength: 254 nm, *t*ₘₐⱼₒᵣ = 9.888min, *t*ₘᵢₙₒᵣ = 16.453min.

### Example 108 Preparation of Compound 18

Taking that X was bromine, R₁ was PMB and R was Ts as an example, a compound 18aab was synthesized through the following synthesis route:

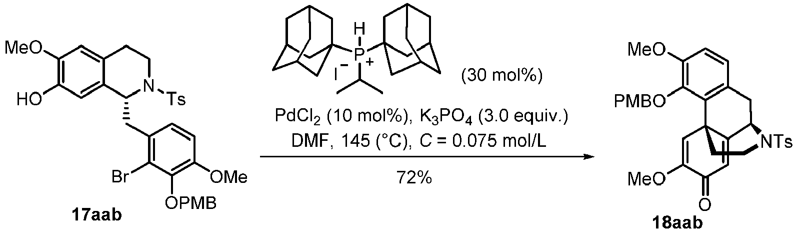

A compound 17aab (4.00g, 5.98mmol, 1.0 equiv.), palladium chloride (106mg, 0.598mmol, 0.1 equiv.), a phosphine ligand (847mg, 1.794mmol, 0.3 equiv.) and potassium phosphate (3.81g, 17.94mmol, 3.0 equiv.) were placed in a reaction vessel. Air was extracted and changed. Under the protection of argon, degassed dry DMF (80mL, c=0.075mol/L) was added. The reaction vessel was placed in an oil bath at 145°C for reaction for 40min. After TLC monitored that the raw materials disappeared completely, the reaction solution was cooled to room temperature. Water (40mL) was added at 0°C to quench the reaction. Extraction was performed by using ethyl acetate (60mL^{∗}3). The organic layers were combined. Washing was performed sequentially by using water (50mL^{∗}1) and saturated sodium chloride solution (50mL^{∗}1). The organic layer was dried by using anhydrous magnesium sulfate. Filtering and concentration were performed. The obtained crude product was purified by silica gel column chromatography (petroleum ether/dichloromethane/acetone=15:15:1, v/v) to obtain a white foam-like solid 18aab (2.07g, with yield of 72%). **Data of 18aab: Optical rotation:** [α]_{D}²⁵ = +57.1 (*c* = 0.8, CHCl₃). **¹H NMR** (400 MHz, CDCl₃) *δ* 7.65 (d, *J* = 8.0 Hz, 2H), 7.37 (d, *J* = 8.4 Hz, 2H), 7.26 (d, *J* = 8.4 Hz, 2H), 7.20 (s, 1H), 6.92-6.79 (m, 4H), 6.17 (s, 1H), 5.26 (d, *J* = 11.2 Hz, 1H), 4.94-4.92 (m, 2H), 3.88 (s, 3H), 3.82 (s, 3H), 3.69 (dd, *J* = 13.6, 3.6 Hz, 1H), 3.40 (s, 3H), 3.31-3.19 (m, 2H), 3.06 - 2.99 (m, 1H), 2.40 (s, 3H), 2.14 (d, *J* = 12.8 Hz, 1H), 1.33-1.25 (m, 1H).**¹³C NMR** (100 MHz, CDCl₃) *δ* 180.8, 159.6, 157.7, 152.0, 151.2, 146.3, 143.9, 137.2, 130.3, 130.0, 129.5, 129.1, 128.0, 127.1, 124.0, 122.4, 120.3, 114.1, 112.4, 74.2, 56.0, 55.4, 54.9, 43.6, 40.3, 39.2, 21.6. **IR** (neat): *v*ₘₐₓ = 1673, 1648, 1613, 1513, 1480, 1277, 1248, 1215, 1202, 1157, 718 cm⁻¹. **HRMS (m/z):** [M + H]⁺ calculated for C₃₃H₃₄NO₇S⁺, 588.2050; found, 588.2051.

### Examples 109-110 Preparation of Compound 18

Taking that X was bromine, R₁ was PMB and R was Ts as an example, a compound 18aab was synthesized through the following synthesis route:

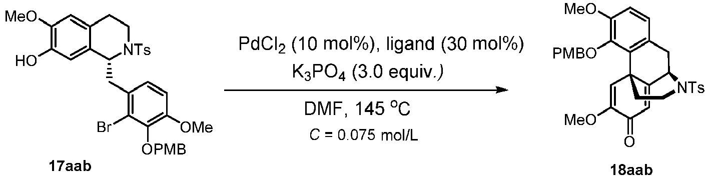

The synthesis steps of the compound 18aab in examples 109-110 were the same as those in example 108. Conditions such as the synthesis reagent, amount thereof and reaction temperature were as shown in the synthesis route. The difference between the examples only lay in that the ligands used in the reaction of preparing the compound 18aac from the compound 17aab were different. The results were as shown in the following table:

| Example | Ligand | Yield % | Example | Ligand | Yield % |
|---|---|---|---|---|---|
| Example 109 | 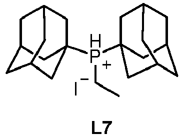 | 71% | Example 110 | 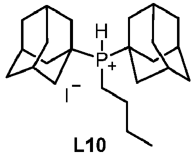 | 71% |

### Example 111 Preparation of Compound 18

Taking that X was bromine, R₁ was Bn and R was Ts as an example, a compound 18aaa was synthesized through the following synthesis route:

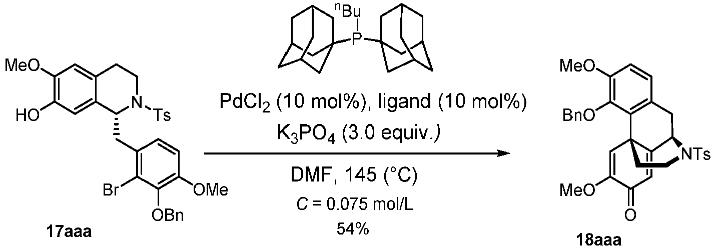

The synthesis steps of the compound 18aab were the same as those in example 83. Conditions such as the synthesis reagent, amount thereof and reaction temperature were as shown in the synthesis route. **¹H NMR** (400 MHz, CDCl₃) *δ* 7.65 (d, *J* = 8.4 Hz, 2H), 7.46-7.45 (m, 2H), 7.41-7.32 (m, 3H), 7.25 (d, *J* = 8.0 Hz, 2H), 7.15 (s, 1H), 6.88-6.81 (m, 2H), 6.18 (s, 1H), 5.30 (s, 1H, overlapped), 5.28 (d, *J* = 12.4 Hz, 1H, overlapped), 5.07 (d, *J* = 11.6 Hz, 1H), 4.93 (d, *J* = 3.6 Hz, 1H), 3.87 (s, 3H), 3.69 (dd, *J* = 14.0, 4.0 Hz, 1H), 3.34 (s, 3H), 3.28-3.20 (m, 1H), 3.07-3.00 (m, 1H), 2.40 (s, 3H), 2.16 (d, *J* = 12.4 Hz, 1H), 1.31 (dd, *J* = 13.2, 5.2 Hz, 1H).**¹³C NMR** (100 MHz, CDCl₃) *δ* 180.8, 157.7, 152.1, 151.3, 146.2, 143.9, 137.5, 137.2, 130.4, 123.0, 128.8, 128.2, 128.1, 127.3, 127.1, 124.2, 122.5, 120.1, 112.5, 74.3, 56.0, 54.9, 43.7, 40.3, 39.2, 21.7. **IR** (neat): *v*ₘₐₓ = 1673, 1647, 1615, 1480, 1433, 1278, 1215, 1158, 747 cm⁻¹. **IR** (neat): *v*ₘₐₓ= 1673, 1647, 1615, 1480, 1433, 1278, 1215, 1158, 747 cm⁻¹. **HRMS (m/z):** [M + H]⁺ calculated for C₃₂H₃₂NO₆S⁺, 558.1945; found, 558.1940.

### Example 112 Preparation of Compound 19

Taking that R₁ was PMB and R was Ts as an example, a compound 19 was synthesized through the following synthesis route:

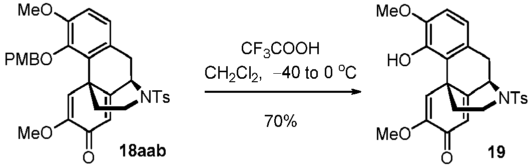

A compound 18aab (100.0mg, 0.170mmol, 1.0 equiv.) was dissolved in CH₂Cl₂ (4mL). Cooling to -40°C was performed. Trifluoroacetic acid (65uL, 0.851mmol, 5.0 equiv.) was added for reaction for 17h. Then, the temperature was increased to 0°C for continuous reaction for 7h. After TLC showed that the raw materials disappeared completely, saturated NaHCO₃ aqueous solution (2mL) was added at 0°C to quench the reaction. Extraction was performed by using CH₂Cl₂ (5mL^{∗}3). The organic layers were combined. Drying was performed by using anhydrous magnesium sulfate. Filtering and concentration were performed. The obtained crude product was purified by silica gel column chromatography (petroleum ether/acetone=3:1, v/v) to obtain a white foam-like solid 19 (56.0mg, with yield of 70%). **Optical rotation:** [α]_{D}²⁵ = +13.8 (*c* = 1.44, CHCl₃). **¹H NMR** (400 MHz, CDCl₃) *δ* 7.67 (d, *J* = 8.2 Hz, 2H), 7.45 (s, 1H), 7.28 (s, 1H), 6.76 (d, *J* = 8.4 Hz, 1H), 6.60 (d, *J* = 8.4 Hz, 1H), 6.23 (d, *J* = 15.6 Hz, 2H), 4.96 (d, *J* = 3.3 Hz, 1H), 3.89 (s, 3H), 3.74-3.71 (m, 1H, overlapped), 3.71 (s, 3H), 3.30-3.18 (m, 2H), 3.03 (td, *J* = 13.2, 3.2 Hz, 1H), 2.41 (s, 3H), 2.41-2.36 (m, 1H, overlapped), 1.31 (td, *J* = 12.8, 4.8 Hz, 1H). **¹³C NMR** (100 MHz, CDCl₃) *δ* 180.9, 157.8, 151.3, 145.7, 143.9, 143.5, 137.3, 129.9, 128.2, 127.1, 122.5, 119.7, 110.0, 56.4, 55.0, 43.4, 40.5, 38.9, 37.6, 21.7. IR (neat): *v*ₘₐₓ = 3350, 2929, 1670, 1640, 1484, 1219, 1158, 1054 cm⁻¹. **HRMS** (m/z): [M + H]⁺ calculated for C₂₅H₂₆NO₆S⁺, 468.1475; found, 468.1477.

### Example 113 Preparation of Compound 19

Taking that R₁ was PMB and R was Ts as an example, a compound 19 was synthesized through the following synthesis route:

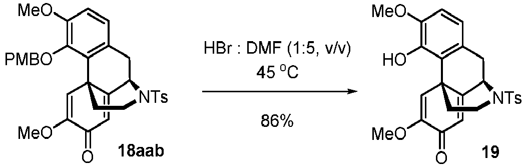

A compound 18aab (200.0mg, 0.34mmol, 1.0 equiv.) was dissolved in DMF (3.5mL). Hydrobromic acid (48% aqueous solution, 0.7mL) was dropped at room temperature. Then, the temperature was increased to 45°C for reaction for 20h. Then, the reaction solution was cooled to room temperature. Hydrobromic acid (48% aqueous solution, 0.3mL) was added. Then, the temperature was increased to 45°C for reaction for 15h. The reaction solution was cooled to room temperature again. Hydrobromic acid (48% aqueous solution, 0.3mL) was supplemented. Then, the temperature was increased to 45°C for reaction for 5h. After TLC showed that the raw materials disappeared completely, saturated NaHCO₃ aqueous solution was added at 0°C until no gas was produced. Extraction was performed by using ethyl acetate (8mL^{∗}3). The organic layers were combined. Washing was performed sequentially by using water (5mL^{∗}1) and saturated sodium chloride (5mL^{∗}1). The organic layer was dried by using anhydrous magnesium sulfate. Filtering and concentration were performed. The obtained crude product was purified by silica gel column chromatography (petroleum ether/acetone=4:1, v/v) to obtain a white foam-like solid 19 (137mg, with yield of 86%). The hydrogen spectrum data was the same as that in example 112.

### Example 114 Preparation of Compound 19

Taking that R₁ was Me and R was Ts as an example, a compound 19 was synthesized through the following synthesis route:

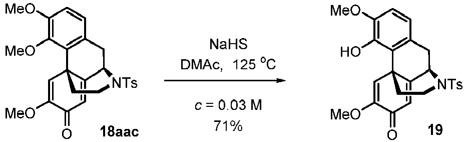

A compound 18aac (100.0mg, 0.208mmol, 1.0 equiv.) was dissolved in dry N,N-dimethylacetamide (DMAC, 7mL). Sodium hydrosulfide (68%-72% purity, 66.4mg, 0.83mmol, 4.0 equiv.) was added for reaction for 1h at 125°C. After TLC showed that the raw materials disappeared completely, cooling to 0°C was performed. Then, 0.5M HCl aqueous solution was added to quench the reaction. Extraction was performed by using ethyl acetate (5mL^{∗}4). The organic layers were combined. Washing was performed sequentially by using water (5mL^{∗}2), saturated NaHCO₃ aqueous solution (5mL^{∗}1) and saturated NaCl aqueous solution (5mL^{∗}1). The organic layer was dried by using anhydrous magnesium sulfate. Filtering and concentration were performed. The obtained crude product was purified by silica gel column chromatography (petroleum ether/acetone=4:1, v/v) to obtain a white solid 19 (69.0mg, with yield of 71%). The hydrogen spectrum data was the same as that in example 112.

### Example 115 Preparation of Intermediate

Taking that R was Ts as an example, a compound 21 (i.e., intermediate I) was synthesized through the following synthesis route:

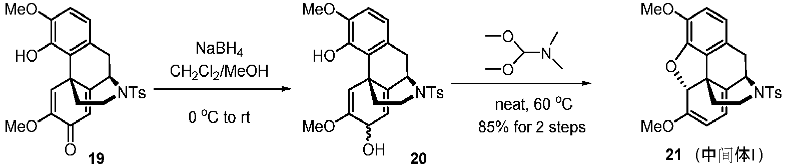

A compound 19 (510.0mg, 1.09mmol, 1.0 equiv.) was dissolved in CH₂Cl₂/MeOH mixed solution (v/v=1:1, 10mL). Cooling to 0°C was performed. NaBH₄ (82.5mg, 2.18mmol, 2.0 equiv.) was slowly added. Then, the reaction solution was heated to room temperature for reaction for about 15min. After TLC detected that the raw materials disappeared completely, the reaction solution was cooled to 0°C. Water was added to quench the reaction. The organic layer was separated. The water layer was extracted by using dichloromethane (10mL^{∗}3). The organic layers were combined. Washing was performed by using saturated NaCl solution (10mL^{∗}1). Drying was performed by using anhydrous magnesium sulfate. Filtering and concentration were performed to obtain a crude white foam-like compound 20, which was directly used for next reaction without purification.

Under the protection of argon, the crude compound 20 was placed into a reaction flask. Air was extracted and changed. Under the protection of argon, N,N-dimethylformamide dimethyl acetal (2.5mL) was added. Heating to 60°C was performed for reaction for about 40min. After TLC showed that the reaction of the raw materials was complete, the pressure of the reaction solution was reduced to remove the solvent. The obtained crude product was separated and purified by silica gel column chromatography (petroleum ether/ethyl acetate, v/v=5:1 to 3.5:1) to obtain a white foam-like solid 21 (449mg, with two-step yield of 85%). **Optical rotation:** [α]_{D}²⁵ = -117.8 (*c* = 0.72, CHCl₃). **¹H NMR** (400 MHz, CDCl₃) *δ* 7.73 (d, *J* = 8.0, 2H), 7.30 (d, *J* = 8.0 Hz, 2H), 6.64 (d, *J* = 8.0 Hz, 1H), 6.51 (d, *J* = 8.0 Hz, 1H), 5.60 (d, *J* = 6.4 Hz, 1H), 5.19 (s, 1H), 4.98 (dd, *J* = 13.6, 6.4 Hz, 2H), 3.82 (s, 3H), 3.74 (dd, *J* = 12.0, 5.2 Hz, 1H), 3.59 (s, 3H), 3.26 (td, *J* = 13.2, 3.6 Hz, 1H), 3.00 (dd, *J* = 18.2, 6.8 Hz, 1H), 2.89 (d, *J* = 18.0 Hz, 1H), 2.44 (s, 3H), 1.95 (td, *J* = 12.8, 5.4 Hz, 1H), 1.74-1.70 (m, 1H). **¹³C NMR** (100 MHz, CDCl₃) *δ* 153.0, 144.8, 143.4, 143.1, 137.3, 132.1, 129.7, 129.0, 127.4, 126.1, 119.5, 113.2, 112.5, 95.4, 88.6, 56.4, 55.0, 54.4, 45.9, 38.9, 37.1, 36.0, 21.6. IR (neat): *v*ₘₐₓ = 2922, 1603, 1502, 1234, 1155, 725 cm⁻¹. **HRMS (m/z):** [M + H]⁺ calculated for C₂₅H₂₆NO₅S⁺, 452.1526; found, 452.1519.

### Example 116 Preparation of Compound 22

Taking that R was Ts as an example, a compound 22 was synthesized through the following synthesis route:

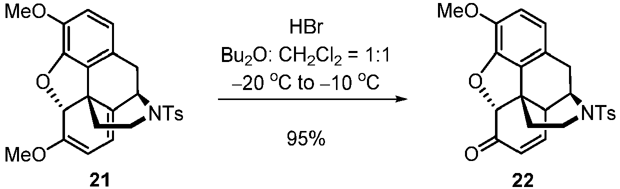

Air in a reaction flash was extracted and changed. Under the protection of argon, n-butyl ether (2.0mL) and 48% HBr aqueous solution (94uL, 0.555mmol, 5.0 equiv.) were added. Cooling to -20°C was performed. Under stirring, dry CH₂Cl₂ (2.0mL) solution of a compound 21 (50.0mg, 0.111mmol, 1.0 equiv.) was dropped for reaction for 20h at -20°C. Then the temperature was increased to -10°C for reaction for about 10h. After TLC monitored that the raw materials disappeared completely, saturated NaHCO₃ aqueous solution (5mL) was added to quench the reaction. Extraction was performed by using dichloromethane (5mL^{∗}4). The organic layers were combined. Drying was performed by using anhydrous magnesium sulfate. Filtering and concentration were performed. The obtained crude product was separated and purified by silica gel column chromatography (petroleum ether/dichloromethane/acetone, v/v/v=15:15:1 to 10:10:1) to obtain a white foam-like solid 22 (46mg, with yield of 95%). **Optical rotation:** [α]_{D}²⁵ = -149.0 (*c* = 0.52, CHCl₃). **¹H NMR** (400 MHz, CDCl₃) *δ* 7.74 (d, *J* = 8.0 Hz, 2H), 7.34 (d, *J* = 8.0Hz, 2H), 6.66 (d, *J* = 4 Hz, 1H), 6.58 (dd, *J* = 8.0, 2.0 Hz, 1H), 6.50 (d, *J* = 8.0 Hz, 1H), 6.10 (dd, *J* = 10.0, 2.8 Hz, 1H), 4.72-4.78 (m, 1H), 4.65 (s, 1H), 3.83 (s, 3H), 3.82-3.73 (m,1H), 3.09 (q, *J* = 2.8 Hz, 1H), 2.96-2.85 (td, *J* = 12.4, 4, 1H), 2.74-2.59 (m, 2H), 2.45 (s, 3H), 1.98 (td, *J* = 12.4, 5.2 Hz, 1H), 1.89 (ddd, *J* = 12.8, 4.0, 1.6 Hz, 1H). **¹³C NMR** (100 MHz, CDCl₃) *δ* 193.6, 146.5, 144.9, 143.7, 142.9, 137.3, 133.3, 130.0, 127.6, 127.1, 124.2, 120.4, 115.3, 87.7, 56.8, 52.4, 43.4, 40.8, 39.3, 33.4, 28.1, 21.6. **IR (neat):** *v*ₘₐₓ = 2923, 2852, 1676, 1505, 1442, 1278, 1158 cm⁻¹. **HRMS (m/z):** [M + H]⁺ calculated for C₂₄H₂₄NO₅S⁺, 438.1370; found, 438.1362.

### Example 117 Preparation of Compound 24 (Codeine)

A compound 24 (codeine) was synthesized through the following synthesis route:

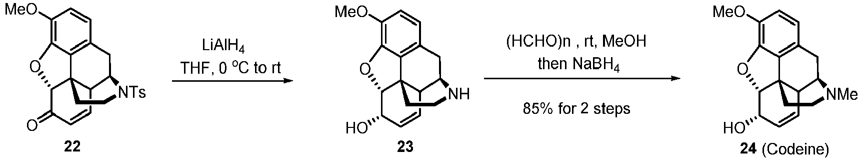

A compound 22 (10.0mg, 0.023mmol, 1.0 equiv.) was placed in a reaction flask. Air was extracted and changed. Under the protection of argon, dry tetrahydrofuran (1.0mL) was added. Cooling to 0°C was performed. Lithium aluminum hydride (1M tetrahydrofuran solution, 0.115mmol, 5.0 equiv.) was added. Then, the temperature was increased to room temperature for reaction for about 10h. After TLC showed that the reaction of the raw materials was complete, cooling to 0°C was performed. Isopropanol (35uL) was added. Stirring was performed for 5min. Water (5uL) was added. Stirring was performed for 5min. Then, 15% sodium hydroxide aqueous solution (5uL) and water (15uL) were added sequentially. The reaction solution was heated to room temperature. Stirring was continuously performed for 30min. The obtained mixture was filtered by using diatomite. The filter cake was washed by using dichloromethane. Reduced-pressure concentration was performed on the filtrate to obtain a crude compound 23, which was directly used for next reaction without separation and purification.

The crude product 23 was dissolved in methanol (1.0mL). Polyformaldehyde (11.0mg, 0.115mmol, 5.0 equiv.) was added. Stirring was performed for 1.5h at room temperature. After LC-MS detected that the raw materials disappeared completely, cooling to 0°C was performed. Sodium borohydride (6.0mg, 0.14mmol, 6.0 equiv.), was added. Then, the reaction solution was heated to room temperature for reaction for 20min. After TLC and LC-MS showed that the reaction was complete, the reaction solution was cooled to 0°C. Saturated ammonium chloride aqueous solution was added to quench the reaction. Dichloromethane (1.0mL) was added to dilute the reaction solution. The pH of the water layer was regulated to 10 by using 15% sodium hydroxide aqueous solution. The organic layer was separated. The water layer was extracted by using dichloromethane (3mL^{∗}4). The organic layers were combined. Drying was performed by using anhydrous sodium sulfate. Filtering and reduced-pressure concentration were performed. The obtained crude product was separated and purified by silica gel column chromatography (dichloromethane/methanol v/v=20:1 to 8:1, gradient elution) to obtain a white solid 24 (6.0mg, with yield of 85%). **Optical rotation:** [α]_{D}²⁵ = -131.0 (*c* = 0.3, EtOH). **¹H NMR** (400 MHz, CDCl₃) *δ* 6.66 (d, *J* = 8.0 Hz, 1H), 6.57 (d, *J* = 8.0 Hz, 1H), 5.71 (d, *J* = 10,0 Hz, 1H), 5.29 (dt, *J* = 10.0, 2.8 Hz, 1H), 4.89 (d, *J* = 6.4 Hz, 1H), 4.21-4.15 (m, 1H), 3.84 (s, 3H), 3.37 (dd, *J* = 6.4, 3.2 Hz, 1H), 3.05 (d, *J* = 18.8 Hz, 1H), 2.76-2.66 (m, 1H), 2.62 (dd, *J* = 12.4, 5.2 Hz, 1H), 2.46 (s, 3H), 2.44-2.37 (m, 1H), 2.32 (dd, *J* = 18.4, 6.0 Hz, 1H), 2.09 (td, *J* = 12.4, 5.2 Hz, 1H), 1.88 (d, *J* = 12.4 Hz, 1H).**¹³C NMR** (100 MHz, CDCl₃) *δ* 146.3, 142.2, 133.5, 131.0, 128.1, 127.0, 119.6, 112.9, 91.3, 66.3, 58.9, 56.3, 46.5, 43.0, 42.9, 40.6, 35.6, 20.5. **IR (neat):** *v*ₘₐₓ= 3370, 2929, 2843, 1635, 1502, 1452, 1274, 1265, 1205, 1120, 1054, 731 cm⁻¹. **HRMS (m/z):** [M + H]⁺ calculated for C₁₈H₂₂NO₃⁺, 300.1594; found, 300.1587.

### Example 118 Preparation of Compound 26

Taking that R was Ts as an example, a compound 26 was synthesized through the following synthesis route:

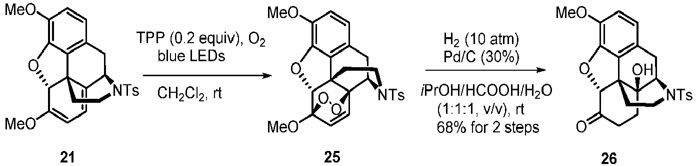

The compound 21 (50.0mg, 0.11mmol, 1.0 equiv.) and tetraphenylporphyrin (14.0mg, 0.022mmol, 0.2 equiv.) were dissolved in dichloromethane (2mL). Under the irradiation of blue LED light (40w, Kessil^{®}), bubbling was performed by using oxygen for reaction for 1h at room temperature. After TLC detected that the raw materials disappeared completely, argon was fed into the reaction solution for ultrasonic degassing for 30min to remove residual oxygen in the solution. Isopropanol/formic acid/water (v/v/v=1:1:1, 1.5mL) and 10% palladium carbon (15mg, 30% wt.) were added to the reaction solution for reaction for 12h at room temperature under hydrogen temperature of 10atm in a high-pressure hydrogenation kettle. After TLC showed that the reaction was complete, the reaction solution was filtered by using diatomite. The filter cake was washed three times by using dichloromethane. The filtrate was concentrated. The crude product was purified by silica gel column chromatography (petroleum ether/acetone=3:1 v/v) to obtain a compound 26 (34mg, with two-step yield of 68%, brown yellow foam-like solid). **Optical rotation:** [α]_{D}²⁵ = -188.1 (*c* = 0.84, CHCl₃). **¹H NMR** (400 MHz, CDCl₃) *δ* 7.72 (d, *J* = 8.0 Hz, 2H), 7.35 (d, *J* = 8.0 Hz, 2H), 6.67 (d, *J* = 8.4 Hz, 1H), 6.47 (d, *J* = 8.0 Hz, 1H), 4.65 (s, 1H), 4.22 (d, *J* = 6.0 Hz, 1H), 3.87 (s, 3H), 3.74 (dd, *J* = 12.8, 4.8 Hz, 1H), 3.23 (s, 1H), 3.03 (td, *J* = 14.4, 5.2 Hz, 1H), 2.81 (dd, *J* = 18.8, 6.0 Hz, 1H), 2.72 (td, *J* = 12.8, 3.6 Hz, 1H), 2.57 (d, *J =* 18.4 Hz, 1H), 2.50-2.39 (m, 1H, overlapped), 2.46 (s, 3H), 2.29 (dt, *J* = 14.4, 2.8 Hz, 1H), 1.96-1.88 (m, 2H), 1.70-1.52 (m, 2H). **¹³C NMR** (100 MHz, CDCl₃) *δ* 207.5, 145.1, 144.1, 143.4, 136.4, 130.1, 128.2, 127.2, 123.2, 119.8, 115.4, 89.9, 70.2, 58.7, 56.8, 50.3, 38.6, 35.9, 31.1, 29.4, 29.2, 21.6. **IR (neat):** *v*ₘₐₓ = 3492, 2929, 1726, 1504, 1440, 1278, 1157, 1048, 753 cm⁻¹. **HRMS (m/z):** [M + H]⁺ calculated for C₂₄H₂₆NO₆S⁺, 456.1475; found, 456.1481.

### Example 119 Preparation of Compound 29

A compound 29 (oxycodone) was synthesized through the following synthesis route:

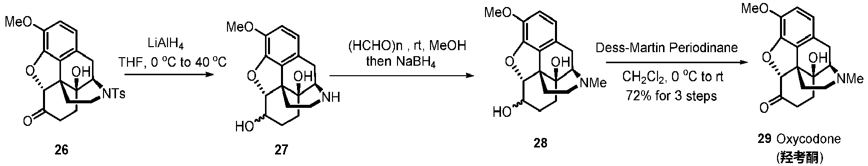

A compound 26 (25.0mg, 0.055mmol, 1.0 equiv.) was placed in a reaction flask. Air was extracted and changed. Under the protection of argon, dry tetrahydrofuran (2.0mL) was added. Cooling to 0°C was performed. Lithium aluminum hydride (1M tetrahydrofuran solution, 0.28mL, 0.28mmol, 5.0 equiv.) was added. Then, the temperature was increased to 40°C for reaction for about 24h. After TLC showed that the reaction of the raw materials was complete, cooling to 0°C was performed. Isopropanol (19uL) was added. Stirring was performed for 5min. Water (11uL) was added. Stirring was performed for 5min. Then, 15% sodium hydroxide aqueous solution (11uL) and water (33uL) were added sequentially. The reaction solution was heated to room temperature. Stirring was continuously performed for 30min. The obtained mixture was filtered by using diatomite. The filter cake was washed by using dichloromethane. Reduced-pressure concentration was performed on the filtrate to obtain a crude compound 27, which was directly used for next reaction without separation and purification.

The crude product 27 was dissolved in methanol (2mL). Polyformaldehyde (30.0mg, 0.33mmol, 6.0 equiv.), was added. Stirring was performed for 2h at room temperature. Then, cooling to 0°C was performed. Sodium borohydride (17.0mg, 0.44mmol, 8.0 equiv.) was added. Then, the reaction solution was heated to room temperature for reaction for 20min. After TLC and LC-MS showed that the reaction was complete, the reaction solution was cooled to 0°C. Saturated ammonium chloride aqueous solution was added to quench the reaction. Dichloromethane (2.0mL) was added to dilute the reaction solution. The pH of the water layer was regulated to 9 by using 15% sodium hydroxide aqueous solution. The organic layer was separated. The water layer was extracted by using dichloromethane (3mL^{∗}4). The organic layers were combined. Drying was performed by using anhydrous sodium sulfate. Filtering and reduced-pressure concentration were performed. The obtained crude product was directly used for next reaction without separation and purification.

The crude product 28 was dissolved in dichloromethane (2.5mL). Cooling to 0°C was performed in an ice bath. Dess-Martin oxidant (70.0mg, 0.165mmol, 3.0 equiv.) was added. The temperature was increased to room temperature for reaction for 1h after which the reaction was complete. Cooling to 0°C was performed. Saturated Na₂S₂O₃ aqueous solution and saturated NaHCO₃ aqueous solution were added sequentially to quench the reaction. Layering was allowed. The water layer was extracted by using dichloromethane (3mL^{∗}4). The organic layers were combined. Drying was performed by using anhydrous sodium sulfate. Filtering and reduced-pressure concentration were performed. The crude product was separated and purified by silica gel column chromatography (dichloromethane/methanol, v/v=20:1) to obtain a white solid 29 (12.0mg, with three-step yield of 72%). M.p.: 204-206°C. **Optical rotation:** [α]_{D}²⁵ = -205 (*c* = 0.28, CHCl₃). **¹H NMR** (400 MHz, CDCl₃) *δ* 6.70 (d, *J* = 8.0 Hz, 1H), 6.63 (d, *J* = 8.0 Hz, 1H), 4.66 (s, 1H), 3.90 (s, 3H), 3.15 (d, *J* = 18.8 Hz, 1H), 3.02 (td, *J* = 14.4, 4.8 Hz, 1H), 2.87 (d, *J* = 5.6 Hz, 1H), 2.56 (dd, *J* = 18.8, 6.0 Hz, 1H), 2.51-2.36 (m, 2H), 2.41 (s, 3H), 2.29 (dt, *J* = 14.4, 3.2 Hz, 1H), 2.22-2.11 (m, 1H), 1.92-1.82 (m, 1H), 1.68-1.61 (m, 1H), 1.61-1.53 (m, 1H). **¹³C NMR** (100 MHz, CDCl3) *δ* 208.5, 145.0, 143.0, 129.4, 124.9, 119.4, 114.9, 90.4, 70.4, 64.6, 56.8, 50.2, 45.2, 42.7, 36.1, 31.4, 30.5, 21.9. **IR (neat):** *v*ₘₐₓ = 3380, 2926, 1724, 1610, 1502, 1438, 1272, 1112, 1039, 941 cm⁻¹. **HRMS (m/z):** [M + H]⁺ calculated for C₁₈H₂₂NO₄⁺, 316.1543; found, 316.1524.

### Example 120 Preparation of Compound 32

A compound 32 (naltrexone) was synthesized through the following synthesis route:

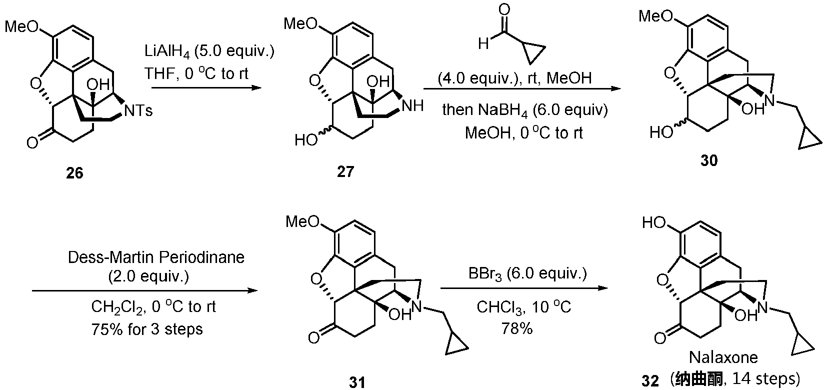

A compound 26 (25.0mg, 0.055mmol, 1.0 equiv.) was placed in a reaction flask. Air was extracted and changed. Under the protection of argon, dry tetrahydrofuran (2.0mL) was added. Cooling to 0°C was performed. Lithium aluminum hydride (1M tetrahydrofuran solution, 0.28mL, 0.28mmol, 5.0 equiv.) was added. Then, the temperature was increased to 40°C for reaction for about 24h. After TLC showed that the reaction of the raw materials was complete, cooling to 0°C was performed. Isopropanol (19uL) was added. Stirring was performed for 5min. Water (11uL) was added. Stirring was performed for 5min. Then, 15% sodium hydroxide aqueous solution (11uL) and water (33uL) were added sequentially. The reaction solution was heated to room temperature. Stirring was continuously performed for 30min. The obtained mixture was filtered by using diatomite. The filter cake was washed by using dichloromethane. Reduced-pressure concentration was performed on the filtrate to obtain a crude compound 27, which was directly used for next reaction without separation and purification.

The crude product 27 was dissolved in methanol (1mL). Cyclopropane formaldehyde (17uL, 0.22mmol, 4.0 equiv.) was added. Stirring was performed for 3h at room temperature. Then, cooling to 0°C was performed. Sodium borohydride (13.0mg, 0.33mmol, 6.0 equiv.) was added. Then, the reaction solution was heated to room temperature for reaction for 20min. After TLC and LC-MS showed that the reaction was complete, the reaction solution was cooled to 0°C. Saturated ammonium chloride aqueous solution was added to quench the reaction. Dichloromethane (1.0mL) was added to dilute the reaction solution. The pH of the water layer was regulated to 9 by using 15% sodium hydroxide aqueous solution. The organic layer was separated. The water layer was extracted by using dichloromethane (2mL*4). The organic layers were combined. Drying was performed by using anhydrous sodium sulfate. Filtering and reduced-pressure concentration were performed to obtain a crude product 30, which was directly used for next reaction without separation and purification.

Under the protection of argon, the crude product 30 was dissolved in dry dichloromethane (1mL). Cooling to 0°C was performed in an ice bath. Dess-Martin oxidant (47.0mg, 0.11mmol, 2.0 equiv.) was added. The temperature was increased to room temperature for reaction for 1h after which the reaction was complete. Cooling to 0°C was performed. Saturated Na₂S₂O₃ aqueous solution and saturated NaHCO₃ aqueous solution were added sequentially to quench the reaction. Layering was allowed. The water layer was extracted by using dichloromethane (2mL*4). The organic layers were combined. Drying was performed by using anhydrous sodium sulfate. Filtering and reduced-pressure concentration were performed. The crude product was separated and purified by silica gel column chromatography (dichloromethane/methanol, v/v=50:1) to obtain a compound 31 (15.0mg, with three-step yield of 75%). **Optical rotation:** [α]_{D}²⁵ = -202 (*c* = 0.6, CHCl₃). **¹H NMR** (400 MHz, CDCl₃) *δ* 6.69 (d, *J* = 8.0 Hz, 1H), 6.61 (d, *J* = 8.0 Hz, 1H), 4.67 (s, 1H), 3.89 (s, 3H), 3.18 (d, *J* = 6.0 Hz, 1H), 3.10-2.97 (m, 2H), 2.69 (dd, *J* = 12.0, 4.8 Hz, 1H), 2.58 (dd, *J* = 18.4, 6.0 Hz, 1H), 2.47-2.37 (m, 3H), 2.30 (dt, *J* = 14.4, 3.2 Hz, 1H), 2.13 (td, *J* = 12.0, 4.0 Hz, 1H), 1.92-1.84 (m, 1H), 1.69-1.54 (m, 2H), 0.93-0.80 (m, 1H), 0.59-0.51 (m, 2H), 0.19-0.10 (m, 2H). **¹³C NMR** (150 MHz, CDCl₃) *δ* 208.5, 145.0, 142.9, 129.5, 124.9, 119.4, 114.9, 90.4, 70.1, 62.1, 59.2, 56.8, 50.8, 43.6, 36.2, 31.5, 30.7, 22.6, 9.4, 3.9, 3.8. **IR (neat)**: *v*ₘₐₓ = 3381, 2928, 1726, 1502, 1439, 1278, 1258, 1048, 941, 799, 748 cm⁻¹. **HRMS (m/z)**: [M + H]⁺ calculated for C₂₁H₂₈NO₄⁺, 356.1856; found, 356.1857.

Under the protection of argon, the compound 31 (10.0mg, 0.028mmol, 1.0 equiv.) was dissolved in dry chloroform (0.6mL). The reaction solution was cooled to 10°C. Chloroform solution (0.4mL) of BBr₃ (1M dichloromethane solution, 169uL, 0.169mmol, 6.0 equiv.) was dropped slowly. The temperature was maintained for reaction for 4h. After TLC detected that the raw materials disappeared completely, the reaction solution was added into ice water and alkalized by using ammonia water. A small amount of saturated sodium chloride solution was added for dilution. Extraction was performed by using chloroform (2mL^{∗}10). The organic layers were combined. Drying was performed by using anhydrous sodium sulfate. Concentration was performed to obtain a crude product. The crude product was separated and purified by silica gel column chromatography (dichloromethane/methanol, v/v=40:1) to obtain a white solid 32 (7.5mg, with yield of 78%). **M.p.**: 160-162°C. **Optical rotation:** [α]_{D}²⁵ = -205 (c = 0.28, CHCl₃). **¹H NMR** (600 MHz, CDCl₃) δ 6.71 (d, *J* = 8.0 Hz, 1H), 6.59 (d, *J* = 8.0 Hz, 1H), 4.68 (s, 1H), 3.18 (d, *J* = 6.0 Hz, 1H), 3.09-2.99 (m, 2H), 2.70 (dd, *J* = 12.0, 5.4 Hz, 1H), 2.56 (dd, *J* = 18.6, 6.0 Hz, 1H), 2.47-2.37 (m, 3H), 2.32 (dt, *J* = 14.4, 3.0 Hz, 1H), 2.16 (td, *J* = 12.0, 3.6 Hz, 1H), 1.92-1.82 (m, 2H), 1.69-1.52 (m, 2H), 0.94-0.79 (m, 1H), 0.61-0.50 (m, 2H), 0.18-0.10 (m, 2H). **¹³C NMR** (150 MHz, CDCl₃) δ 209.8, 143.4, 138.7, 129.0, 124.3, 119.9, 117.8, 90.6, 70.2, 62.1, 59.2, 51.0, 43.6, 36.2, 31.3, 30.7, 22.6, 9.4, 4.0, 3.8. **IR (neat)**: *v*ₘₐₓ = 3356, 2924, 1724, 1455, 1259, 1015, 796, 757 cm⁻¹.

**HRMS (m/z)**: [M + H]⁺ calculated for C₂₀H₂₄NO₄⁺, 342.1700; found, 342.1702.

### Example 121 Preparation of Compound 35

A compound 35 (nalaxone) was synthesized through the following synthesis route:
A compound 26 (25.0mg, 0.055mmol, 1.0 equiv.) was placed in a reaction flask. Air was extracted and changed. Under the protection of argon, dry tetrahydrofuran (2.0mL) was added. Cooling to 0°C was performed. Lithium aluminum hydride (1M tetrahydrofuran solution, 0.28mL, 0.28mmol, 5.0 equiv.) was added. Then, the temperature was increased to 40°C for reaction for about 24h. After TLC showed that the reaction of the raw materials was complete, cooling to 0°C was performed. Isopropanol (19uL) was added. Stirring was performed for 5min. Water (11uL) was added. Stirring was performed for 5min. Then, 15% sodium hydroxide aqueous solution (11uL) and water (33uL) were added sequentially. The reaction solution was heated to room temperature. Stirring was continuously performed for 30min. The obtained mixture was filtered by using diatomite. The filter cake was washed by using dichloromethane. Reduced-pressure concentration was performed on the filtrate to obtain a crude compound 27, which was directly used for next reaction without separation and purification.

Under the protection of argon, the crude product 27 was dissolved in NMP/H₂O (10:1 v/v, 0.55mL). Et3N (15µL, 0.11mmol, 2.0 equiv.) was added. Under stirring, allyl bromide (7µL, 0.077mmol, 1.4 equiv.) was added slowly. Then, the reaction solution was heated to 70°C for reaction for 1h. After TLC detected that the raw materials disappeared completely, the reaction solution was cooled to room temperature. The mixture was diluted by using dichloromethane (2mL). Washing was performed by using saturated sodium bicarbonate solution (1mL*3). The water layers were combined and extracted once by using dichloromethane (3mL). The organic layers were combined. Drying was performed by using anhydrous sodium sulfate. Concentration was performed to obtain a crude product 33, which was directly used for next reaction without separation and purification.

The crude product 33 was dissolved in dry dichloromethane (1mL). Cooling to 0°C was performed in an ice bath. Dess-Martin oxidant (70.0mg, 0.165mmol, 3.0 equiv.) was added. The temperature was increased to room temperature for reaction for 1h after which the reaction was complete. Cooling to 0°C was performed. Saturated Na₂S₂O₃ aqueous solution and saturated NaHCO₃ aqueous solution were added sequentially to quench the reaction. Layering was allowed. The water layer was extracted by using dichloromethane (2mL*4). The organic layers were combined. Drying was performed by using anhydrous sodium sulfate. Filtering and reduced-pressure concentration were performed. The obtained crude product was separated and purified by silica gel column chromatography (dichloromethane/methanol, v/v=20:1) to obtain a compound 34 (13.7mg, with three-step yield of 73%). [α]_{D}²⁵ = -190.0 (c = 0.36, CHCl₃). **¹H NMR** (400 MHz, CDCl₃) *δ.* 6.71 (d, *J* = 8.2 Hz, 1H), 6.68-6.61 (m, 1H), 5.96-5.83 (m, 1H), 5.30-5.20 (m, 2H), 4.69 (s, 1H), 3.90 (s, 3H), 3.40-2.96 (m, 5H), 2.78-2.57 (m, 2H), 2.56-2.42 (m, 1H), 2.30 (dt, *J* = 14.4, 3.2 Hz, 1H), 2.20 (td, *J* = 12.0, 4.0 Hz, 1H), 1.96-1.88 (m, 1H), 1.71-1.53 (m, 2H). **¹³C NMR** (100 MHz, CDCl₃) δ 208.3, 145.1, 143.1, 134.0, 129.2, 124.3, 119.5, 119.1, 90.3, 70.3, 62.2, 57.6, 56.8, 50.5, 43.7, 36.0, 31.5, 30.1, 22.8. **IR (neat)**: *v*ₘₐₓ = 3386, 2925, 1725, 1607, 1503, 1440, 1276, 1112, 1047, 940 cm⁻¹. **HRMS (m/z):** [M + H]⁺ calculated for C₂₀H₂₄NO₄⁺, 342.1700; found, 342.1700.

Under the protection of argon, the compound 34 (10.0mg, 0.029mmol, 1.0 equiv.) was dissolved in dry chloroform (0.5mL). The reaction solution was cooled to 10°C. Chloroform solution (0.5mL) of BBr3 (1M dichloromethane solution, 176uL, 0.176mmol, 6.0 equiv.) was added slowly. The temperature was maintained for reaction for 2h. After TLC detected that the raw materials disappeared completely, the reaction solution was added into ice water and alkalized by using ammonia water. A small amount of saturated sodium chloride solution was added for dilution. Extraction was performed by using chloroform (2mL^{∗}10). The organic layers were combined. Drying was performed by using anhydrous sodium sulfate. Concentration was performed to obtain a crude product. The crude product was separated and purified by silica gel column chromatography (dichloromethane/methanol, v/v=20:1) to obtain a white solid 35 (7.2mg, with yield of 76%). **M.p.:** 176-177°C. [α]_{D}²⁵ = -195.0 (*c* = 0.5, CHCl₃). **¹H NMR** (600 MHz, CDCl₃) *δ* 6.74 (d, *J* = 8.0 Hz, 1H), 6.61 (d, *J* = 8.0 Hz, 1H), 5.90-5.75 (m, 1H), 5.35-5.10 (m, 2H), 4.69 (s, 1H), 3.30-2.92 (m, 6H), 2.77-2.51 (m, 2H), 2.50-2.36 (m,1H), 2.31 (dt, *J* = 14.4, 3.2 Hz, 1H), 2.17 (td, *J* = 12.0, 3.6 Hz,1H), 1.94-1.85 (m, 1H), 1.73-1.48 (m, 2H). **¹³C NMR** (150 MHz, CDCl₃) *δ* 209.7, 143.5, 138.9, 120.0, 135.0, 128.9, 124.0, 119.9, 118.1, 117.9, 90.5, 70.4, 62.2, 57.6, 50.9, 36.1, 31.2, 30.3, 22.8.; **IR (neat)**: *v*ₘₐₓ = 3362, 2925, 1721, 1616, 1503, 1315, 1280, 1111, 1055, 940 cm⁻¹. **HRMS (m/z)**: [M + H]⁺ calculated for Cl₉H₂₂NO₄⁺, 328.1543; found, 328.1543.

What are described above are just preferred examples of the present application. It should be understood that the present application is not limited to the examples disclosed herein, the examples should not be regarded as the exclusion of other examples, especially the synthesis of other morphine drugs from the intermediate I, the present application can be used for various other combinations, modifications and environments, and modifications can be made through the above teaching or the technology or knowledge in the related arts within the scope of the concept described herein. However, any modifications and changes made by those in the art without departing from the spirit and scope of the present application should fall within the scope of protection of the attached claims of the present application.

## Claims

1. An intermediate, wherein the structural formula is as follow: 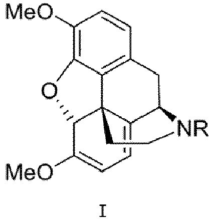 where R is a secondary amine protection group.

2. The intermediate according to claim 1, wherein the secondary amine protection group is one selected from the group consisting of benzenesulfonyl, p-toluenesulfonyl, p-nitrobenzenesulfonyl, methyl, methyl formate, tert-butoxycarbonyl, benzyl, benzyloxycarbonyl, trifluorsulfonyl, methanesulfonyl and trimethylbenzenesulfonyl.

3. A method for preparing the intermediate according to claim 1 or 2, wherein the method comprises the following steps: 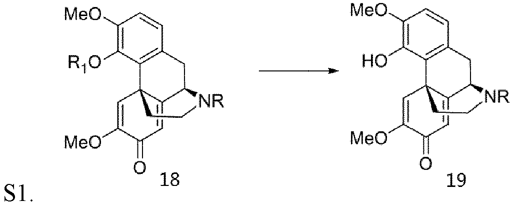
providing a compound 18 and producing a compound 19 through removal reaction of a hydroxyl protection group R₁, where R₁ is a hydroxyl protection group I; 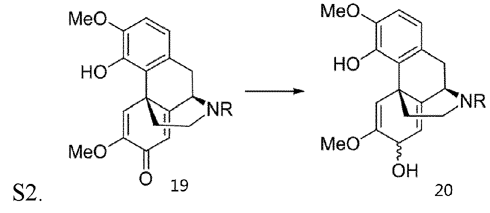
producing a compound 20 through reduction reaction of the compound 19; 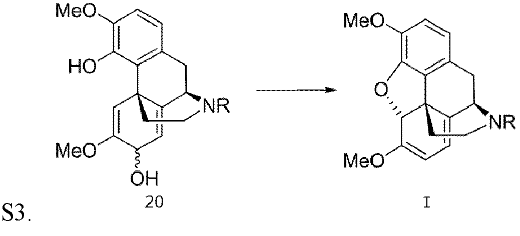
producing the intermediate I through cyclization reaction of the compound 20.

4. The method according to claim 3, wherein the hydroxyl protection group I is one selected from the group consisting of p-methoxybenzyl, benzyl, acetyl, benzyloxycarbonyl, methoxymethylene, methyl, triisopropylsilyl ether, triethylsilyl ether and tert-butyl diphenylsilyl.

5. The method according to claim 3, wherein in S1, a removal reagent for the removal reaction of the hydroxyl protection group R₁ is one selected from the group consisting of sodium hydrosulfide, sodium sulfide, sodium ethanethiolate, thiophenol, sodium p-thiocresol, potassium fluoride, tetrabutylammonium fluoride, acetic acid, trifluoroacetic acid, hydrobromic acid, trimethyliodosilane, cerium trichloride, ceric ammonium nitrate, camphor sulfonic acid, p-toluenesulfonic acid, phosphorus oxychloride, 2,3-dichloro-5,6-dicyano-1,4-benzoquinone and hydrochloric acid; and/or
in S1, a reaction solvent for the removal reaction of the hydroxyl protection group R₁ is one selected from the group consisting of N.N-dimethylacetamide, N-methylpyrrolidone, methanol, N,N-dimethylformamide, acetonitrile, tetrahydrofuran, dichloromethane, 1,2-dichloroethane and acetic acid.

6. The method according to claim 5, wherein in S1, the molar ratio of the compound 18 to the removal reagent is 1 :(3-25); and/or
in S 1, the removal reagent is hydrobromic acid, trifluoroacetic acid or sodium hydrosulfide; and/or
in S 1, the reaction solvent is dichloromethane, N,N-dimethylformamide or N,N-dimethylacetamide.

7. The method according to claim 3, wherein in S2, a reducing agent for the reduction reaction is one selected from the group consisting of sodium borohydride, lithium borohydride, lithium aluminum hydride and lithium tri-tert-butyl aluminum hydride; and/or
in S2, the reaction solvent for the reduction reaction is one or two selected from the group consisting of methanol, ethanol, tetrahydrofuran and dichloromethane; and/or in S2, the reaction temperature for the reduction reaction is -10 to 40°C.

8. The method according to claim 7, wherein in S2, the molar ratio of the compound 19 to the reducing agent is 1:(1.8-3); and/or
in S2, the reducing agent is sodium borohydride; and/or
in S2, the reaction solvent is methanol and dichloromethane; and/or
in S2, the temperature for the reduction reaction is 0-25°C.

9. The method according to claim 3, wherein in S3, the reaction solvent for the cyclization reaction is one selected from the group consisting of N,N-dimethylformamide, N,N-dimethylformamide dimethyl acetal, acetonitrile, tetrahydrofuran, dichloromethane and 1,4-dioxane; and/or
in S3, a cyclizing reagent for the cyclization reaction is one selected from the group consisting of N,N-dimethylformamide dinovaentyl acetal, N,N-dimethylformamide dimethyl acetal, N,N-dimethylformamide diethyl acetal and N,N-dimethylformamide diisopropyl acetal; and/or
in S3, the reaction temperature for the cyclization reaction is 0-130°C.

10. The method according to claim 9, wherein in S3, the molar ratio of the compound 20 to the cyclizing reagent is 1 :(2-12); and/or
in S3, the cyclizing reagent is N,N-dimethylformamide dimethyl acetal; and/or
in S3, the reaction solvent is tetrahydrofuran, 1,4-dioxane or N,N-dimethylformamide dimethyl acetal; and/or
in S3, the reaction temperature for the cyclization reaction is 50-130°C.

11. The method according to claim 3, wherein a synthesis route of the compound 18 is as follows: 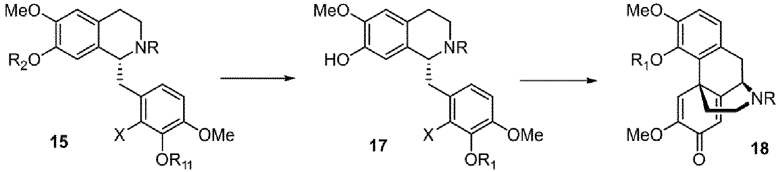
where R₂ is a hydroxyl protection group II, X is a halogen atom, R₁₁ is a hydroxyl protection group I or a hydrogen atom, and R₁ is a hydroxyl protection group I;
when R₁₁ is the hydroxyl protection group I, a synthesis method comprises the following steps:
1) providing a compound 15;
2) producing a compound 17 through removal of the hydroxyl protection group II from the compound 15; and
3) producing the compound 18 through intramolecular oxidative dearomatization Heck reaction of the compound 17;
when R₁₁ is the hydrogen atom, the hydroxyl protection group I is introduced into the compound 15, and then step 2) and step 3) are performed.

12. The method according to claim 11, wherein the hydroxyl protection group II is one selected from the group consisting of p-methoxybenzyl, benzyl, acetyl, benzoyl, tervalyl, tert-butyldimethylsilyl, tert-butyldiphenylsilyl, triisopropylsilyl and triethylsilyl.

13. The method according to claim 11, wherein the halogen atom is one selected from the group consisting of chlorine atom, bromine atom and iodine atom.

14. The method according to claim 11, wherein in step 2), a removal reagent for the removal of the hydroxyl protection group II is one or two selected from the group consisting of potassium carbonate, sodium methoxide, sodium hydroxide, potassium hydroxide, trifluoroacetic acid, hydrochloric acid, boron trichloride, acetic acid, tetrabutylammonium fluoride, tetraethyl ammonium fluoride, hydrobromic acid, potassium fluoride and cesium fluoride; and/or
in step 2), a reaction solvent for the removal of the hydroxyl protection group II is one or two selected from the group consisting of methanol, N,N-dimethylformamide, acetonitrile, tetrahydrofuran, dichloromethane and water.

15. The method according to claim 14, wherein in step 2), the removal reagent is potassium carbonate; and/or
in step 2), the reaction solvent is methanol; and/or
in step 2), the reaction temperature for the removal of the hydroxyl protection group II is 40-60°C.

16. The method according to claim 14, wherein in step 2), the removal reagent is potassium fluoride; and/or
in step 2), the reaction solvent is acetonitrile and water; and/or
in step 2), the reaction temperature for the removal of the hydroxyl protection group II is 0-60°C.

17. The method according to claim 11, wherein in step 3), the intramolecular oxidative dearomatization Heck reaction is performed in the presence of a reaction reagent and an alkali.

18. The method according to claim 17, wherein in step 3), the reaction reagent is a complex, or a ligand II and a transition metal catalyst II.

19. The method according to claim 18, wherein in step 3), the complex is one selected from the group consisting of Pd(PPh₃)₄, Pd(PPh₃)₂Cl₂, Pd(PtBu₃)₂, Pd(PCy₃)₂, Pd(PPhtBu₂)₂Cl₂, [1,2-bis(diphenylphosphoryl)ethane] palladium dichloride, [1,3-bis(diphenylphosphoryl)propane] palladium dichloride and [1,4-bis(diphenylphosphoryl)butane] palladium dichloride; and/or
the molar ratio of the compound 17 to the complex to the alkali is 1 :(0.025-0.5):(1-3).

20. The method according to claim 18, wherein in step 3), the ligand II is as expressed by formula (II), or is a stereoisomer or tautomer of formula (II) or a phosphonium hydrogen halide corresponding to formula (II); 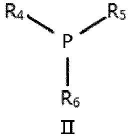 where
R₄ and R₅ are one selected from the group consisting of amaryl and tert-butyl;
R₆ is one selected from the group consisting of alkyl, cycloalkyl, aryl and heteroaryl, and the alkyl, cycloalkyl, aryl and heteroaryl are substituted by one or more independent hydrogen atoms, alkyl, halogen, cycloalkyl, aryl and heteroaryl respectively; and/or
in step 3), the transition metal catalyst II is one selected from the group consisting of [Pd(cinnamy)Cl]₂, [Pd(allyl)Cl]₂, Pd₂(dba)₃, Pd(OAc)₂, Pd(Tfa)₂, Pd(acac)₂, Pd(MeCN)₂Cl₂, Pd(PhCN)₂Cl₂, PdCl₂, Pd(Cp)(allyl), Pd(MeCN)₄(BF₄)₂, Pd(MeCN)₄(OTf)₂, Pd(cod)Cl₂, Pd(norbomadiene)Cl₂, Pd(TMEDA)Cl₂ and Pd(amphos)Cl₂; and/or
the molar ratio of the compound 17 to the ligand II to the transition metal catalyst II to the alkali is 1:(0.05-0.5):(0.05-0.15):(2-4).

21. The method according to claim 20, wherein in step 3), the ligand II is selected from 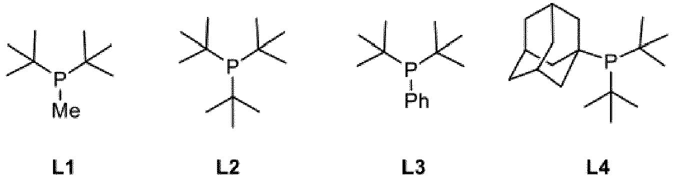 or 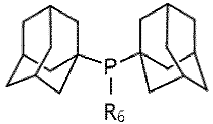 and phosphonium hydrogen halide 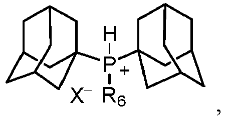 where R₆ is selected from the group consisting of C₁₋₂₀ alkyl or benzyl, and X is a halogen atom.

22. The method according to claim 17, wherein in step 3), the alkali is one or two selected from the group consisting of potassium t-butoxide, lithium carbonate, sodium carbonate, cesium carbonate, silver carbonate, potassium bicarbonate, potassium carbonate, potassium borofluorite, potassium phosphate, dipotassium hydrogen phosphate, sodium tert-butanol, lithium tert-butanol, sodium hydride, potassium hydride, sodium acetate, sodium methoxide, sodium benzoate, potassium benzoate, pyridine, triethylamine, diisopropylethylamine, cesium fluoride, potassium hydroxide, and pivalate; and/or
in step 3), a reaction solvent for the intramolecular oxidative dearomatization Heck reaction is one selected from the group consisting of anisole, trifluorotoluene, N,N-dimethylformamide, N,N-dimethylacetamide, dimethyl sulfoxide, N-methylpyrrolidone, trimethylbenzene, dimethyl ether, ethanol, tert-butyl alcohol, methylbenzene, chlorobenzene, xylene, 1,4-dioxane, diethylene glycol dimethyl ether, methyl tert-butyl ether, tetrahydrofuran and ethylene glycol dimethyl ether; and/or
in step 3), the concentration of the compound 17 in the intramolecular oxidative dearomatization Heck reaction is 0.05-2.5mol/L; and/or
in step 3), the temperature for the intramolecular oxidative dearomatization Heck reaction is 50-160°C.

23. The method according to claim 22, wherein in step 3), the alkali is potassium phosphate and potassium carbonate; and/or
in step 3), the reaction solvent is one of N,N-dimethylformamide, N,N-dimethylacetamide and anisole; and/or
in step 3), the concentration of the compound 17 in the intramolecular Heck reaction is 0.05-1mol/L; and/or
in step 3), the temperature for the intramolecular Heck reaction is 60-150°C.

24. The method according to claim 11, wherein a synthesis route of the compound 15 is as follows: 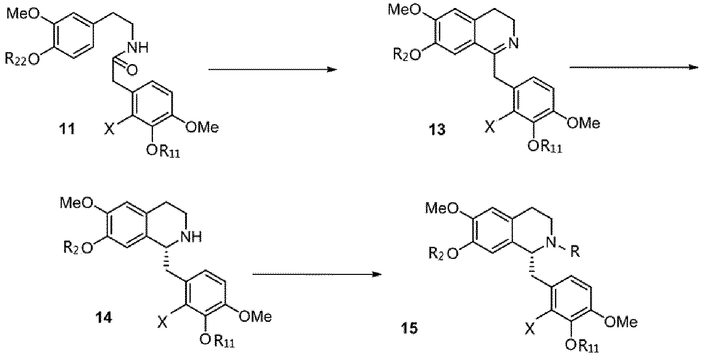
where R₂ is a hydroxyl protection group II, R₂₂ is a hydroxyl protection group II or a hydrogen atom, X is a halogen atom, and R₁₁ is a hydroxyl protection group I or a hydrogen atom;
when R₂₂ is the hydroxyl protection group II, a synthesis method comprises the following steps:
a. providing a compound 11;
b. producing a compound 13 through Bischler-Napieralski reaction of the compound 11;
c. producing a chiral tetrahydroisoquinoline type compound 14 through asymmetric transfer hydrogenation of the compound 13; and
d. performing secondary amine protection on the compound 14 to produce the compound 15;
when R₂₂ is the hydrogen atom, the hydroxyl protection group II is introduced into the compound 11, and then steps b, c and d are performed.

25. The method according to claim 24, wherein in step b, the Bischler-Napieralski reaction is performed in the presence of a condensation agent and an alkali; the molar ratio of the compound 11 to the condensation agent to the alkali is 1:(0.9-1.3):(1.5-2.5).

26. The method according to claim 25, wherein the condensation agent is one selected from the group consisting of phosphine oxychloride, phosphorus pentoxide and trifluoromethyl sulfonic anhydride; and/or
in step b, the alkali is one selected from the group consisting of 2-fluoropyridine, pyridine, 4-dimethylaminopyridine, dimethylpyridine and triethylamine; and/or
in step b, a reaction solvent for the Bischler-Napieralski reaction is one selected from the group consisting of dichloromethane, dichloroethane, tetrahydrofuran and toluene; and/or
in step b, the temperature for the Bischler-Napieralski reaction is -50 to 40°C.

27. The method according to claim 26, wherein in step b, the condensation agent is trifluoromethyl sulfonic anhydride; and/or
in step b, the alkali is 2-fluoropyridine; and/or
in step b, the reaction solvent is dichloromethane; and/or
in step b, the temperature for the Bischler-Napieralski reaction is -30 to 35°C.

28. The method according to claim 24, wherein in step c, the asymmetric transfer hydrogenation reaction is performed in the presence of a chiral ligand I, a hydrogen source I and a metal catalyst I; the molar ratio of the compound 13 to the metal catalyst I to the chiral ligand I to the hydrogen source I is 1:(0.001-0.01):(0.002-0.02):(1.2-3).

29. The method according to claim 28, wherein in step c, the chiral ligand I is one selected from the group consisting of 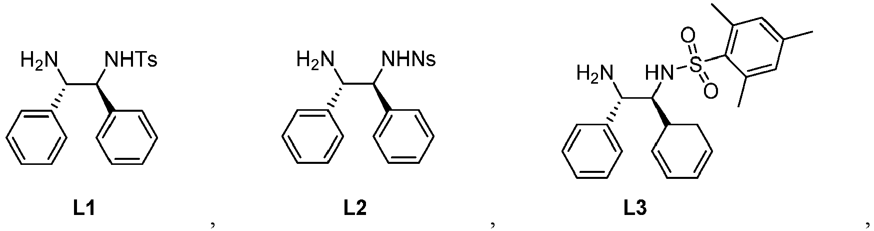 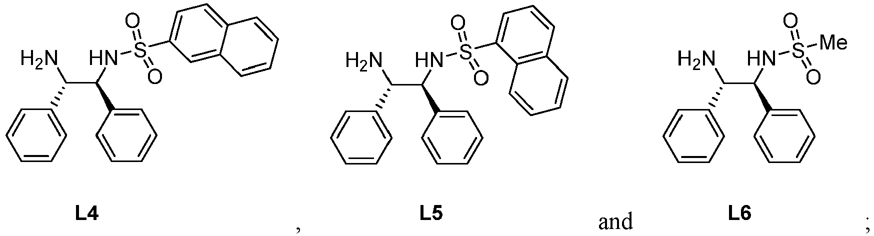 and/or
in step c, the hydrogen source I is one selected from the group consisting of formic acid, ammonium formate and a complex of formic acid and trialkylamine; and/or
in step c, the metal catalyst I is one selected from the group consisting of 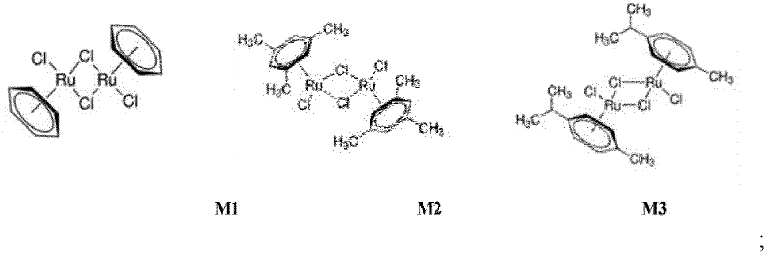 and/or
in step c, a reaction solvent for the asymmetric hydrogenation reaction is one selected from the group consisting of dichloromethane, dichloroethane, chloroform, tetrahydrofuran, dimethyl ether, tert-butyl methyl ether, trifluoroethanol, anisole, N,N-dimethylformamide, trifluorotoluene, N,N-dimethylacetamide, dimethyl sulfoxide, N-methylpyrrolidone, trimethylbenzene, ethanol, tert-butyl alcohol, toluene, chlorobenzene, xylene, 1,4-dioxane, dichlorobenzene, hexafluoroisopropanol, methanol and isopropanol; and/or
in step c, the temperature for the transfer hydrogenation reaction is -10 to 40°C.

30. The method according to claim 29, wherein in step c, the hydrogen source I is a complex of methanol and triethylamine; and/or
in step c, the reaction solvent is N,N-dimethylformamide; and/or
in step c, the temperature for the transfer hydrogenation reaction is 0-35°C.

31. The method according to claim 24, wherein in step d, the secondary amine protection is performed under an alkaline condition; an alkali used in the alkaline condition is one selected from the group consisting of disodium hydrogen phosphate, sodium dihydrogen phosphate, potassium carbonate, sodium carbonate, triethylamine, N,N-diisopropylethylamine, pyridine and 4-dimethylaminopyridine.

32. The method according to claim 31, wherein in step d, the reaction temperature for the secondary amine protection is -10 to 50°C.

33. The method according to claim 24, wherein a method for preparing the compound 11 comprises the following steps: providing a compound 9 and a compound 5, and performing amine acid condensation reaction to obtain the compound 11I, and the reaction formula is as follow: 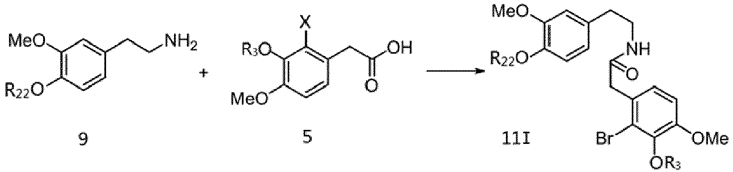 where R₃ is a methyl or hydrogen atom, X is a halogen atom, and R₂₂ is a hydrogen atom or a hydroxyl protection group II.

34. The method according to claim 33, wherein the amine acid condensation reaction is performed in the presence of a condensation reagent and an alkali; the molar ratio of the compound 9 to the compound 5 to the condensation reagent to the alkali is (1-1.6):1:(1-1.2):(1.5-3).

35. The method according to claim 34, wherein the condensation reagent is one selected from the group consisting of O-benzotriazole-N,N,N',N'-tetramethylurea tetrafluoroboric acid, 1-ethyl-(3-dimethylaminopropyl)carbodiimide hydrochloride, 2-(7-azobenzotriazole)-N,N,N',N'-tetramethylurea hexafluorophosphate, dicyclohexylcarbodiimide and benzotriazole-1-yloxytris(dimethylamino) phosphonium hexafluorophosphate; and/or
the alkali is one selected from the group consisting of triethylamine, N,N-diisopropylethylamine, 4-dimethylaminopyridine and pyridine; and/or
the temperature for the amine acid condensation reaction is -10 to 50°C.

36. The method according to claim 35, wherein the condensation reagent is O-benzotriazole - N,N,N',N'-tetramethylurea tetrafluoroboric acid; and/or
the alkali is triethylamine; and/or
the temperature for the amine acid condensation reaction is 0-25°C.

37. The method according to claim 33, wherein a reaction formula for R₃ in the compound 11I to be substituted by the hydroxyl protection group R1 to obtain a compound 11II is as follow: 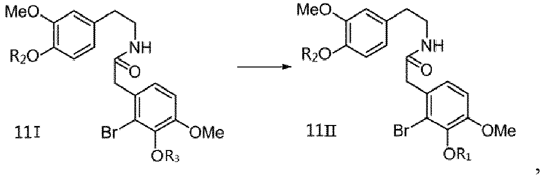 where R₁ is a hydroxyl protection group I and R₂ is a hydroxyl protection group II.

38. The method according to claim 33, wherein a method for preparing the compound 9 comprises the following steps: 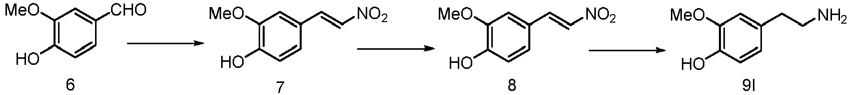
A. providing a compound 6;
B. producing a compound 7 through Henry reaction of the compound 6 and nitromethane;
C. producing a compound 8 through double bond reduction reaction of the compound 7; and
D. producing a compound 9I through nitro reduction reaction of the compound 8.

39. The method according to claim 38, wherein in step B, the Henry reaction of the compound 6 and nitromethane is performed under the catalysis of an alkali, and the alkali is one or more of ethanediamine, ammonium acetate, sodium hydroxide, piperidine, diethylamine and morpholine.

40. The method according to claim 38, wherein in step C, a reducing agent for the double bond reduction reaction is one selected from the group consisting of lithium aluminum hydride, sodium borohydride, palladium carbon+hydrogen, Raney nickel+hydrogen, lithium borohydride, Red-Al, zinc powder and iron powder; and/or
in step C, a reaction solvent for the double bond reduction reaction is one selected from the group consisting of ethanol, ether, isopropyl ether, methyl tert-butyl ether, tetrahydrofuran, 1,4-dioxane, toluene, ethylene glycol dimethyl ether and diethylene glycol dimethyl ether; and/or
in step C, the temperature for the double bond reduction reaction is -10 to 10°C.

41. The method according to claim 38, wherein in step C, the molar ratio of the compound 7 to the reducing agent is 1 :(1-3); and/or
in step C, the reducing agent is sodium borohydride; and/or
in step C, the reaction solvent is ethanol and tetrahydrofuran; and/or
in step C, the temperature for the reduction reaction is 0°C.

42. The method according to claim 38, wherein in step D, a reducing agent for the nitro reduction reaction is one selected from the group consisting of lithium aluminum hydride, sodium borohydride, palladium carbon+hydrogen, Raney nickel+hydrogen, lithium borohydride, Red-Al, zinc powder and iron powder; and/or
in step D, a reaction solvent for the nitro reduction reaction is one selected from the group consisting of ethanol, ether, isopropyl ether, methyl tert-butyl ether, tetrahydrofuran, 1,4-dioxane, toluene, ethylene glycol dimethyl ether and diethylene glycol dimethyl ether; and/or
in step D, the temperature for the nitro reduction reaction is -10 to 80°C.

43. The method according to claim 42, wherein in step D, the molar ratio of the compound 8 to the reducing agent is 1 :(1-3); and/or
in step D, the reducing agent is Raney nickel+hydrogen; and/or
in step D, the reaction solvent is ethanol; and/or
in step D, the temperature for the reduction reaction is 10-80°C.

44. The method according to claim 38, wherein a compound 9II is further produced through hydroxyl protection reaction of the compound 9I, and the reaction formula is as follow: 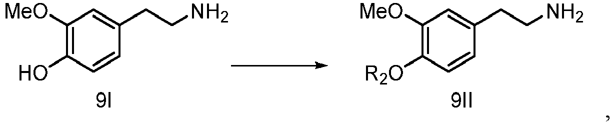 R₂ is a hydroxyl protection group II.

45. The method according to claim 44, wherein the hydroxyl protection reaction of the compound 9I is performed under an alkaline condition; an alkali used in the alkaline condition is one or two selected from the group consisting of 4-dimethylaminopyridine, sodium hydride, triethylamine, pyridine and imidazole; and/or,
a reaction solvent for the hydroxyl protection reaction of the compound 9I is one selected from the group consisting of dichloromethane, dichloroethane, tetrahydrofuran and toluene; and/or
the temperature for the hydroxyl protection reaction of the compound 9I is 0-40°C.

46. The method according to claim 33, wherein a method for preparing the compound 5 comprises the following steps: 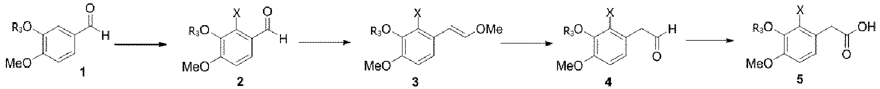 where R₃ is methyl or hydrogen atom
(1) providing a compound 1;
(2) producing a compound 2 through halogenation reaction of the compound 1;
(3) producing a compound 3 through Wittig reaction of the compound 2;
(4) producing a compound 4 through hydrolysis reaction of the compound 3; and
(5) producing the compound 5 through oxidization reaction of the compound 4.

47. Application of the intermediate according to claim 1 or 2 or the method according to any one of claims 3-46 to the preparation of morphine and a morphine derivative.

48. The application according to claim 47, wherein the morphine derivative comprises one of codeine, oxycodone, hydrocodone, buprenorphine, nalaxone, naltrexone and nalbuphine.

49. The application according to claim 48, wherein a method for producing codeine through further reaction of the intermediate I comprises the following steps:
A) producing a compound 22 through hydrolysis reaction of the intermediate I under an acidic condition: 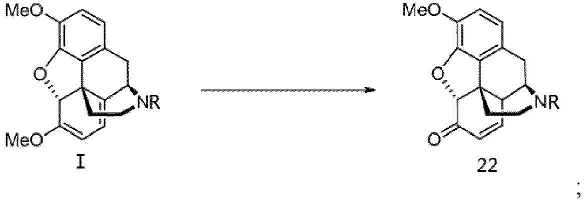
B) producing a compound 23 by removing a secondary amine protection group R from the compound 22 and reducing carbonyl: 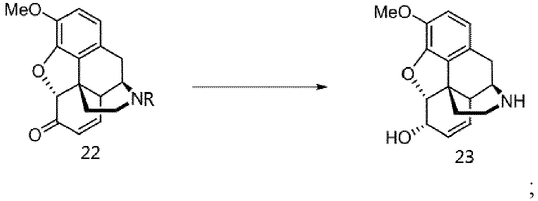 and
C) producing codeine 24 through reductive amine methylation reaction of the compound 23:

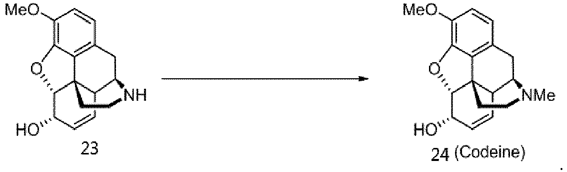

50. The application according to claim 49, wherein in step A), an acid in the hydrolysis reaction is one or more selected from the group consisting of hydrogen bromide, sulfuric acid, boron trichloride, boron tribromide, silica gel, hydrochloric acid, p-toluene sulfonic acid, camphor sulfonic acid, acetic acid and trifluoroacetic acid; and/or
in step A), a solvent for the hydrolysis reaction is one or more selected from the group consisting of n-butyl ether, chloroform, isopropanol, ethanol, tetrahydrofuran, ethylene glycol dimethyl ether, methanol, dichloromethane and water; and/or
in step A), the reaction temperature for the hydrolysis reaction is -40 to 80°C.

51. The application according to claim 49, wherein in step B), a reaction reagent used in the process of removing the secondary amine protection group and reducing carbonyl is at least one selected from the group consisting of red aluminum, sodium-naphthalene, lithium aluminum hydride and magnesium powder; and/or
in step B), the molar ratio of the compound 23 to the reaction reagent is 1:(2-6); and/or
in step B), the reaction solvent used in the process of removing the secondary amine protection group and reducing carbonyl is at least one selected from the group consisting of tetrahydrofuran, ethylene glycol dimethyl ether and toluene; and/or
in step B), the reaction temperature is 25-80°C in the process of removing the secondary amine protection group and reducing carbonyl.

52. The application according to claim 49, wherein in step C), a reaction reagent for the reductive amine methylation reaction is two or more selected from the group consisting of polyformaldehyde, formaldehyde aqueous solution and sodium borohydride; and/or
in step C), the molar ratio of the compound 22 to the reaction reagent is 1:(1-3); and/or
in step C), a reaction solvent for the reductive amine methylation reaction is at least one selected from the group consisting of methanol, ethanol, tetrahydrofuran and acetic acid; and/or
in step C), the reaction temperature for the reductive amine methylation reaction is 25-60°C.

53. The application according to claim 48, wherein a method for producing oxycodone through further reaction of the intermediate I comprises the following steps:
a) producing a compound 25 through oxa-D-A reaction of the intermediate I: 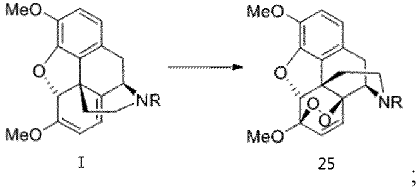
b) producing a compound 26 through catalytic hydrogenation reaction of the compound 25: 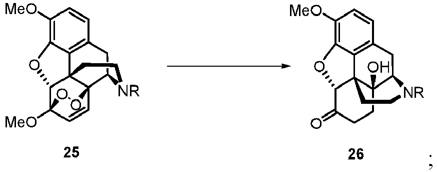
c) producing a compound 27 by removing the secondary amine protection group from the compound 26 and reducing carbonyl and alkenyl: 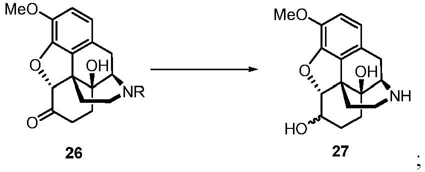
d) producing a compound 28 through reductive amine methylation reaction of the compound 27: 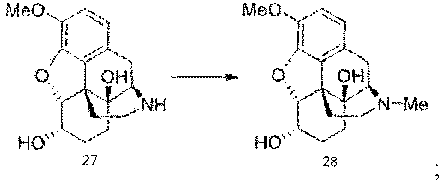 and
e) producing oxycodone 29 through selective oxidization reaction of the compound 28:

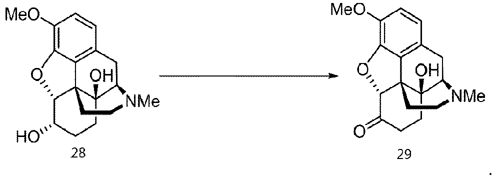

54. The application according to claim 53, wherein in step a), the oxa-D-A reaction is performed under an illumination condition; a light source for the illumination condition is one selected from the group consisting of natural light and LED light.

55. The application according to claim 53, wherein in step a), the oxa-D-A reaction is performed in the presence of an oxidant and a photocatalyst.

56. The application according to claim 53, wherein in step a), the photocatalyst is tetraphenylporphyrin; and/or
in step a), the oxidant is one selected from the group consisting of oxygen, formic acid+hydrogen peroxide, acetic acid+hydrogen peroxide, tert-butyl peroxide and m-chloroperoxybenzoic acid; and/or
in step a), a reaction solvent for the oxa-D-A reaction is one selected from the group consisting of N,N-dimethylformamide, tetrahydrofuran, dichloromethane, dichloroethane and toluene; and/or
in step a), the temperature for the oxa-D-A reaction is 0-30°C.

57. The application according to claim 56, wherein in step a), the molar ratio of the intermediate I to the photocatalyst is 1 :(0.1-0.3); and/or
in step a), the oxidant is oxygen; and/or
in step a), the reaction solvent is dichloromethane; and/or
in step a), the temperature for the oxa-D-A reaction is 10-30°C.

58. The application according to claim 53, wherein in step b), a catalyst for the catalytic hydrogenation reaction is one selected from the group consisting of palladium carbon, palladium chloride and palladium hydroxide; and/or
in step b), a reaction solvent for the catalytic hydrogenation reaction is one or more selected from the group consisting of methanol, ethanol, dichloromethane, dichloroethane, ethyl acetate, isopropanol, formic acid and water; and/or
in step b), the hydrogen pressure of the catalytic hydrogenation reaction is 1-25atm; and/or
in step b), the temperature for the catalytic hydrogenation reaction is 0-80°C.

59. The application according to claim 58, wherein in step b), the molar ratio of the compound 25 to the catalyst is 1:(0.1-0.3); and/or
in step b), the catalyst is palladium carbon; and/or
in step b), the reaction solvent is isopropanol, water and formic acid; and/or
in step b), the hydrogen pressure of the catalytic hydrogenation reaction is 6-12atm; and/or
in step b), the temperature for the catalytic hydrogenation reaction is 10-40°C.

60. The application according to claim 53, wherein in step c), a reaction reagent used in the process of removing the secondary amine protection group and reducing carbonyl and alkenyl is one selected from the group consisting of red aluminum, lithium aluminum hydride, magnesium powder and sodium naphthalene; and/or
in step c), a reaction solvent used in the process of removing the secondary amine protection group and reducing carbonyl and alkenyl is one selected from the group consisting of tetrahydrofuran, ethylene glycol dimethyl ether and toluene; and/or
in step c), the reaction temperature is 25-80°C in the process of removing the secondary amine protection group and reducing carbonyl and alkenyl.

61. The application according to claim 60, wherein in step c), the molar ratio of the compound 26 to the reaction reagent is 1 :(3-6); and/or
in step c), the reaction reagent is lithium aluminum hydride; and/or
in step c), the reaction solvent is tetrahydrofuran; and/or
in step c), the reaction temperature is 40-70°C.

62. The application according to claim 53, wherein in step d), a reaction reagent for the reductive amine methylation reaction is one selected from the group consisting of polyformaldehyde+sodium borohydride and formaldehyde aqueous solution+sodium borohydride; and/or
in step d), a reaction solvent for the reductive amine methylation reaction is one selected from the group consisting of methanol, ethanol, tetrahydrofuran and acetic acid; and/or
in step d), the temperature for the reductive amine methylation reaction is 25-60°C.

63. The application according to claim 62, wherein in step d), the molar ratio of the compound 27 to the reaction reagent is 1 :(2-5); and/or
in step d), the reaction reagent is polyformaldehyde+sodium borohydride; and/or
in step d), the reaction solvent is methanol; and/or
in step d), the temperature for the reductive amine methylation reaction is 10-40°C.

64. The application according to claim 53, wherein in step e), an oxidant for the selective oxidization reaction is one selected from the group consisting of Dess-Martin oxidant, 2-iodobenzoic acid, oxalyl chloride+dimethyl sulfoxide and acetic anhydride; and/or
in step e), a reaction solvent for the selective oxidization reaction is one selected from the group consisting of dichloromethane, ethyl acetate and dimethyl sulfoxide; and/or in step e), the temperature for the selective oxidization reaction is -10 to 40°C.

65. The application according to claim 64, wherein in step e), the molar ratio of the compound 28 to the oxidant is 1 :(3-5); and/or
in step e), the oxidant is Dess-Martin oxidant; and/or
in step e), the reaction solvent is dichloromethane; and/or
in step e), the temperature is 0-30°C.

66. The application according to claim 48, wherein a method for producing naltrexone through further reaction of the intermediate I comprises the following steps:
(1) preparing the compound 27 according to the application according to claim 53, and producing a compound 30 through reductive amination reaction of the compound 27 and cyclopropyl formaldehyde: 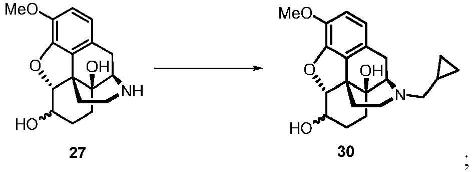
(2) producing a compound 31 through selective oxidization reaction of the compound 30: 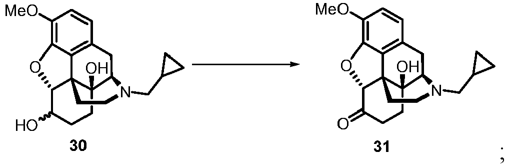 and
(3) producing naltrexone 32 through demethylation reaction of the comound 31:

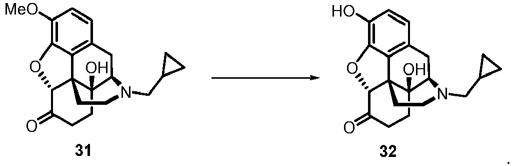

67. The application according to claim 66, wherein in step (1), a reaction reagent for the reductive amination reaction is one selected from the group consisting of cyclopropyl formaldehyde+sodium borohydride, cyclopropyl formaldehyde+sodium cyanide borohydride, and cyclopropyl formaldehyde+sodium triacetoxyborohydride;
and/or
in step (1), a reaction solvent for the reductive amination reaction is one selected from the group consisting of methanol, ethanol, tetrahydrofuran, 1,2-dichloroethane and acetic acid; and/or
in step (1), the temperature for the reductive amination reaction is 25-60°C.

68. The application according to claim 67, wherein in step (1), the molar ratio of the compound 27 to the reaction reagent is 1 :(2-5); and/or
in step (1), the reaction reagent is cyclopropyl formaldehyde+sodium borohydride; and/or
in step (1), the reaction solvent is methanol; and/or
in step (1), the temperature for the reductive amination reaction is 10-40°C.

69. The application according to claim 66, wherein in step (2), an oxidant for the selective oxidization reaction is one selected from the group consisting of Dess-Martin oxidant, 2-iodobenzoic acid, oxalyl chloride+dimethyl sulfoxide and acetic anhydride; and/or
in step (2), a reaction solvent for the selective oxidization reaction is one selected from the group consisting of dichloromethane, ethyl acetate and dimethyl sulfoxide; and/or
in step (2), the temperature for the selective oxidization reaction is -10 to 40°C.

70. The application according to claim 69, wherein in step (2), the molar ratio of the compound 30 to the oxidant is 1:(3-5); and/or
in step (2), the oxidant is Dess-Martin oxidant; and/or
in step (2), the reaction solvent is dichloromethane; and/or
in step (2), the temperature is 0-30°C.

71. The application according to claim 66, wherein in step (3), a demethylation reagent used in the demethylation reaction is one or two selected from the group consisting of boron tribromide, hydrobromic acid, oxalic acid, methanesulfonic acid, trifluoromethanesulfonic acid, phenylthiol, isopropyl mercaptan, sodium ethanethiolate, potassium t-butoxide, potassium hydroxide, potassium carbonate, potassium hydride, sodium hydride and sodium ethanol; and/or
in step (3), a reaction solvent for the demethylation reaction is one or two selected from the group consisting of dichloromethane, chloroform, water, methanol, ethanol, diethylene glycol, ethylene glycol, N-methylpyrrolidone, N,N-dimethylformamide, N,N-dimethylacetamide, toluene, chlorobenzene, and hexamethylphosphoric triamide.

72. The application according to claim 71, wherein in step (3), the molar ratio of the compound 31 to the demethylation reagent is 1:(3-8); and/or
in step (3), the demethylation reagent is boron tribromide; and/or
in step (3), the reaction solvent is chloroform; and/or
in step (3), the temperature is 0-30°C.

73. The application according to claim 48, wherein a method for producing nalaxone through further reaction of the intermediate I comprises the following steps:
i. preparing a compound 27 according to the application according to claim 53, and producing a compound 33 through alkylation reaction of the compound 7 and allyl bromide: 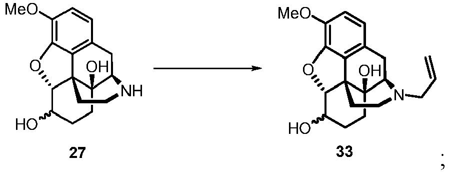
ii. producing a compound 34 through selective oxidization reaction of the compound 33: 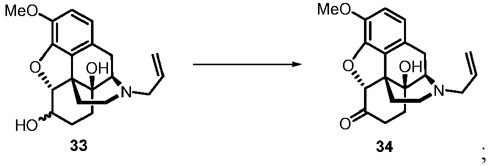 and
iii. producing nalaxone through demethylation reaction of the compound 34:

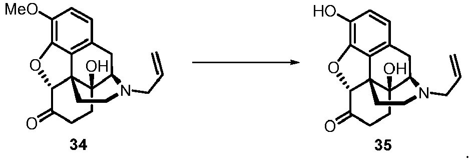

74. The application according to claim 73, wherein in step i, an alkali for the nitrogen alkylation reaction is one or two selected from the group consisting of potassium carbonate, sodium carbonate, sodium bicarbonate, triethylamine and diisopropylethylamine; and/or
in step i, a solvent for the nitrogen alkylation reaction is one or two selected from the group consisting of nitromethylpyrrolidone, tetrahydrofuran, acetone and water; and/or
in step i, the temperature for the nitrogen alkylation reaction is 25-80°C.

75. The application according to claim 74, wherein in step i, the molar ratio of the compound 27 to the allyl bromide to the alkali is 1 :(2-5):(1.2-2); and/or
in step i, the alkali is triethylamine; and/or
in step i, the reaction solvent is nitromethylpyrrolidone and water; and/or
in step i, the temperature for the nitrogen alkylation reaction is 50-70°C.

76. The application according to claim 73, wherein in step ii, an oxidant for the selective oxidization reaction is one selected from the group consisting of Dess-Martin oxidant, 2-iodobenzoic acid, oxalyl chloride+dimethyl sulfoxide and acetic anhydride; and/or
in step ii, a reaction solvent for the selective oxidization reaction is one selected from the group consisting of dichloromethane, ethyl acetate and dimethyl sulfoxide; and/or
in step ii, the temperature for the selective oxidization reaction is -10 to 40°C.

77. The application according to claim 76, wherein in step ii, the molar ratio of the compound 33 to the oxidant is 1:(3-5); and/or
in step ii, the oxidant is Dess-Martin oxidant; and/or
in step ii, the reaction solvent is dichloromethane; and/or
in step ii, the temperature is 0-30°C.

78. The application according to claim 73, wherein in step iii, a demethylation reagent used in the demethylation reaction is one or two selected from the group consisting of boron tribromide, hydrobromic acid, oxalic acid, methanesulfonic acid, trifluoromethanesulfonic acid, phenylthiol, isopropyl mercaptan, sodium ethanethiolate, potassium t-butoxide, potassium hydroxide, potassium carbonate, potassium hydride, sodium hydride and sodium ethylate; and/or
in step iii, a reaction solvent for the demethylation reaction is one or two selected from the group consisting of dichloromethane, chloroform, water, methanol, ethanol, diethylene glycol, ethylene glycol, N-methylpyrrolidone, N,N-dimethylformamide, N,N-dimethylacetamide, toluene, chlorobenzene, and hexamethylphosphoric triamide.

79. The application according to claim 78, wherein in step iii, the molar ratio of the compound 31 to the demethylation reagent is 1:(3-8); and/or
in step iii, the demethylation reagent is boron tribromide; and/or
in step iii, the reaction solvent is chloroform; and/or
in step iii, the temperature is 0-30°C.
